# EUROPEAN PATENT APPLICATION

(11) **EP 4 741 492 A2**
(43) Date of publication of application: **13.05.2026**
(21) Application number: 26165239.0
(22) Date of filing: 09.01.2024
(51) Int. Cl.: C12N 5/00

(54) **NECROSIS INHIBITOR**

(30) Priority: 09.01.2023 GB 202300310
(62) Divisional of application: 24705734.2
(71) Applicant: Linkgevity Limited, London SW7 4AG (GB)
(72) Inventor: KERN, Carina, London, SW7 4AG (GB)
(74) Representative: Carpmaels & Ransford LLP

(57) **Abstract**

Provided herein are compositions comprising an endoplasmic/sarcoplasmic reticulum calcium channel inhibitor and a gap junction inhibitor for use in a variety of methods, such as methods for treating or preventing necrosis, methods for treating or preventing cell death, methods for treating or preventing tissue damage, or methods for treating or preventing organ damage. The compositions may also comprise a plasma membrane calcium channel inhibitor and/or a calcium chelator. Also provided are compositions comprising a calpain inhibitor and a cathepsin inhibitor for use in a variety of methods. Also provided are compositions comprising calcium chelators, preferably in relatively high doses, for use in a variety of methods.

## Description

### Field of the Invention

The present invention relates to methods of treating or preventing necrosis, cell death, tissue damage, or organ damage.

The invention relates to medical use of calcium activity inhibitors, calpain inhibitors, cathepsin inhibitors and/or gap junction inhibitors, particularly in treating or preventing necrosis, cell death, tissue damage, or organ damage. The invention also extends to cosmetic compositions, food products and food preservatives comprising calcium activity inhibitors, calpain inhibitors, cathepsin inhibitors and/or gap junction inhibitors, and methods of using the same to store food, cells, tissues, and organs, and methods of using the same to culture cells, tissues or organs. For example, the invention may relate to calpain inhibitors in combination with cathepsin inhibitors. For example, the invention may relate to calcium chelators, particularly where the calcium chelators are used at relatively high doses.

The invention also relates to the advantageous combination of one or more endoplasmic/sarcoplasmic reticulum calcium channel inhibitors (such as one or more ryanodine receptor antagonists) and one or more gap junction inhibitors. Thus, the invention also relates to medical use of one or more endoplasmic/sarcoplasmic reticulum calcium channel inhibitors (such as one or more ryanodine receptor antagonists) in combination with one or more gap junction inhibitors, particularly in treating or preventing necrosis, cell death, tissue damage, or organ damage. The invention also extends to cosmetic compositions, food products and food preservatives comprising one or more endoplasmic/sarcoplasmic reticulum calcium channel inhibitors (such as one or more ryanodine receptor antagonists) in combination with one or more gap junction inhibitors, and methods of using the same to store food, cells, tissues, and organs, and methods of using the same to culture cells, tissues or organs. The combination of the one or more endoplasmic/sarcoplasmic reticulum calcium channel inhibitors (such as one or more ryanodine receptor antagonists) and the one or more gap junction inhibitors may be further combined with (i) one or more plasma membrane calcium channel inhibitors, and/or (ii) one or more calcium chelators.

### Background

Necrosis is one of two main types of cell death that occur in living organisms, the other being programmed cell death (also known as apoptosis).

One aspect of the invention herein relates to treating or preventing cell death, which encompasses treating or preventing necrosis and/or apoptosis.

Necrosis has been characterized as passive, accidental cell death resulting from environmental perturbations (i.e., a stressful trigger) with uncontrolled release of cellular contents. It is believed to be mediated by random intracellular events. If necrotic cells in a tissue are left untreated, the necrosis will spread to neighbouring cells and tissues. This, in turn, can lead to irreversible organ damage (e.g., organ failure), and even death. Apoptosis, on the other hand, is described as an active, programmed process of autonomous cellular dismantling. Unlike necrosis, apoptosis does not typically spread to neighbouring cells or tissues.

Current treatments for necrosis target the *triggers* of necrosis, rather than trying to directly stop/prevent the cellular changes associated with necrosis; this is because necrosis is traditionally believed to be mediated by random intracellular events. As these work by only attempting to block *triggers,* their effectiveness is limited to the extent that the trigger is the sole cause of necrosis, treatment must be given early enough to supress the trigger, and the treatment may be associated with an unwanted trade off. One such class of drugs is immunosuppressants. They work by supressing secondary inflammation that can trigger necrosis, e.g., TNF-α inhibition. A trade-off associated with immunosuppressants is the increased risk of infection.

There is therefore a need for an improved therapy for directly treating cell death, necrosis and other medical conditions that occur downstream of necrosis.

### Statements of the Invention

According to a first aspect of the invention, there is provided a calcium activity inhibitor, a calpain inhibitor, a cathepsin inhibitor and/or a gap junction inhibitor for use in treating or preventing (i) necrosis, (ii) tissue damage, (iii) organ damage, or (iv) cell death in a subject. For example, one or more cathepsin inhibitors in combination with one or more calpain inhibitors may be used. As another example, one or more calcium chelators may be used.

According to a preferred aspect of the invention, there is provided a composition comprising one or more endoplasmic/sarcoplasmic reticulum calcium channel inhibitors (such as one or more ryanodine receptor antagonists) and one or more gap junction inhibitors for use in a method of treating or preventing (i) necrosis, (ii) tissue damage, (iii) organ damage, or (iv) cell death in a subject.

According to a preferred aspect of the invention, there is provided one or more endoplasmic/sarcoplasmic reticulum calcium channel inhibitors (such as one or more ryanodine receptor antagonists) for use in a method of treating or preventing (i) necrosis, (ii) tissue damage, (iii) organ damage, or (iv) cell death, in a subject, wherein said endoplasmic/sarcoplasmic reticulum calcium channel inhibitor is to be administered in combination with one or more gap junction inhibitors (for example, one gap junction inhibitor or two gap junction inhibitors). The endoplasmic/sarcoplasmic reticulum calcium channel inhibitor(s) and the gap junction inhibitor(s) may be administered simultaneously, separately or sequentially. For example, the endoplasmic/sarcoplasmic reticulum calcium channel inhibitor(s) and the gap junction inhibitor(s) may be administered simultaneously.

According to a preferred aspect of the invention, there is provided one or more gap junction inhibitors for use in a method of treating or preventing (i) necrosis, (ii) tissue damage, (iii) organ damage, or (iv) cell death in a subject, wherein said gap junction inhibitor is to be administered in combination with one or more endoplasmic/sarcoplasmic reticulum calcium channel inhibitors (such as one or more ryanodine receptor antagonists). The endoplasmic/sarcoplasmic reticulum calcium channel inhibitor(s) and the gap junction inhibitor(s) may be administered simultaneously, separately or sequentially. For example, the endoplasmic/sarcoplasmic reticulum calcium channel inhibitor(s) and the gap junction inhibitor(s) may be administered simultaneously.

The combination of the one or more endoplasmic/sarcoplasmic reticulum calcium channel inhibitors and the one or more gap junction inhibitors may be further combined with one or more plasma membrane calcium channel inhibitors, and/or one or more calcium chelators.

According to another aspect of the invention, there is provided a method of treating or preventing (i) necrosis, (ii) tissue damage, (iii) organ damage, or (iv) cell death in a subject, the method comprising: administering a calcium activity inhibitor, a calpain inhibitor, a cathepsin inhibitor and/or a gap junction inhibitor to the subject to treat or prevent necrosis, tissue damage, organ damage or cell death. For example, one or more cathepsin inhibitors in combination with one or more calpain inhibitors may be administered. As another example, one or more calcium chelators may be administered.

According to a preferred aspect of the invention, there is provided a method of treating or preventing (i) necrosis, (ii) tissue damage, (iii) organ damage, or (iv) cell death in a subject, the method comprising: administering one or more endoplasmic/sarcoplasmic reticulum calcium channel inhibitors (such as one or more ryanodine receptor antagonists) and one or more gap junction inhibitors to the subject to treat or prevent necrosis, tissue damage, organ damage or cell death. The endoplasmic/sarcoplasmic reticulum calcium channel inhibitor(s) and the gap junction inhibitor(s) may be administered simultaneously, separately or sequentially. For example, the endoplasmic/sarcoplasmic reticulum calcium channel inhibitor(s) and the gap junction inhibitor(s) may be administered simultaneously. Additionally, one or more plasma membrane calcium channel inhibitors, and/or one or more calcium chelators may be administered.

According to another aspect of the invention, there is provided a method of preventing necrosis or cell death in a cell or tissue, the method comprising: contacting the cell or tissue with a calcium activity inhibitor, a calpain inhibitor, a cathepsin inhibitor and/or a gap junction inhibitor to prevent necrosis or cell death in the cell or tissue. For example, one or more cathepsin inhibitors in combination with one or more calpain inhibitors may be used. As another example, one or more calcium chelators may be used. The methods of preventing necrosis or cell death in a cell or tissue may occur *in vivo* or *in vitro.* For *in vitro* methods, the calcium chelator(s) are preferably used in relatively high doses.

According to another preferred aspect of the invention, there is provided a method of preventing (i) necrosis or (ii) cell death in a cell or tissue, the method comprising: contacting the cell or tissue with one or more endoplasmic/sarcoplasmic reticulum calcium channel inhibitors (such as one or more ryanodine receptor antagonists) and one or more gap junction inhibitors to prevent necrosis or cell death in the cell or tissue. The contacting step may further comprise contacting the cell or tissue with one or more plasma membrane calcium channel inhibitors, and/or one or more calcium chelators.

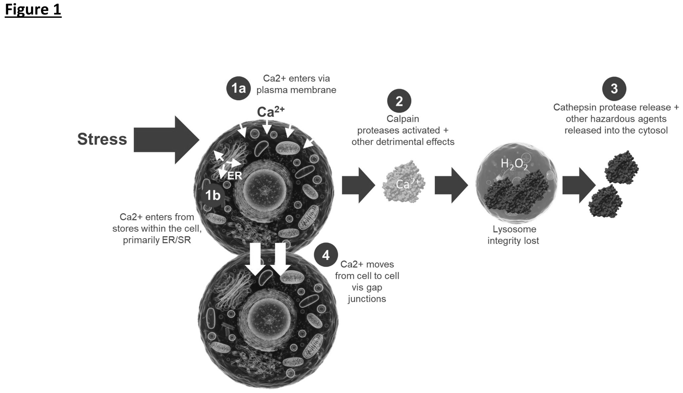

The present invention is based on the discovery that cellular and tissue necrosis are mediated by a cascade of specific intracellular and potentially intercellular events. As shown in the Examples (in particular, see Example 1) and in Figure 1, blocking one or more key steps in the cascade enables one to treat, delay or prevent necrosis and associated downstream conditions, such as tissue or organ damage, which may occur in a subject. The downstream conditions will be dependent on the cells or tissue in which necrosis occurs. For example, if the necrosis occurs in a kidney, a downstream condition may be kidney failure.

Preferably the invention is used to treat or prevent organ damage in a non-neuronal organ or an organ that is not part of the central nervous system (CNS). Thus, the invention may be used to treat or prevent a selection of one, more, or all of the group consisting of an integumentary organ, a skeletal organ, a muscular organ, a circulatory organ, a respiratory organ, a digestive organ, a urinary organ, an immune system organ, an endocrine organ, and a reproductive organ. Thus, the invention may be used to treat or prevent damage in an organ selected from the group comprising or consisting of the heart, the kidneys, the liver, the skin, the spleen, the pancreas, the intestines, the stomach, lungs, bladder, eye, capillaries, joints, tendons, arteries, tongue, diaphragm, ovaries, scrotum, thyroid, adrenal glands, ears, larynx, oesophagus, trachea, ligaments, penis, thymus gland, bones, fallopian tubes, lymph nodes, ureters, bronchi, genitals, pharynx, salivary glands, urethra, gallbladder, lymphatic vessel, placenta, skeletal muscles, uterus, bone marrow, mouth, prostate, seminal vesicles, vulva, bulbourethral glands, hair follicle, mesentery, pineal gland, subcutaneous tissue, veins, colon, mammary glands, pituitary gland, teeth, vagina, cervix, interstitium, nose, parathyroid glands, tonsils, vas deferens, nails, rectum, testes, and vestigial organ. Furthermore, the combinations described herein (such as the combination of one or more endoplasmic/sarcoplasmic reticulum calcium channel inhibitors and one or more gap junction inhibitors) may be used to treat or prevent organ damage in a neuronal organ or an organ that is part of the central nervous system (CNS).

The compounds, combinations, and compositions described herein may be used to treat or prevent tissue damage. For example, a calcium activity inhibitor, a calpain inhibitor, a cathepsin inhibitor and/or a gap junction inhibitor (such as (a) one or more calpain inhibitors in combination with one or more cathepsin inhibitors, or (b) one or more calcium chelators) may be used to treat or prevent tissue damage. Preferably, one or more endoplasmic/sarcoplasmic reticulum calcium channel inhibitors (such as one or more ryanodine receptor antagonists) and one or more gap junction inhibitors (optionally in combination with one or more plasma membrane calcium channel inhibitors and/or one or more calcium chelators) may be used to treat or prevent tissue damage. The tissue damage may be a selection of one, more or all of the group consisting of: burns, multiple organ dysfunction syndrome, organ failure, surgical trauma, bedsores, chemical burns, radiation burns, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure. The tissue damage may be damage associated with infectious disease.

The compounds, combinations, and compositions described herein may be used to treat or prevent autoimmune disease including inflammatory bowel disease (such as Crohn's disease and ulcerative colitis); arthritis; lupus; diabetes; liver disease/injury; kidney disease/injury; and metabolic syndrome.

The combinations (such as the combination of one or more endoplasmic/sarcoplasmic reticulum calcium channel inhibitors and one or more gap junction inhibitors) described herein may also be used to treat or prevent traumatic brain injury (TBI); other forms of head injury including concussion, closed head injury, penetrating head injury, diffuse brain injury, brain contusion caused for example by a depressed skull fracture or a penetrating skull fracture, and cerebral palsy; intracranial hematoma (ICH); hematoma; stroke; brain aneurysm; hypoxic and anoxic brain injury; haemorrhage; meningitis; encephalitis; epilepsy; ataxia; motor neuron disease; multiple system atrophy; progressive supranuclear palsy; spinal cord injury; neurodegenerative disease including Alzheimer's disease (AD), Parkinson's disease (PD), prion diseases, amyotrophic lateral sclerosis (ALS), Huntington's disease, spinal muscular atrophy, spinocerebellar ataxia; autoimmune disease including inflammatory bowel disease (such as Crohn's disease and ulcerative colitis); arthritis; lupus; diabetes; multiple sclerosis (MS); liver disease/injury; kidney disease/injury; pancreatic disease/injury; metabolic syndrome; cardiac arrest; and ischemia.

The compounds, combinations, and compositions described herein may also be used to treat or prevent cancer. For example, the present invention relates to methods of preventing or treating the damage (such as the cellular damage) associated with the development of tumours and/or metastases in a subject.

Necrosis is not reversible, but the inventors have discovered that it can be prevented, attenuated and/or treated by using the invention, e.g., one or more of the inhibitors, combinations, and compositions recited herein. The necrosis may be cellular necrosis and/or tissue necrosis. If necrosis persists, then organ damage (e.g., organ dysfunction or organ failure) will occur. Thus, the organ damage may be associated with or caused by necrosis (e.g., cellular or tissue necrosis).

The inventors have identified that a key step in a cascade of intracellular events that lead to necrosis is a rise in intracellular calcium, which may be caused by an influx of extracellular calcium through calcium channels (e.g., voltage- and/or ligand-gated ion channels) and/or an efflux of calcium from intracellular stores, such as the endoplasmic reticulum (e.g., via ryanodine receptors) as well as through the gap junctions that connect cells to one another. These changes in the cytosolic calcium levels, as well as changes to levels of calcium within internal stores, may further activate receptors/channels (e.g. store-operated calcium channels (SOCC), also called calcium release-activated calcium (CRAC) channels) and thus amplify the increase.

As shown in the Examples (in particular, see Example 1) and the Figures (in particular, see Figure 1), the inventors have identified that a critical step that leads to necrosis is an increase of calcium ions in the cytosol, which may be caused by any one of the three main pathways of calcium ion entry into a cell: via cell surface membrane channels, via gap junctions, and via internal stores in the cell, in particular the endoplasmic reticulum (ER)/sarcoplasmic reticulum (SR). The inventors have identified that inhibiting the ER/SR calcium channels in combination with inhibiting the gap junctions is particularly effective for preventing or reducing an increase in calcium ions. Thus, the present invention provides a combination therapy of one or more endoplasmic/sarcoplasmic reticulum calcium channel inhibitors and one or more gap junction inhibitors. The inventors have found that this combination is synergistic beyond simple additive effects of the endoplasmic/sarcoplasmic reticulum calcium channel inhibitor and the gap junction inhibitor used individually. The inventors have found that also simultaneously blocking calcium ion entry via plasma membranes, and/or by preventing or reducing a rise in the intercellular concentration of calcium ions by using a calcium chelator, further improves the efficacy of the treatment. Thus, the present invention also provides a combination therapy of (i) one or more endoplasmic/sarcoplasmic reticulum calcium channel inhibitors, (ii) one or more gap junction inhibitors, and (iii) one or more plasma membrane calcium channel inhibitors and/or one or more calcium chelators. For example, the combination therapy may use one or more endoplasmic/sarcoplasmic reticulum calcium channel inhibitors, one or more gap junction inhibitors, and one or more plasma membrane calcium channel inhibitors. As another example, the combination therapy may use one or more endoplasmic/sarcoplasmic reticulum calcium channel inhibitors, one or more gap junction inhibitors, and one or more calcium chelators. As another example, the combination therapy may use one or more endoplasmic/sarcoplasmic reticulum calcium channel inhibitors, one or more gap junction inhibitors, one or more plasma membrane calcium channel inhibitors and one or more calcium chelators. It will be appreciated that these preferred combinations can be used for each of the medical uses, uses, and methods described herein.

It will be appreciated that the compounds described herein may be used in a therapeutically effective amount. The term "therapeutically effective amount" refers to an amount of a compound that provides the desired therapeutic result. That result can be the prevention, reduction, amelioration, palliation, lessening, delaying, and/or alleviation of one or more of the signs, symptoms, or causes of a disease, or any other desired alteration of a biological system.

It will also be appreciated that references to compounds and agents may also include pharmaceutically acceptable salts and solvates thereof.

The calcium activity inhibitor referred to herein may be an agent that prevents or reduces a rise in the intracellular concentration of free calcium ions or lowers the intracellular concentration of free (unbound) calcium ions. In other words, the calcium activity inhibitor is an agent that reduces the availability of free intracellular calcium ions. This may be achieved by chelation (using a chelator), blocking the pore of a calcium channel (using a calcium channel blocker), blocking the ligand binding site of a calcium channel (using a calcium channel antagonist), blocking a ryanodine receptor (using an antagonist), or blocking an inositol triphosphate receptor (InsP3R) (using an antagonist). Thus, the calcium activity inhibitor may be a calcium chelator, a calcium channel blocker or antagonist, a ryanodine receptor antagonist, and/or an InsP3R antagonist.

The calcium activity inhibitor may be a calcium chelator (an agent that sequesters free calcium ions). The calcium chelator may be one or more members selected from the group consisting of: EDTA (Ethylenedioxy-diethylene-dinitrilo-tetraacetic acid); derivatives of EDTA (such as EDTA tetrasodium tetrahydrate; EDTA-d12; DMNP-EDTA); EGTA (Ethylene glycol-bis-(2-aminoethyl)-N,N,N',N'-tetraacetic acid); derivatives of EGTA (such as EGTA tetrasodium and DMNP-EGTA); EGTA-AM; NP-EGTA (nitrophenyl-EGTA); NP-EGTA-AM; DTPA (diethylenetriaminepentaacetic acid); HEDTA (N-(2-Hydroxyethyl)ethylenediamine-N,N',N'-triacetic acid Trisodium salt); NTA (Nitrilotriacetic acid); BAPTA ((1,2-bis(o-aminophenoxy)ethane-N,N,N',N'-tetraacetic acid)); a derivative of BAPTA (such as BAPTA tetrasodium; BAPTA tetrapotassium; BAPTA tetracesium salt; 5,5'-Dimethyl BAPTA tetrapotassium; 5-Nitro BAPTA tetramethyl ester; 5-Nitro BAPTA, 5,5'-Dibromo BAPTA tetrapotassium; 5,5'-dimethyl BAPTA; and 5,5'-difluoro BAPTA); BAPTA AM (2-[N-[2-(acetyloxymethoxy)-2-oxoethyl]-2-[2-[2-[bis[2-(acetyloxymethoxy)-2-oxoethyl]amino]phenoxy]ethoxy]anilino]acetic acid acetyloxymethyl ester); a derivative of BAPTA AM (such as 5,5'-dimethyl BAPTA AM; 5,5'-difluoro BAPTA AM; and 5,5'-Dinitro BAPTA AM); citric acid; TPEN (N,N,N',N'-Tetrakis(2-pyridylmethyl)ethylenediamine); DMSA (dimercapto succinic acid); Fura-5F AM; Fura-4F AM; Fluo-3AM; Mag-Fluo-4AM; calcium trinatrium diethylenetriaminepentaacetic acid hydrate; DP-b99; Quin-2AM; Ethylenediaminetetraacetic acid-d16; sodium citrate; calcimycin; 4-bromo A-23187 (4-Bromo-calcimycin); diazo-2; DTPA ITC (1-(*p-*isothiocyanatobenzyl)diethylenetriaminepentaacetic acid); and ionomycin. For example, the calcium chelator may be one or more members selected from the group consisting of: EDTA (Ethylenedioxy-diethylene-dinitrilo-tetraacetic acid); EGTA (Ethylene glycol-bis-(2-aminoethyl)-N,N,N', N'-tetraacetic acid); DTPA (diethylenetriaminepentaacetic acid); HEDTA (N-(2-Hydroxyethyl)ethylenediamine-N, N', N'-triacetic acid Trisodium salt); NTA (Nitrilotriacetic acid); BAPTA ((1,2-bis(o-aminophenoxy)ethane-N,N,N',N'-tetraacetic acid)); BAPTA AM (2-[N-[2-(acetyloxymethoxy)-2-oxoethyl]-2-[2-[2-[bis[2-(acetyloxymethoxy)-2-oxoethyl]amino]phenoxy]ethoxy]anilino]acetic acid acetyloxymethyl ester); citric acid; TPEN (N,N,N',N'-Tetrakis(2-pyridylmethyl)ethylenediamine); and DMSA (dimercapto succinic acid).

Preferably the calcium chelator is specific for calcium (e.g., BAPTA or BAPTA AM). Preferably the calcium chelator is one or more members selected from the group comprising BAPTA, BAPTA AM, EGTA, EDTA, citric acid and DMSA. Preferably, the calcium chelator is selected from the group comprising EGTA, EDTA and BAPTA AM. Most preferably, the calcium chelator is selected from DTPA, BAPTA AM, sodium citrate, and citric acid.

The calcium activity inhibitor may be an agent that modulates intracellular concentration of free calcium ions. Thus, the calcium activity inhibitor may be a calcium modulator, such as calreticulin (also known as calregulin) or a stromal interaction molecule (STIM).

The calcium channel blocker referred to herein may prevent a rise in the intracellular concentration of free calcium ions by blocking voltage-dependent Ca²⁺ channels, orai channels and/or store-operated calcium channels. The calcium channel blocker may block voltage-dependent Ca²⁺ channels including one or more selected from the group consisting of the L-, N-, T-, P/Q-, and R-type. Preferably, the calcium channel blocker blocks L-type, N-type, T-type, P-type, or Q-type voltage gated calcium channels. Most preferably, the calcium channel blocker blocks L-type voltage gated calcium channels. The calcium channel blocker may be a dihydropyridine, a non-dihydropyridine or a gabapentinoid. Dihydropyridines block L-type voltage gated calcium channels. The non-dihydropyridine may be a phenylalkylamine or a benzothiazepine. The non-dihydropyridine may be one or more members selected from the group comprising or consisting of: diltiazem, mibefradil, bepridil, fendiline, flunarizine, fluspirilene, gabapentin, and pregabalin.

The endoplasmic/sarcoplasmic reticulum calcium channel inhibitors referred to herein are preferably one or more ryanodine receptor antagonists. It will be appreciated that endoplasmic/sarcoplasmic reticulum inhibitors are also referred to herein as endoplasmic reticulum/sarcoplasmic reticulum (ER/SR) calcium channel inhibitors.

The ryanodine receptor antagonist referred to herein may be one or more members selected from the group comprising or consisting of: dantrolene, DHBP dibromide, cis-Ned 19, trans-Ned 19, ryanodine, SKF 86365 hydrochloride, ruthenium red, procaine, and tetracaine. Preferably the ryanodine receptor antagonist is dantrolene, ryanodine, trans-Ned 19, cis-Ned 19, procaine or ruthenium red. Preferably the ryanodine receptor antagonist is dantrolene and/or ryanodine. Most preferably the ryanodine receptor antagonist is dantrolene. The ryanodine receptor antagonist may be a xestospongin, including a hydroxylated xestospongin.

The InsP3R antagonist referred to herein may be one or more members selected from the group comprising or consisting of: a macrocyclic 1-oxaquinolizidine, 2-aminoethoxydiphenyl borate, an aromatic polyphosphate, and a tetrakisphosphate (e.g., a myo-Inositol 1,3,4,5-tetrakisphosphate). The aromatic polyphosphate or derivatives thereof may be benzene 1,2,4-trisphosphate [Bz(1,2,4)P3]; biphenyl derivative BiPh(2,3',4,5',6)P5; or a dimeric benzene phosphate. The macrocyclic 1-oxaquinolizidine may be a xestospongin (such as xestospongin A, B, C or D), araguspongine B, araguspongine C, 7S-Hydroxyxestospongin A (7-OHXeA); and demethylxestospongin B (DMXeB). The InsP3R antagonist referred to herein may be heparin, caffeine, IP3R-Binding protein released with Inositol 1,4,5-Trisphosphate (IRBIT), or ci-IP3/PM. Preferably the InsP3R antagonist is heparin, caffeine, 2-APB (2-aminoethoxydiphenyl borate), xestospongin A, xestospongin B, xestospongin C or xestospongin D.

A calpain is a member of a family of calcium-activated cysteine protease proteolytic enzymes. They comprise an 80kD catalytic subunit and a 20kD regulatory subunit that stabilises the catalytic subunit. Members of the family are listed in the table below.

**Table 1 - Calpain family members**

| Gene | Protein | Aliases |
|---|---|---|
| CAPN1 | Calpain 1 | Calpain-1 large subunit, Calpain mu-type |
| CAPN2 | Calpain 2 | Calpain-2 large subunit, Calpain m-type |
| CAPN3 | Calpain 3 | |
| CAPN5 | Calpain 5 | |
| CAPN6 | Calpain 6 | CAPNX, Calpamodulin |
| CAPN7 | Calpain 7 | palBH |
| CAPN8 | Calpain 8 | |
| CAPN9 | Calpain 9 | |
| CAPN10 | Calpain 10 | |
| CAPN11 | Calpain 11 | |
| CAPN12 | Calpain 12 | |
| CAPN13 | Calpain 13 | |
| CAPN14 | Calpain 14 | |
| CAPN16/C6orf103 | Calpain 16 | |
| SOLH | Calpain 15 | Sol H (homolog of the drosophila gene sol) |
| CAPNS1 | Calpain small subunit 1 | Calpain 4 |
| CAPNS2 | Calpain small subunit 2 | |

Calpains tend to be located in the cytoplasm and the nucleus. Consequently, a rise in intracellular calcium causes activation of calpains and thus leads to autolysis. Thus, a calpain inhibitor may be any agent that inhibits calpain enzyme proteolytic activity. The calpain inhibitor may block the active site of the enzyme. The calpain inhibitor may allosterically inhibit the proteolytic activity of the enzyme. Preferably the calpain inhibitor blocks calpain 1 (mu-type) and/or calpain 2 (m-type). The calpain inhibitor referred to herein may be one or more members selected from the group comprising or consisting of: a calpastatin, MG101, MG132, vidupiprant, an α-mercaptoacrylate, a 5-azolone, a carboxamide and a α-helical cysteine protease inhibitor. The calpain inhibitor may be an agent that binds (specifically) to the calpain hexapeptide, CYGRKK; or the calpain 5-mer peptide CYGAK. The calpain inhibitor may be an agent that also inhibits cathepsins, such as E64 ([L-trans-3-Carboxyoxirane-2-carbonyl]-L-Leu-agmatine), E64d, or calpeptin. It will be understood that agents that inhibit calpains and cathepsins may also be referred to as "non-specific calpain/cathepsin inhibitors". The calpain inhibitor may be an agent that is specific for calpains, such as MG101, MG132, or PD150606. The calpain inhibitor may be MDL 28170. For example, the calpain inhibitor may be MG101, vidupiprant, calpeptin, E64, PD150606 or MDL 28170. Calpain inhibition may be measured by cleavage of the substrate, Ac-LLY-AFC, which emits blue light (Amax = 400 nm) upon cleavage of the substrate by calpain. Free AFC emits a yellow-green fluorescence (Amax = 505 nm), which can be quantified using a fluorometer or a fluorescence plate reader. Comparison of the fluorescence intensity from a treated sample with a normal control allows determination of the changes in calpain activity.

A cathepsin inhibitor may be any agent that inhibits cathepsin enzyme activity. The cathepsin inhibitor may block the active site of the enzyme. The cathepsin inhibitor may allosterically inhibit the activity of the enzyme. Preferably the cathepsin inhibitor inhibits cathepsins L, B, and/or D. The cathepsin inhibitor referred to herein may be one or more members selected from the group comprising: a cystatin, a triterpene, and a thiosemicarbazone. The cathepsin inhibitor may be one or more of dexamethasone, rifampicin, E64, E64d, calpeptin, CA 074, SID26681509, L006235, bafilomycin, aloxistatin, astaxanthin, chloroquine, clofazimine, dec-RVKR, or dexamethasone. The cathepsin inhibitor may be an agent that also inhibits calpain enzymes, such as E64, E64d, or calpeptin. The cathepsin inhibitor may be an agent that is specific for cathepsins, such as CA 074 ([L-3-*trans*-(Propylcarbamoyl)oxirane-2-carbonyl]-L-isoleucyl-L-proline Methyl Ester), SID26681509, L006235, or bafilomycin. Cathepsin inhibition may be measured by performing a fluorometric assay with a preferred substrate. For example, the sequence of a cathepsin-B substrate is RR labelled with AFC (amino-4-trifluoromethyl coumarin). Cell lysates or other samples that contain cathepsin-B will cleave the synthetic substrate RR-AFC to release free AFC. The released AFC can easily be quantified using a fluorometer or fluorescence plate reader at Ex/Em 400/505 nm. The sequence of a cathepsin-D substrate is GKPILFFRLK(Dnp)-D-R-NH2) labelled with MCA. Cell lysates or other samples that contain cathepsin-D will cleave the synthetic substrate to release fluorescence, which can then easily be quantified using a fluorometer or fluorescence plate reader at Ex/Em = 328/460 nm.

For example, a combination of one or more calpain inhibitors and one or more cathepsin inhibitors can be used, such as PD150606 in combination with dexamethasone, or PD150606 in combination with rifampicin.

In embodiments of the present invention which comprise the combination of one or more ER/SR endoplasmic/sarcoplasmic reticulum calcium channel inhibitors and one or more gap junction inhibitors, the composition or combination may not further comprise a calpain inhibitor and/or may not further comprise a cathepsin inhibitor.

Gap junctions are intercellular channels that permit cell-cell transfer of ions (such as Ca²⁺) and small molecules. Thus, a rise in intracellular calcium in one cell may spread to neighbouring cells through corresponding gap junctions and thus cause necrosis in neighbouring cells. Thus, a gap junction inhibitor may be used to treat or prevent necrosis. In particularly preferred embodiments of the invention, one or more gap junction inhibitors are used in combination with one or more endoplasmic/sarcoplasmic reticulum calcium channel inhibitors to treat or prevent necrosis.

The gap junction inhibitor referred to herein may be a product of vitamin A (such as retinoic acid, glycyrrhetinic acid and its derivative carbenoxolone), long-chain alcohols (such as heptanol and octanol), halogenated general volatile anaesthetics (such as halothane), fatty acids (such as linoleic acid, arachidonic acid and oleic acid), fatty acid amides (such as oleamide), fenamates (arylaminobenzoates, such as flufenamic acid, niflumic acid and meclofenamic acid), quinine, quinidine and quinine-derivatives (such as mefloquine). The gap junction inhibition may be a fatty acid (preferably a fatty acid containing 13-21 carbons), a polyamine or a cyclodextrin. The gap junction inhibitor referred to herein may be a pannexin inhibitor and/or a connexin inhibitor, preferably a connexin inhibitor. The gap junction inhibitor referred to herein may be a retinoid which is a derivative of vitamin A.

Preferably, the gap junction inhibitor referred to herein may be selected from one or more (e.g., one or two) of 18α-glycyrrhetinic acid, 18β-glycyrrhetinic acid, carbenoxolone disodium, heptanol, octanol, halothane, oleic acid, oleamide, linoleic acid, arachidonic acid, palmitoleic acid, flufenamic acid, myristic acid, lauric acid, quinine, quinidine, dihydroquinidine, mefloquine, meclofenamic acid, probenecid, niflumic acid, flufenamic acid, astaxanthin, and retinoic acid. Preferably, the gap junction inhibitor is one or more (e.g., one or two) of retinoic acid, oleic acid, linoleic acid, octanol, heptanol, arachidonic acid, palmitoleic acid, flufenamic acid, myristic acid, halothane, astaxanthin and quinine. Preferably, the gap junction inhibitor is one or more of retinoic acid, oleic acid, linoleic acid, octanol and heptanol. Most preferably, the gap junction inhibitor is one or more of retinoic acid, oleic acid and linoleic acid.

Where there are two or more gap junction inhibitors, the gap junction inhibitor may be two or more (e.g., two) of retinoic acid, oleic acid, linoleic acid, octanol, heptanol, arachidonic acid, palmitoleic acid, flufenamic acid, myristic acid, halothane, astaxanthin and quinine. Preferably, the gap junction inhibitor is two of heptanol, octanol, oleic acid, retinoic acid, linoleic acid, palmitoleic acid, astaxanthin and quinine. For example, it may be retinoic acid in combination with one or more of oleic acid, linoleic acid, octanol and heptanol, or it may be octanol in combination with one or more of retinoic acid, oleic acid, linoleic acid, and heptanol. As another preferred example, the gap junction inhibitor may be two or more of retinoic acid, oleic acid and linoleic acid.

Determination of the transfer of fluorescent dye tracers between adjacent cells via techniques like fluorescence recovery after photobleaching (FRAP) or flow cytometry can be used to as an assay to measure gap junction inhibition.

The plasma membrane calcium channel inhibitors referred to herein may be one or more of dihydropyridines, nondihydropyridines (such as phenylalkylamines and benzothiazepines), non-selective calcium channel inhibitors and gabapentinoids. The plasma membrane calcium channel inhibitors may be selected from one or more of amlodipine, aranidipine, azelnidipine, barnidipine, benidipine, cilnidipine, clevidipine, efonidipine, felodipine, isradipine, lacidipine, lercanidipine, manidipine, nicardipine, nifedipine, nilvadipine, nimodipine, nisoldipine, nitrendipine, pranidipine, fendiline, gallopamil, verapamil, diltiazem, mibefradil, bepridil, flunarizine, fluspirilene, fendiline, gabapentin and pregabalin. Preferably, the plasma membrane calcium channel inhibitors may be selected from one or more of arandipine, clevidipine, efonidipine, felodipine, isradipine, lercanidipine, manidipine, nifedipine, nimodipine, nitrendipine, gallopamil, verapamil, diltiazem, mibefradil, fluspirilene, gabapentin and pregabalin.

Thus, a calcium activity inhibitor, a calpain inhibitor, a cathepsin inhibitor and/or a gap junction inhibitor can be used to treat or prevent necrosis, tissue damage or organ damage in a subject.

The invention may comprise a selection of one or more of a calcium-activity inhibitor, a calpain inhibitor, a cathepsin inhibitor and/or a gap junction inhibitor. The invention may comprise a selection of two or more of a calcium activity inhibitor, a calpain inhibitor, a cathepsin inhibitor and/or a gap junction inhibitor. Thus, the invention may comprise a combination of a calcium activity inhibitor and a cathepsin inhibitor. The invention may comprise a combination of a calcium activity inhibitor and a calpain inhibitor. In a preferred embodiment, the invention may comprise a combination of a cathepsin inhibitor and a calpain inhibitor. In another preferred embodiment, the invention comprises a combination of one or more endoplasmic/sarcoplasmic reticulum calcium channel inhibitors and one or more gap junction inhibitors (optionally also in combination with one or more plasma membrane calcium channel inhibitors and/or one or more calcium chelators).

The invention may comprise one endoplasmic/sarcoplasmic reticulum calcium channel inhibitor and one or more gap junction inhibitors. In one preferred embodiment, the invention may comprise one endoplasmic/sarcoplasmic reticulum calcium channel inhibitor and two or more gap junction inhibitors. For example, the endoplasmic/sarcoplasmic reticulum calcium channel inhibitor may be dantrolene, and the two or more gap junction inhibitors may be selected from heptanol, octanol, oleic acid, retinoic acid, linoleic acid, palmitoleic acid, astaxanthin and quinine. As another example, the endoplasmic/sarcoplasmic reticulum calcium channel inhibitor may be dantrolene, and the two or more gap junction inhibitors may be selected from oleic acid, retinoic acid and linoleic acid (i.e., the two or more gap junction inhibitors may be selected from (i) oleic acid and retinoic acid, (ii) linoleic acid and retinoic acid, and (iii) oleic acid and linoleic acid). As another example, the endoplasmic/sarcoplasmic reticulum calcium channel inhibitor may be dantrolene, and the two or more gap junction inhibitors may be selected from retinoic acid, heptanol and octanol. As another example, the endoplasmic/sarcoplasmic reticulum calcium channel inhibitor may be dantrolene, and the two or more gap junction inhibitors may be selected from (i) retinoic acid and heptanol, and (ii) heptanol and octanol. As another preferred embodiment, the invention may comprise two or more endoplasmic/sarcoplasmic reticulum calcium channel inhibitors and two or more gap junction inhibitors. For example, the endoplasmic/sarcoplasmic reticulum calcium channel inhibitors may be dantrolene and ryanodine, and the two or more gap junction inhibitors may be selected from any of the combinations recited hereinabove (such as two or more of oleic acid, retinoic acid and linoleic acid).

The invention may comprise a selection of three or more of a calcium activity-inhibitor, a calpain inhibitor, a cathepsin inhibitor and/or a gap junction inhibitor. For example, the invention may comprise one or more endoplasmic/sarcoplasmic reticulum calcium channel inhibitors (such as dantrolene), one or more gap junction inhibitors (such as retinoic acid, oleic acid, and/or linoleic acid), and one or more calcium chelators (such as DTPA, BAPTA-AM, sodium citrate, and/or citric acid). Alternatively, the invention may comprise one or more endoplasmic/sarcoplasmic reticulum calcium channel inhibitors (such as dantrolene), one or more gap junction inhibitors (such as retinoic acid, oleic acid, and/or linoleic acid), and one or more plasma membrane calcium channel inhibitors (such as aranidipine, clevidipine, nimodipine, pranidipine, verapamil, nisoldipine, efonidipine, gabapentin, gallopamil and/or felodipine). Alternatively, the invention may comprise one or more endoplasmic/sarcoplasmic reticulum calcium channel inhibitors (such as dantrolene), one or more gap junction inhibitors (such as retinoic acid, oleic acid, and/or linoleic acid), one or more calcium chelators (such as DTPA, BAPTA-AM, sodium citrate, and/or citric acid), and one or more plasma membrane calcium channel inhibitors (such as aranidipine, clevidipine, nimodipine, pranidipine, verapamil, nisoldipine, efonidipine, gabapentin, gallopamil and/or felodipine).

The invention may further comprise an SOCC inhibitor, such as 2-aminoethoxydiphenyl borate, GNF362, Leflunomide, Roflumilast, Teriflunomide, Tolvaptan, Xestospongin C and/or zinc chloride. In some embodiments of the present invention, the composition or combination does not comprise an SOCC inhibitor.

In embodiments in which the invention comprises a calcium activity inhibitor, the invention referred to herein may comprise one or more of the following classes of agent: a calcium chelator, a calcium channel blocker, a calcium channel antagonist, a ryanodine receptor antagonist, and an InsP3R antagonist. The invention may comprise two or more of the following classes of agent: calcium chelator, a calcium channel blocker, a calcium channel antagonist, a ryanodine receptor antagonist, and an InsP3R antagonist. Most preferably, the invention comprises a ryanodine receptor antagonist and a gap junction inhibitor. The invention may comprise three or more of the following classes of agent: a calcium chelator, a calcium channel blocker, a calcium channel antagonist, a ryanodine receptor antagonist, and an InsP3R antagonist. Preferably the invention comprises two or more different classes agents selected from the group consisting of a calcium chelator, a calcium channel blocker, a calcium channel antagonist, a ryanodine receptor antagonist, and an InsP3R antagonist. Preferably, the invention comprises a calcium chelator, a ryanodine receptor antagonist, and a gap junction inhibitor.

In one embodiment, a calcium activity inhibitor, a calpain inhibitor, a cathepsin inhibitor and/or a gap junction inhibitor may be used to treat or prevent necrosis in a subject. Thus, a calcium activity inhibitor (e.g., a calcium chelator, a calcium channel blocker or antagonist, a ryanodine receptor antagonist, and/or an InsP3R antagonist) may be used to treat or prevent necrosis. Thus, a calcium chelator may be used to treat or prevent necrosis. A calcium channel blocker or antagonist may be used to treat or prevent necrosis. A ryanodine receptor antagonist may be used to treat or prevent necrosis. An InsP3R antagonist may be used to treat or prevent necrosis. A calpain inhibitor may be used to treat or prevent necrosis in a subject. A cathepsin inhibitor may be used to treat or prevent necrosis in a subject. A calpain inhibitor in combination with a cathepsin inhibitor may be used to treat or prevent necrosis in a subject. A gap junction inhibitor may be used to treat or prevent necrosis. Preferably, one or more endoplasmic/sarcoplasmic reticulum calcium channel inhibitors (preferably one or more ryanodine receptor antagonists) and one or more gap junction inhibitors (optionally also in combination with one or more plasma membrane calcium channel inhibitors and/or one or more calcium chelators) may be used to treat or prevent necrosis.

In one embodiment, a calcium activity inhibitor, a calpain inhibitor, a cathepsin inhibitor and/or a gap junction inhibitor may be used to treat or prevent tissue damage in a subject. Thus, a calcium activity inhibitor (e.g., a calcium chelator, a calcium channel blocker or antagonist, a ryanodine receptor antagonist, and/or an InsP3R antagonist) may be used to treat or prevent tissue damage. Thus, a calcium chelator may be used to treat or prevent tissue damage. A calcium channel blocker or antagonist may be used to treat or prevent tissue damage. A ryanodine receptor antagonist may be used to treat or prevent tissue damage. An InsP3R antagonist may be used to treat or prevent tissue damage. A calpain inhibitor may be used to treat or prevent tissue damage. A cathepsin inhibitor may be used to treat or prevent tissue damage. A calpain inhibitor in combination with a cathepsin inhibitor may be used to treat or prevent tissue damage. A gap junction inhibitor may be used to treat or prevent tissue damage. Preferably, one or more endoplasmic/sarcoplasmic reticulum calcium channel inhibitors (preferably one or more ryanodine receptor antagonists) and one or more gap junction inhibitors (optionally also in combination with one or more plasma membrane calcium channel inhibitors and/or one or more calcium chelators) may be used to treat or prevent tissue damage. The tissue damage may occur in one or more organs referred to herein. The tissue damage may occur in one or more tissues selected from the group comprising or consisting of: (i) epithelial tissue, (e.g., squamous, cuboidal, columnar, simple, stratified, pseudostratified, and specialized tissue); (ii) connective tissue (e.g., collagen, reticular and elastic, and tissue classified as proper (dense, loose), embryonic (mesenchyme, mucous), and specialized (cartilage, adipose, bone, blood)); and (iii) muscle tissue (e.g., skeletal, cardiac (gap junctions, intercalated discs), smooth, striated, and nonstriated). Therefore, a calcium activity inhibitor, a calpain inhibitor, a cathepsin inhibitor and/or a gap junction inhibitor may be used to treat or prevent tissue damage in epithelial tissue, connective tissue, and/or muscle tissue. Therefore, one or more endoplasmic/sarcoplasmic reticulum calcium channel inhibitors and one or more gap junction inhibitors may be used to treat or prevent tissue damage in epithelial tissue, connective tissue, and/or muscle tissue. This combination therapy may also be used to treat or prevent tissue damage in nervous tissue (e.g., neurons (soma, dendrites, axons, ganglia (PNS), nuclei (CNS)) and/or glia (astrocytes, oligodendrocytes, Schwann cells and microglia).

In one embodiment, a calcium activity inhibitor, a calpain inhibitor, a cathepsin inhibitor and/or a gap junction inhibitor may be used to treat or prevent organ damage in a subject. Thus, a calcium activity inhibitor (a calcium chelator, a calcium channel blocker or antagonist, a ryanodine receptor antagonist, and/or an InsP3R antagonist) may be used to treat or prevent organ damage. Thus, a calcium chelator may be used to treat or prevent organ damage. A calcium channel blocker or antagonist may be used to treat or prevent organ damage. A ryanodine receptor antagonist may be used to treat or prevent organ damage. An InsP3R antagonist may be used to treat or prevent organ damage. A calpain inhibitor may be used to treat or prevent organ damage. A cathepsin inhibitor may be used to treat or prevent organ damage. A calpain inhibitor in combination with a cathepsin inhibitor may be used to treat or prevent organ damage. A gap junction inhibitor may be used to treat or prevent organ damage. One or more endoplasmic/sarcoplasmic reticulum calcium channel inhibitors (preferably one or more ryanodine receptor antagonists) and one or more gap junction inhibitors (optionally also in combination with one or more plasma membrane calcium channel inhibitors and/or one or more calcium chelators) may be used to treat or prevent organ damage. The organ damage may be multiorgan failure. The organ damage may occur in one or more organs selected from the group comprising the heart, the kidneys, the liver, the skin, the spleen, the pancreas, the intestines, the stomach, lungs, bladder, eye, capillaries, joints, tendons, arteries, tongue, diaphragm, ovaries, scrotum, thyroid, adrenal glands, ears, larynx, oesophagus, trachea, ligaments, penis, thymus gland, bones, fallopian tubes, lymph nodes, ureters, bronchi, genitals, pharynx, salivary glands, urethra, gallbladder, lymphatic vessel, placenta, skeletal muscles, uterus, bone marrow, mouth, prostate, seminal vesicles, vulva, bulbourethral glands, hair follicle, mesentery, pineal gland, subcutaneous tissue, veins, colon, mammary glands, pituitary gland, teeth, vagina, cervix, interstitium, nose, parathyroid glands, tonsils, vas deferens, nails, rectum, testes, and vestigial organ. Preferably the invention is used to treat organ damage in a non-neuronal or a non-central nervous system (CNS) organ. Thus, the invention may be used to an integumentary organ, a skeletal organ, a muscular organ, a respiratory organ, a digestive organ, a urinary organ, an immune system organ, an endocrine organ, a reproductive organ. Furthermore, the combinations described herein may be used to treat or prevent organ damage in a neuronal organ or an organ that is part of the central nervous system (CNS). Furthermore, the combinations described herein may be used to treat or prevent organ damage in the brain, spinal cord, and/or the nerves connected to both of these which make up the central nervous system.

In one embodiment, a calcium activity inhibitor (such as a calcium chelator) may be used to treat or prevent a selection of one, more or all of the group consisting of: burns, multiple organ dysfunction syndrome, surgical trauma, bedsores, chemical burns, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure.

In another embodiment, a calpain inhibitor (e.g., a calcium chelator, a calcium channel blocker or antagonist, a ryanodine receptor antagonist, and/or an InsP3R antagonist) may be used to treat or prevent a selection of one, more or all of the group consisting of: burns, multiple organ dysfunction syndrome, surgical trauma, bedsores, chemical burns, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure. Preferably the calpain inhibitor is used in combination with a cathepsin inhibitor.

In another embodiment, a cathepsin inhibitor may be used to treat or prevent a selection of one, more or all of the group consisting of: burns, multiple organ dysfunction syndrome, organ failure, surgical trauma, bedsores, chemical burns, radiation burns, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure. Preferably the cathepsin inhibitor is used in combination with a calpain inhibitor.

In another embodiment, the gap junction inhibitor may be used to treat or prevent a selection of one, more or all of the group consisting of: burns, multiple organ dysfunction syndrome, organ failure, surgical trauma, bedsores, chemical burns, radiation burns, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure.

In another embodiment, one or more endoplasmic/sarcoplasmic reticulum calcium channel inhibitors and one or more gap junction inhibitors (optionally also in combination with one or more plasma membrane calcium channel inhibitors and/or one or more calcium chelators) may be used to treat or prevent a selection of one, more or all of the group consisting of: burns, multiple organ dysfunction syndrome, organ failure, surgical trauma, bedsores, chemical burns, radiation burns, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure.

According to another aspect, there is provided a composition comprising a selection of one or more of a calcium activity-inhibitor, a calpain inhibitor, a cathepsin inhibitor and a gap junction inhibitor.

In one embodiment, the composition is a pharmaceutical composition comprising a pharmaceutically acceptable carrier, and a selection of one or more of a calcium activity-inhibitor (e.g., a calcium chelator, a calcium channel blocker or antagonist, a ryanodine receptor antagonist, and/or an InsP3R antagonist), a calpain inhibitor, a cathepsin inhibitor and a gap junction inhibitor. In one embodiment, the composition comprises a calcium chelator. In one embodiment, the composition comprises a calpain inhibitor and a cathepsin inhibitor.

In another embodiment, there is provided a composition comprising one or more endoplasmic/sarcoplasmic reticulum calcium channel inhibitors and one or more gap junction inhibitors. For example, the composition may comprise one endoplasmic/sarcoplasmic reticulum calcium channel inhibitor and one gap junction inhibitor, or may comprise one endoplasmic/sarcoplasmic reticulum calcium channel inhibitor and two or more gap junction inhibitors, or may comprise two or more endoplasmic/sarcoplasmic reticulum calcium channel inhibitors and two or more gap junction inhibitors. These compositions may further comprise one or more plasma membrane calcium channel inhibitors and/or one or more calcium chelators. For example, the composition may comprise an endoplasmic/sarcoplasmic reticulum calcium channel inhibitor, a gap junction inhibitor, and a plasma membrane calcium channel inhibitor. As another example, the composition may comprise an endoplasmic/sarcoplasmic reticulum calcium channel inhibitor, a gap junction inhibitor, and a calcium chelator. As another example, the composition may comprise an endoplasmic/sarcoplasmic reticulum calcium channel inhibitor, a gap junction inhibitor, a plasma membrane calcium channel inhibitor, and a calcium chelator. These compositions may each further comprise a pharmaceutically acceptable carrier.

In another embodiment, the composition comprises two or more of a calcium activity inhibitor (e.g., a calcium chelator, a calcium channel blocker or antagonist, a ryanodine receptor antagonist, and/or an InsP3R antagonist), a calpain inhibitor, a cathepsin inhibitor and a gap junction inhibitor. In one embodiment, the composition comprises three or more of a calcium activity inhibitor, a calpain inhibitor, a cathepsin inhibitor and a gap junction inhibitor. The invention may comprise two or more different classes of agent selected from the group consisting of a calcium chelator, a calcium channel blocker, a calcium channel antagonist, a ryanodine receptor antagonist, and an InsP3R antagonist.

The invention may comprise one or more of a calcium chelator, a calcium channel blocker, a calcium channel antagonist, a ryanodine receptor antagonist, and an InsP3R antagonist. The invention may comprise two or more of a calcium chelator, a calcium channel blocker, a calcium channel antagonist, a ryanodine receptor antagonist, and an InsP3R antagonist. The invention may comprise three or more of a calcium chelator, a calcium channel blocker, a calcium channel antagonist, a ryanodine receptor antagonist, and an InsP3R antagonist. Preferably the invention comprises two or more or three or more different subclasses of agent selected from the group consisting of a calcium chelator, a calcium channel blocker, a calcium channel antagonist, a ryanodine receptor antagonist, and an InsP3R antagonist.

The composition may be formulated as a powder, a liquid, an encapsulated tablet, or an unencapsulated tablet. The composition may be incorporated into a food product, or provided as a food additive or supplement.

Exposure of skin tissue to a trigger may cause it experience necrosis. A composition or an inhibitor recited herein may be used to prevent necrosis in skin (tissue).

The compounds, combinations and compositions described herein can be administered via any suitable route known in the art. Thus, the compounds, combinations and compositions of the present invention can be administered by injection, infusion, continuous infusion, intravenously, intradermally, intraarterially, intraperitoneally, intralesionally, intravitreally, intravaginally, intrarectally, topically, intratumourally, intramuscularly, intraperitoneally, subcutaneously, subconjunctivaly, sublingually, intravesicularlly, mucosally, intrapericardially, intraumbilically, intraocularally, orally, intracranially, intraarticularly, intraprostaticaly, intrapleurally, intratracheally, intranasally, topically, locally, via inhalation (e.g. aerosol inhalation), via a catheter, via a lavage, buccally, rectally, vaginally, via the ocular route, via the otic route, nasally, by nebulization, cutaneously, systemically, transdermally, or by other method or any combination of the foregoing as would be known to one of ordinary skill in the art.

Each of the compounds, combinations and compositions described herein may be provided as a pharmaceutical composition, which may further comprise a pharmaceutically acceptable carrier. "Pharmaceutically acceptable carrier" includes any and all solvents, dispersion media, coatings, surfactants, antioxidants, preservatives (e.g., antibacterial agents, antifungal agents), isotonic agents, absorption delaying agents, salts, drug stabilizers, gels, binders, excipients, disintegration agents, lubricants, sweetening agents, flavouring agents, dyes, such like materials and combinations thereof, as would be known to one of ordinary skill in the art.

Each of the compounds described herein may be provided as a pharmaceutical composition, wherein the pH of the pharmaceutical composition has been adjusted. For example, any acidic compounds described herein (such as citric acid) may require the pH of the composition to be adjusted.

### Cosmetic compositions

Each of the compounds, combinations and compositions described herein may be provided as a cosmetic composition. The cosmetic compositions may be used for the cosmetic treatment of skin ageing, such as treating or preventing one or more signs of skin aging, such as the presence of fine lines and wrinkles, loss of elasticity, uneven skin and blotchiness.

Thus, according to another aspect, there is provided a cosmetic composition comprising a calcium activity inhibitor, a calpain inhibitor, a cathepsin inhibitor and/or a gap junction inhibitor; and a cosmetically acceptable carrier. For example, the cosmetic composition may comprise one or more calcium chelators, optionally in a relatively high dose (such as the doses recited hereinbelow in aspect A).

In another preferred aspect, there is provided a cosmetic composition comprising one or more endoplasmic/sarcoplasmic reticulum calcium channel inhibitors (such as one or more ryanodine receptor antagonists) and one or more gap junction inhibitors, optionally further comprising one or more plasma membrane calcium channel inhibitors and/or one or more calcium chelators.

In another embodiment, the composition comprises two or more of a calcium activity inhibitor (e.g., a calcium chelator, a calcium channel blocker or antagonist, a ryanodine receptor antagonist, and/or an InsP3R antagonist), a calpain inhibitor, a cathepsin inhibitor and a gap junction inhibitor. For example, the composition may comprise a calpain inhibitor and a cathepsin inhibitor. In one embodiment, the composition comprises three or more of a calcium activity inhibitor, a calpain inhibitor, a cathepsin inhibitor and a gap junction inhibitor. The invention may comprise two or more different classes of agent selected from the group consisting of a calcium chelator, a calcium channel blocker, a calcium channel antagonist, a ryanodine receptor antagonist, and an InsP3R antagonist.

The invention may comprise one or more of a calcium chelator, a calcium channel blocker, a calcium channel antagonist, a ryanodine receptor antagonist, and an InsP3R antagonist. The invention may comprise two or more of a calcium chelator, a calcium channel blocker, a calcium channel antagonist, a ryanodine receptor antagonist, and an InsP3R antagonist. The invention may comprise three or more of a calcium chelator, a calcium channel blocker, a calcium channel antagonist, a ryanodine receptor antagonist, and an InsP3R antagonist. Preferably the invention comprises two or more or three or more different subclasses of agent selected from the group consisting of a calcium chelator, a calcium channel blocker, a calcium channel antagonist, a ryanodine receptor antagonist, and an InsP3R antagonist.

The cosmetic composition may be a liquid, a cream, or a powder. The cosmetic composition may be for topical use (e.g., use on skin).

According to another aspect, there is provided a method of making a cosmetic composition, the method comprising: contacting a selection of one or more of a calcium activity inhibitor, a calpain inhibitor, a cathepsin inhibitor and/or a gap junction inhibitor with a cosmetically acceptable carrier to create a cosmetic composition. In another preferred aspect, there is provided a method of making a cosmetic composition, the method comprising: contacting one or more endoplasmic/sarcoplasmic reticulum calcium channel inhibitors and one or more gap junction inhibitors with a cosmetically acceptable carrier to create a cosmetic composition. The method may further comprise contacting one or more plasma membrane calcium channel inhibitors and/or one or more calcium chelators.

In another embodiment, the composition comprises two or more of a calcium activity inhibitor (e.g., a calcium chelator, a calcium channel blocker or antagonist, a ryanodine receptor antagonist, and/or an InsP3R antagonist), a calpain inhibitor, a cathepsin inhibitor and a gap junction inhibitor. For example, the composition may comprise a calpain inhibitor and a cathepsin inhibitor. In one embodiment, the composition comprises three or more of a calcium activity inhibitor, a calpain inhibitor, a cathepsin inhibitor and a gap junction inhibitor. The invention may comprise two or more different classes of agent selected from the group consisting of a calcium chelator, a calcium channel blocker, a calcium channel antagonist, a ryanodine receptor antagonist, and an InsP3R antagonist.

The invention may comprise one or more of a calcium chelator, a calcium channel blocker, a calcium channel antagonist, a ryanodine receptor antagonist, and an InsP3R antagonist. The invention may comprise two or more of a calcium chelator, a calcium channel blocker, a calcium channel antagonist, a ryanodine receptor antagonist, and an InsP3R antagonist. The invention may comprise three or more of a calcium chelator, a calcium channel blocker, a calcium channel antagonist, a ryanodine receptor antagonist, and an InsP3R antagonist. Preferably the invention comprises two or more or three or more different subclasses of agent selected from the group consisting of a calcium chelator, a calcium channel blocker, a calcium channel antagonist, a ryanodine receptor antagonist, and an InsP3R antagonist. Most preferably, the invention comprises one or more endoplasmic/sarcoplasmic reticulum calcium channel inhibitors and one or more gap junction inhibitors, and optionally further comprises one or more plasma membrane calcium channel inhibitors and/or one or more calcium chelators.

According to another aspect, there is provided a method of using a cosmetic composition, the method comprising: applying a cosmetic composition according to the invention to the skin of a subject.

### Food preservatives and food products

Each of the compounds, combinations and compositions described herein may be provided as a food preservative.

As food ages the cells within it eventually begin to die due to necrosis. Thus, a composition disclosed herein may be used to prevent necrosis of food, and thus prolong its viability.

According to another aspect, there is provided a food preservative comprising a selection of one or more of a calcium activity inhibitor, a calpain inhibitor, a cathepsin inhibitor and a gap junction inhibitor. The food preservative may comprise one or more calcium chelators, preferably wherein the calcium chelator(s) are used at relatively high doses (such as the doses recited hereinbelow in **aspect A**).

According to another preferred aspect, there is provided a food preservative comprising one or more endoplasmic/sarcoplasmic reticulum calcium channel inhibitors (such as one or more ryanodine receptor antagonists) and one or more gap junction inhibitors, and optionally further comprising one or more plasma membrane calcium channel inhibitors and/or one or more calcium chelators.

According to another preferred aspect, there is provided a food preservative comprising a combination of over-the-counter compounds, such as a combination of any of the over-the-counter compounds described herein. For example, the food preservative may comprise two or more of citric acid, arachidonic acid, palmitoleic acid, quinine, oleic acid, linoleic acid, retinoic acid and astaxanthin. For example, the food preservative may comprise two or more of oleic acid, linoleic acid, retinoic acid and astaxanthin. For example, the food preservative may comprise three of citric acid, arachidonic acid, palmitoleic acid, quinine, oleic acid, linoleic acid, retinoic acid and astaxanthin. For example, the food preservative may comprise three of oleic acid, linoleic acid, retinoic acid and astaxanthin, such as linoleic acid, retinoic acid and astaxanthin.

According to another aspect, there is provided a food product comprising a food preservative according to the invention. The food product may be for use as, for example, a sport supplement.

The food preservative may comprise two or more of a calcium activity inhibitor (e.g., a calcium chelator, a calcium channel blocker or antagonist, a ryanodine receptor antagonist, and/or an InsP3R antagonist), a calpain inhibitor, a cathepsin inhibitor and a gap junction inhibitor. The food preservative may preferably comprise a calpain inhibitor and a cathepsin inhibitor. In one embodiment, the food preservative comprises three or more of a calcium activity inhibitor, a calpain inhibitor, a cathepsin inhibitor and a gap junction inhibitor. The invention may comprise two or more different classes of agent selected from the group consisting of a calcium chelator, a calcium channel blocker, a calcium channel antagonist, a ryanodine receptor antagonist, and an InsP3R antagonist.

The invention may comprise one or more of a calcium chelator, a calcium channel blocker, a calcium channel antagonist, a ryanodine receptor antagonist, and an InsP3R antagonist. The invention may comprise two or more of a calcium chelator, a calcium channel blocker, a calcium channel antagonist, a ryanodine receptor antagonist, and an InsP3R antagonist. The invention may comprise three or more of a calcium chelator, a calcium channel blocker, a calcium channel antagonist, a ryanodine receptor antagonist, and an InsP3R antagonist. Preferably the invention comprises two or more or three or more different subclasses of agent selected from the group consisting of a calcium chelator, a calcium channel blocker, a calcium channel antagonist, a ryanodine receptor antagonist, and an InsP3R antagonist.

The food product may be any foodstuff that is capable of being eaten or drunk.

According to another aspect, there is provided a method of storing a food product, the method comprising contacting a food product with a food preservative according to the invention to store the food product. Preferably, there is provided a method of storing a food product, the method comprising contacting a food product with a food preservative comprising one or more endoplasmic/sarcoplasmic reticulum calcium channel inhibitors (such as one or more ryanodine receptor antagonists) and one or more gap junction inhibitors (and optionally further comprising one or more plasma membrane calcium channel inhibitors and/or one or more calcium chelators) to store the food product.

### Methods for preserving live tissue or an organ

Each of the compounds, combinations and compositions described herein may be used in a method for preserving a live tissue or an organ.

According to another aspect, there is provided a method of preserving a live tissue or an organ, the method comprising storing or culturing the tissue or organ in a culture media comprising a calcium activity inhibitor, a calpain inhibitor, a cathepsin inhibitor and/or a gap junction inhibitor to preserve the live tissue or organ.

According to a preferred aspect, there is provided a method of preserving a live tissue or an organ, the method comprising storing or culturing the tissue or organ in a culture media comprising one or more calcium chelators (such as DTPA, sodium citrate, citric acid, and/or BAPTA-AM) to preserve the live tissue or organ.

The calcium chelator(s) may be used at relatively high doses to effectively prevent necrosis and hence preserve the live tissue or organ. The inventors have found that using calcium chelators as a monotherapy is particularly effective for *ex-vivo* and *in vitro* uses, particularly in relatively high doses, but that these relatively high doses may be less suitable for *in vivo* uses due to potential associated toxicity issues *in vivo.*

The term "relatively high dose" used herein for a compound or agent may refer to a dose which is about four times, or about five times, or about six times, or about seven times, or preferably about eight times, the dose typically used in the art for said compound or agent for said use.

For example, the term "relatively high dose" used herein may refer to one of the following doses (and these doses are referred to herein as "**aspect A**"):
- When the calcium chelator is BAPTA or a derivative of BAPTA (such as BAPTA tetrasodium; BAPTA tetrapotassium; BAPTA tetracesium salt; 5,5'-Dimethyl BAPTA tetrapotassium; 5-Nitro BAPTA tetramethyl ester; 5-Nitro BAPTA, 5,5'-Dibromo BAPTA tetrapotassium; 5,5'-dimethyl BAPTA; 5,5'-difluoro BAPTA), the chelator may be present (e.g., may be in the culture media) in an amount of at least about 50 µM, or at least about 60 µM, or at least about 70 µM, or at least about 80 µM, or at least about 90 µM, or at least about 100 µM.

- When the calcium chelator is BAPTA-AM or a derivative of BAPTA-AM (such as 5,5'-dimethyl BAPTA AM; 5,5'-difluoro BAPTA AM; 5,5'-Dinitro BAPTA AM), the chelator may be present (e.g., may be in the culture media) in an amount of at least about 10 µM, or at least about 15 µM, or at least about 20 µM, or at least about 25 µM, for example at least about 50 µM or at least about 100 µM or at least about 150 µM.
- When the calcium chelator is EGTA or a derivative of EGTA (such as EGTA tetrasodium or DMNP-EGTA), the chelator may be present (e.g., may be in the culture media) in an amount of at least about 500 µM, or at least about 1000 µM, or at least about 1500 µM, or at least about 2000 µM, or at least about 2250 µM, or at least about 2500 µM.
- When the calcium chelator is EGTA-AM, NP-EGTA, or NP-EGTA-AM, the chelator may be present (e.g., may be in the culture media) in an amount of at least about 10 µM, or at least about 20 µM, or at least about 50 µM.
- When the calcium chelator is EDTA or a derivative of EDTA (such as EDTA tetrasodium tetrahydrate or EDTA-d12), the chelator may be present (e.g., may be in the culture media) in an amount of at least about 500 µM, or at least about 750 µM, or at least about 850 µM.
- When the calcium chelator is Fura-5F AM or Fura-4F AM, the chelator may be present (e.g., may be in the culture media) in an amount of at least about 20 µM, or at least about 30 µM, or at least about 50 µM.
- When the calcium chelator is Fluo-3AM or Mag-Fluo-4AM, the chelator may be present (e.g., may be in the culture media) in an amount of at least about 20 µM, or at least about 30 µM, or at least about 50 µM.
- When the calcium chelator is Calcium trinatrium diethylenetriaminepentaacetic acid hydrate, the chelator may be present (e.g., may be in the culture media) in an amount of at least about 500 µM, or at least about 750 µM, or at least about 850 µM.
- When the calcium chelator is DP-b99, the chelator may be present (e.g., may be in the culture media) in an amount of at least about 20 µM, or at least about 30 µM, or at least about 50 µM.
- When the calcium chelator is Quin-2AM, the chelator may be present (e.g., may be in the culture media) in an amount of at least about 20 µM, or at least about 30 µM, or at least about 50 µM.
- When the calcium chelator is Ethylenediaminetetraacetic acid-d16, the chelator may be present (e.g., may be in the culture media) in an amount of at least about 100 µM, or at least about 125 µM, or at least about 150 µM.
- When the calcium chelator is DTPA, the chelator may be present (e.g., may be in the culture media) in an amount of at least about 200 µM, or at least about 500 µM, or at least about 1000 µM, or at least about 1500 µM, or at least about 2000 µM, or at least about 2500 µM, or at least about 3000 µM, or at least about 3500 µM, or at least about 4000 µM, for example at least about 8000 µM or at least about 10,000 µM.
- When the calcium chelator is HEDTA, the chelator may be present (e.g., may be in the culture media) in an amount of at least about 200 µM, or at least about 500 µM, or at least about 1000 µM.
- When the calcium chelator is NTA, the chelator may be present (e.g., may be in the culture media) in an amount of at least about 200 µM, or at least about 500 µM, or at least about 1000 µM.
- When the calcium chelator is citric acid, the chelator may be present (e.g., may be in the culture media) in an amount of at least about 200 µM, or at least about 500 µM, or at least about 1000 µM.
- When the calcium chelator is sodium citrate, the chelator may be present (e.g., may be in the culture media) in an amount of at least about 200 µM, or at least about 500 µM, or at least about 1000 µM.
- When the calcium chelator is TPEN, the chelator may be present (e.g., may be in the culture media) in an amount of at least about 10 µM, or at least about 20 µM, or at least about 50 µM.
- When the calcium chelator is DMSA, the chelator may be present (e.g., may be in the culture media) in an amount of at least about 50 µM, or at least about 60 µM, or at least about 70 µM, or at least about 80 µM, or at least about 90 µM, or at least about 100 µM.
- When the calcium chelator is calcimycin, the chelator may be present (e.g., may be in the culture media) in an amount of at least about 0.02 µM, or at least about 0.03 µM, or at least about 0.05 µM.
- When the calcium chelator is 4-bromo A-23187, the chelator may be present (e.g., may be in the culture media) in an amount of at least about 20 µM, or at least about 30 µM, or at least about 50 µM.
- When the calcium chelator is diazo-2, the chelator may be present (e.g., may be in the culture media) at a concentration of at least about 200 µM, or at least about 500 µM, or at least about 1000 µM.
- When the calcium chelator is DTPA ITC, the chelator may be present (e.g., may be in the culture media) in an amount of at least about 10 µM, or at least about 20 µM, or at least about 50 µM.
- When the calcium chelator is ionomycin, the chelator may be present (e.g., may be in the culture media) in an amount of at least about 10 µM, or at least about 20 µM, or at least about 50 µM.

According to another preferred aspect, there is provided a method of preserving a live tissue or an organ, the method comprising storing or culturing the tissue or organ in a culture media comprising one or more endoplasmic/sarcoplasmic reticulum calcium channel inhibitors (such as one or more ryanodine receptor antagonists) and one or more gap junction inhibitors. The culture media optionally further comprises one or more plasma membrane calcium channel inhibitors and/or one or more calcium chelators.

According to another preferred aspect, there is provided a method of preserving a live tissue or an organ, the method comprising storing or culturing the tissue or organ in a culture media comprising one or more calpain inhibitors and one or more cathepsin inhibitors.

The methods of preserving a live tissue or an organ discussed herein include methods comprising storing or culturing the tissue or organ at room temperature (such as at around 20 °C). Additionally, or alternatively, the methods comprise storing or culturing the tissue or organ under hypothermic conditions (e.g., a temperature of about 4 °C). Additionally, or alternatively, the methods comprise storing or culturing the tissue or organ via cryopreservation (e.g., at -80 °C, -196 °C or intermediate temperatures).

The methods of preserving a live tissue or an organ discussed herein may comprise (i) simple cold storage preservation (SCS), and/or (ii) machine perfusion (MP) preservation (which may also be called dynamic preservation method). Machine perfusion preservation may comprise hypothermic machine perfusion (HMP), normothermic machine perfusion (NMP) and/or oxygen persufflation (OP).

The culture media used in the method of preserving a live tissue or an organ (preferably an organ) may be any culture media known in the art, in particular the University of Wisconsin (UW) solution which is generally used for organ preservation. This solution may comprise potassium lactobionate (about 100 mM), KH₂PO₄ (about 25 mM), MgSO₄ (about 5 mM), raffinose (about 30 mM), adenosine (about 5 mM), glutathione (about 3 mM), allopurinol (about 1 mM), and hydroxyethyl starch (about 50 g/L).

Thus, the invention may comprise storing the organ in a culture media (such as the UW solution described hereinabove) with one or more endoplasmic/sarcoplasmic reticulum calcium channel inhibitors and one or more gap junction inhibitors, and optionally one or more plasma membrane calcium channel inhibitors and/or optionally one or more calcium chelators.

Also provided herein is a culture media suitable for preserving an organ or tissue, wherein the culture media comprises the University of Wisconsin (UW) solution, one or more endoplasmic/sarcoplasmic reticulum calcium channel inhibitors (such as one or more ryanodine receptor antagonists) and one or more gap junction inhibitors. The culture media optionally further comprises one or more plasma membrane calcium channel inhibitors and/or one or more calcium chelators.

Also provided herein is a culture media suitable for preserving an organ or tissue, wherein the culture media comprises the University of Wisconsin (UW) solution, one or more calpain inhibitors, and one or more cathepsin inhibitors.

Also provided herein is a culture media suitable for preserving an organ or tissue, wherein the culture media comprises the University of Wisconsin (UW) solution and one or more calcium chelators, preferably wherein the calcium chelator(s) are present in the amounts recited hereinabove in **aspect A.**

### Methods of engineering a tissue or an organ

One of the biggest challenges with engineering tissue *in vitro* is the small surface area to volume ratio. Consequently, as large tissues are engineered/grown they begin to develop necrotic cores. An inhibitor, combination or composition recited herein may be used to overcome this problem.

According to another aspect, there is provided a method of engineering a tissue or an organ, the method comprising contacting the organ or tissue with a culture media comprising a calcium activity inhibitor, a calpain inhibitor, a cathepsin inhibitor and/or a gap junction inhibitor.

According to a preferred aspect, there is provided a method of engineering a tissue or an organ, the method comprising contacting the organ or tissue with a culture media comprising one or more calcium chelators (such as citric acid, sodium citrate, DTPA and/or BAPTA-AM).

The calcium chelator(s) may be used at relatively high doses to effectively prevent necrosis and hence maximise the growth and engineering of the tissue or organ. For example, the calcium chelator(s) may be used at the doses recited hereinabove in **aspect A.**

According to another preferred aspect, there is provided a method of engineering a tissue or an organ, the method comprising contacting the organ or tissue with a culture media comprising one or more endoplasmic/sarcoplasmic reticulum calcium channel inhibitors (such as one or more ryanodine receptor antagonists) and one or more gap junction inhibitors. The culture media may further comprise one or more plasma membrane calcium channel inhibitors and/or one or more calcium chelators.

According to another preferred aspect, there is provided a method of engineering a tissue or an organ, the method comprising contacting the organ or tissue with a culture media comprising one or more cathepsin inhibitors and one or more calpain inhibitors.

Contacting may refer to synthesising the organ or tissue in the culture media. Therefore, the method may comprise synthesising the tissue or organ in a culture media comprising a calcium activity inhibitor, a calpain inhibitor, a cathepsin inhibitor and/or a gap junction inhibitor. For example, the method may comprise synthesising the tissue or organ in a culture media comprising one or more calcium chelators (such as citric acid, sodium citrate, DTPA and/or BAPTA-AM). Additionally, or alternatively, the method may comprise synthesising the tissue or organ in a culture media comprising one or more endoplasmic/sarcoplasmic reticulum calcium channel inhibitors and one or more gap junction inhibitors. The culture media may further comprise one or more plasma membrane calcium channel inhibitors and/or one or more calcium chelators. Additionally, or alternatively, the method may comprise synthesising the tissue or organ in a culture media comprising one or more calpain inhibitors and one or more cathepsin inhibitors.

In one embodiment, the method comprises contacting the tissue or organ with a culture media comprising two or more of a calcium activity inhibitor (e.g., a calcium chelator, a calcium channel blocker or antagonist, a ryanodine receptor antagonist, and/or an InsP3R antagonist), a calpain inhibitor, a cathepsin inhibitor and/or a gap junction inhibitor. For example, the combination of a calpain inhibitor and a cathepsin inhibitor may be used. In one embodiment, the method comprises contacting the tissue or organ with a culture media comprising three or more of a calcium activity inhibitor, a calpain inhibitor, a cathepsin inhibitor and/or a gap junction inhibitor. The invention may comprise contacting the tissue or organ with a culture media comprising two or more different classes agents selected from the group consisting of a calcium chelator, a calcium channel blocker, a calcium channel antagonist, a ryanodine receptor antagonist, and an InsP3R antagonist.

The invention may comprise contacting the tissue or organ with a culture media comprising one or more of a calcium chelator, a calcium channel blocker, a calcium channel antagonist, a ryanodine receptor antagonist, and an InsP3R antagonist. The invention may comprise contacting the tissue or organ with a culture media comprising one or more calcium chelators. Additionally, or alternatively, the invention may comprise contacting the tissue or organ with a culture media comprising one or more endoplasmic/sarcoplasmic reticulum calcium channel inhibitors and one or more gap junction inhibitors. The culture media may further comprise one or more plasma membrane calcium channel inhibitors and/or one or more calcium chelators. The invention may comprise contacting the tissue or organ with a culture media comprising two or more of a calcium chelator, a calcium channel blocker, a calcium channel antagonist, a ryanodine receptor antagonist, and an InsP3R antagonist. The invention may comprise contacting the tissue or organ with a culture media comprising three or more of a calcium chelator, a calcium channel blocker, a calcium channel antagonist, a ryanodine receptor antagonist, and an InsP3R antagonist. Preferably the invention comprises contacting the tissue or organ with a culture media comprising two or more or three or more different subclasses of agent selected from the group consisting of a calcium chelator, a calcium channel blocker, a calcium channel antagonist, a ryanodine receptor antagonist, and an InsP3R antagonist.

Preferably, the method comprises contacting the tissue or organ with a culture media comprising a calcium activity inhibitor and a calpain inhibitor. Preferably, the method comprises contacting the tissue or organ with a culture media comprising a calcium activity inhibitor and a cathepsin inhibitor. Preferably, the method comprises contacting the tissue or organ with a culture media comprising a cathepsin inhibitor and a calpain inhibitor. Most preferably, the method comprises contacting the tissue or organ with a culture media comprising one or more endoplasmic/sarcoplasmic reticulum calcium channel inhibitors and one or more gap junction inhibitors, and optionally further comprising one or more plasma membrane calcium channel inhibitors and/or one or more calcium chelators.

The culture media described herein may be any culture media known in the art, in particular the University of Wisconsin (UW) solution which is generally used for organ preservation. This solution may comprise potassium lactobionate (about 100 mM), KH₂PO₄ (about 25 mM), MgSO₄ (about 5 mM), raffinose (about 30 mM), adenosine (about 5 mM), glutathione (about 3 mM), allopurinol (about 1 mM), and hydroxyethyl starch (about 50 g/L).

Thus, the invention may comprise contacting the tissue or organ with a culture media (such as the UW solution described hereinabove) with one or more endoplasmic/sarcoplasmic reticulum calcium channel inhibitors (such as one or more ryanodine receptor antagonists) and one or more gap junction inhibitors, and optionally one or more plasma membrane calcium channel inhibitors and/or optionally one or more calcium chelators.

Alternatively, the invention may comprise contacting the tissue or organ with a culture media (such as the UW solution described hereinabove) with one or more calcium chelators, preferably wherein the one or more calcium chelators are present in the culture media in relatively high doses, such as the amounts recited hereinabove in **aspect A.**

Alternatively, the invention may comprise contacting the tissue or organ with a culture media (such as the UW solution described hereinabove) with one or more calpain inhibitors and one or more cathepsin inhibitors.

### Methods of preserving or culturing blood cells

According to another aspect of the invention, there is provided a method of preserving or culturing blood cells, the method comprising contacting a culture media comprising blood cells with a calcium activity inhibitor, a calpain inhibitor, a cathepsin inhibitor and/or a gap junction inhibitor to preserve or culture the blood cells.

The method may comprise contacting a culture media comprising blood cells with a calpain inhibitor and a cathepsin inhibitor to preserve or culture the blood cells. The method may comprise contacting a culture media comprising blood cells with one or more calcium chelators (preferably at relatively high doses) to preserve or culture the blood cells.

Additionally, or alternatively, there is provided a method of preserving or culturing blood cells, the method comprising contacting a culture media comprising blood cells with one or more endoplasmic/sarcoplasmic reticulum calcium channel inhibitors (such as one or more ryanodine receptor antagonists) and one or more gap junction inhibitors to preserve or culture the blood cells. The culture media may further comprise one or more plasma membrane calcium channel inhibitors and/or one more calcium chelators.

The culture media may be blood, plasma or serum. Preferably the culture media is blood or plasma. The blood cells may be white blood cells (or leukocytes), red blood cells (erythrocytes) and/or platelets. The white blood cells may be a selection of one or more from the group consisting of: neutrophils, monocytes, lymphocytes, granulocytes, and natural killer cells. The lymphocytes may be B lymphocytes or T lymphocytes.

The calcium chelators may be used at relatively high doses to effectively prevent necrosis and hence effectively preserve or culture the blood cells. For example, in methods of preserving or culturing blood cells, the calcium chelator(s) may be used at the doses recited hereinabove in **aspect A.**

### Methods of preserving blood, DNA or fertility samples

According to another aspect of the invention, there is provided a method of preserving blood, DNA, or fertility samples, the method comprising contacting the samples with a calcium activity inhibitor, a calpain inhibitor, a cathepsin inhibitor and/or a gap junction inhibitor to preserve the samples. The method may comprise contacting the samples with a calpain inhibitor and a cathepsin inhibitor. The method may comprise contacting the samples with one or more calcium chelators (preferably at relatively high doses).

Additionally, or alternatively, there is provided a method of preserving blood, DNA or fertility samples, the method comprising contacting the samples with one or more endoplasmic/sarcoplasmic reticulum calcium channel inhibitors (such as one or more ryanodine receptor antagonists) and one or more gap junction inhibitors to preserve the samples. Optionally, the contacting step further comprises contacting the samples with one or more plasma membrane calcium channel inhibitors. Optionally, the contacting step further comprises contacting the samples with one or more calcium chelators.

The calcium chelators may be used at relatively high doses to effectively prevent necrosis and hence effectively preserve blood, DNA or fertility samples. For example, in methods of preserving blood, DNA, or fertility samples, the calcium chelator(s) may be used at the doses recited hereinabove in **aspect A.**

The methods of preserving blood, DNA or fertility samples may be particularly useful for enabling the preservation of biological test(s) and/or biological kit(s).

### Methods of preserving grafts

According to another aspect of the invention, there is provided a method of preserving grafts, the method comprising contacting the graft with a calcium activity inhibitor, a calpain inhibitor, a cathepsin inhibitor and/or a gap junction inhibitor to preserve the graft. The method may comprise contacting the samples with one or more calpain inhibitors and one or more cathepsin inhibitors. Additionally or alternatively, the method may comprise contacting the samples with one or more calcium chelators.

Additionally, or alternatively, there is provided a method of preserving grafts, the method comprising contacting the graft with one or more endoplasmic/sarcoplasmic reticulum calcium channel inhibitors (such as one or more ryanodine receptor antagonists) and one or more gap junction inhibitors to preserve the graft. Optionally, the contacting step further comprises contacting the graft with one or more plasma membrane calcium channel inhibitors. Additionally, or alternatively, the contacting step optionally further comprises contacting the graft with one or more calcium chelators.

It will be appreciated that the compounds, combinations, and compositions described herein protect grafts against necrosis and/or cell death, and thus the grafts can be preserved prior to transplanting these grafts into patients.

The methods of preserving grafts discussed herein include methods wherein said preservation includes cold storage, cryopreservation, or vitrification, followed by thawing and/or washing of the graft. For example, the method may comprise cold storage under hypothermic conditions (e.g., a temperature of about 4 °C). Alternatively, the method may comprise cryopreservation (e.g., at -80 °C, -196 °C or intermediate temperatures).

The calcium chelators may be used at relatively high doses to effectively prevent necrosis and hence effectively preserve grafts. For example, in methods of preserving grafts, the calcium chelator(s) may be used at the doses recited hereinabove in **aspect A.**

### Bioreactors

It will be appreciated that cell death is a major problem in bio-industry, as cell death often occurs within bioreactors due to various stressors. These stresses therefore negatively affect the culture longevity and the overall product yield. Thus, methods of improving the product yield in bioreactors are desired.

According to another aspect of the invention, there is provided a method of preserving or culturing cells during cultivation of cells lines in a bioreactor. The method may comprise contacting the cells with a calcium activity inhibitor, a calpain inhibitor, a cathepsin inhibitor and/or a gap junction inhibitor to preserve or culture the cells in the production bioreactor. For example, the method may comprise contacting the cells with one or more calpain inhibitors and one or more cathepsin inhibitors. For example, the method may comprise contacting the cells with one or more calcium chelators (preferably at relatively high doses, such as the doses recited hereinabove in **aspect A**). Preferably, the method comprises contacting the cells with one or more endoplasmic/sarcoplasmic reticulum calcium channel inhibitors (such as one or more ryanodine receptor antagonists) and one or more gap junction inhibitors to preserve or culture the cells. Optionally, the contacting step further comprises contacting the cells with one or more plasma membrane calcium channel inhibitors and/or one or more calcium chelators.

Thus, also provided herein is a method of increasing bioreactor efficiency by enhancing cell survival and/or function. The method may comprise introducing a calcium activity inhibitor, a calpain inhibitor, a cathepsin inhibitor and/or a gap junction inhibitor to the bioreactor to increase bioreactor efficiency. For example, the method may comprise introducing one or more calcium chelators. For example, the method may comprise introducing one or more calpain inhibitors and one or more cathepsin inhibitors. Preferably, the method may comprise introducing one or more endoplasmic/sarcoplasmic reticulum calcium channel inhibitors (such as one or more ryanodine receptor antagonists) and one or more gap junction inhibitors to the bioreactor to increase bioreactor efficiency. Optionally, one or more plasma membrane calcium channel inhibitors and/or one or more calcium chelators may also be introduced.

The inhibitors referred to herein may be specific for calcium channel pores, calcium channel receptors, calcium ions, calpains, cathepsins and/or gap junctions. The inhibitors referred to herein may be selective for calcium ions. The preferred inhibitors referred to herein are endoplasmic/sarcoplasmic reticulum calcium channel inhibitors and gap junction inhibitors, particularly in combination.

The calcium chelators may be used at relatively high doses to effectively prevent necrosis and hence effectively preserve or culture cells, or increase bioreactor efficiency. For example, in these methods, the calcium chelator(s) may be used at the doses recited hereinabove in **aspect A.**

### Preserving or growing cell cultures

According to another aspect of the invention, there is provided a method of preserving or growing cell cultures, the method comprising contacting a culture media with a calcium activity inhibitor, a calpain inhibitor, a cathepsin inhibitor and/or a gap junction inhibitor to preserve or grow the cells.

The method may comprise contacting a culture media with a calpain inhibitor and a cathepsin inhibitor. The method may comprise contacting a culture media with one or more calcium chelators (preferably at relatively high doses).

Preferably, there is provided a method of preserving or growing cell cultures, the method comprising contacting a culture media with one or more endoplasmic/sarcoplasmic reticulum calcium channel inhibitors (such as one or more ryanodine receptor antagonists) and one or more gap junction inhibitors to preserve or grow the cell cultures. The contacting step may further comprise contacting the culture media with one or more plasma membrane calcium channel inhibitors and/or one or more calcium chelators.

The calcium chelators may be used at relatively high doses to effectively prevent necrosis and hence maximise the preservation or growth of cell cultures. For example, in methods of preserving or culturing cell cultures, the calcium chelator(s) may be used at the doses recited hereinabove in **aspect A.**

### Definitions

The term *"tissue damage"* can refer to (biological) tissue dysfunction and/or (biological) tissue failure. Preferably the tissue damage is within an organ referred to herein. Similarly, the term *"organ damage"* can refer to organ dysfunction and/or organ failure.

The organ referred to herein may be a selection of one more or all of the following group consisting of: the kidneys, the liver, the skin, the spleen, the pancreas, the intestines, the stomach, lungs, bladder, eye, capillaries, joints, tendons, arteries, tongue, diaphragm, ovaries, scrotum, thyroid, adrenal glands, ears, larynx, oesophagus, trachea, ligaments, penis, thymus gland, bones, fallopian tubes, lymph nodes, ureters, bronchi, genitals, pharynx, salivary glands, urethra, gallbladder, lymphatic vessel, placenta, skeletal muscles, uterus, bone marrow, heart, mouth, prostate, seminal vesicles, vulva, bulbourethral glands, hair follicle, mesentery, pineal gland, subcutaneous tissue, veins, colon, mammary glands, pituitary gland, teeth, vagina, cervix, interstitium, nose, parathyroid glands, tonsils, vas deferens, nails, rectum, testes, and vestigial organ.

The tissue referred to herein may be one or more tissues selected from the group comprising or consisting of: epithelial tissue (e.g., squamous, cuboidal, columnar, simple, stratified, pseudostratified, and specialized tissue); (ii) connective tissue (e.g., collagen, reticular and elastic, and tissue classified as proper (dense, loose), embryonic (mesenchyme, mucous), and specialized (cartilage, adipose, bone, blood)); and (iii) muscle tissue (e.g., skeletal, cardiac (gap junctions, intercalated discs), smooth, striated, and nonstriated).

Preferably, the organ referred to herein is a selection of one more or all of the following group consisting of: the kidneys, the liver, the pancreas, the intestines, the stomach, and lungs.

The tissue referred to herein may be from an organ selected from the group consisting of or comprising (i) epithelial tissue, (ii) connective tissue, and (iii) muscle tissue.

The methods according to the invention may be performed *in vitro,* or *in vivo.*

The term *"burn"* can be defined as an injury caused by exposure to heat or flame. A major contributor to cell and tissue damage following heat or flame is cellular necrosis. The term *"multiple organ dysfunction syndrome (MODS)",* previously known as multiple organ failure, can be defined as sequential and progressive organ dysfunction, associated with a sustained, massive inflammatory response, and tissue injury, inflammatory states such as pancreatitis, any form of shock (i.e. a medical condition in which an individual is in a state of cellular and tissue hypoxia due to reduced oxygen delivery, increased oxygen consumption, inadequate oxygen utilization, or a combination of these three processes; this includes different types of shock such as distributive shock, hypovolemic shock, cardiogenic shock, and obstructive shock) and insults such as sepsis. A major contributor to MODS is cellular necrosis. The term *"organ failure"* can be defined as organ dysfunction to such a degree that normal homeostasis cannot be maintained without external clinical intervention. It can be classified by the cause, but when the cause is not known, it can also be classified by whether the onset is chronic or acute. A major contributor to organ failure is cellular necrosis, e.g., kidney failure, liver failure. The term *"surgical trauma"* can be defined as any injury produced by or related to surgery. Necrosis greatly contributes to surgical trauma due to for example hypoxia or mechanical force. The term *"bedsores"* can be defined as damage to an area of the skin caused by constant pressure on the area for a long time. This pressure can lessen blood flow to the affected area, which causes necrosis that then produces the sore. The term *"chemical burn"* can be defined as tissue damage caused by strong acids, drain cleaners, paint thinner, gasoline and other chemical substances. Such chemicals cause cell necrosis that then cause the chemical burn. The term *"radiation burn"* can be defined as tissue damage caused by exposure to radiation, including UV-rays, X-rays or radiation therapy. A major contributor to tissue damage following exposure to radiation is cellular necrosis. The term *"gangrene"* can be defined as a serious condition where a loss of blood supply causes body tissue to die. Necrosis contributes to the death of the tissue in gangrene. The term *"alopecia (hair loss)"* can be defined as the lack or loss of hair from areas of the body where hair is usually found. Necrosis contributes to hair loss. The term *"solar erythema (sun burn)"* can be defined as a short-term skin response to excessive amounts of ultraviolet (UV) radiation, mostly from the sun or from tanning lamps. Necrosis contributes to solar erythema. The term *"acute tubular necrosis"* can be defined as a kidney disorder involving damage to the cells of the kidneys, which can lead to acute kidney failure. Necrosis contributes to acute tubular necrosis. The term *"ulcer"* can be defined as a break in the epithelium, skin, in the lining of an organ, or on the surface of a tissue. Necrosis contributes to an ulcer. The term *"physical injury"* can be defined as damage/injury to the body from mechanical trauma, heat and cold, electrical discharges, changes in pressure, and radiation. Necrosis contributes to or complicates physical injury. The term *"mechanical trauma"* can be defined as damage/injury to the body from a blow, crush, cut, or penetrating wound. Necrosis contributes to or complicates mechanical trauma. The term *"heat injury"* can be defined as damage/injury to the body from a heat source, causing an increase in the temperature of local tissue. Necrosis contributes to or complicates thermal injury. The term *"cold injury"* can be defined as damage/injury to the body from cold exposure, including primarily peripheral cold injuries that are localized to the extremities and exposed skin and includes chilblain, trench foot and frostbite. Necrosis due to the decrease in temperature contributes to or complicates cold injury. The term *"chilblain"* can be defined as small swellings on the skin that occur as a reaction to cold temperatures. Necrosis due to the decrease in temperature contributes to chilblain. The term *"trench foot"* can be defined as a painful condition of the feet caused by long immersion in cold water or mud and marked by blackening and death of surface tissue. Necrosis due to the decrease in temperature and/or stress from prolonged water immersion, contributes to trench foot. The term *"frostbite"* can be defined as damage/injury caused by freezing of the skin and underlying tissues. Necrosis due to a decrease in temperature contributes to frostbite. The term *"avascular necrosis"* can be defined as a condition in which there is a loss of blood flow to bone tissue, which causes the bone to die. Necrosis contributes to avascular necrosis. The term *"pressure injury"* can be defined as an area(s) of necrosis and often ulceration (also called pressure ulcers) where soft tissues are compressed, and are caused by unrelieved mechanical pressure in combination with friction, shearing forces, and moisture. Necrosis contributes to pressure injury. The term "*skin graft failure*" can be defined as the failure of transplantation of one patch of skin and its derivatives (hair, nails, sweat and oil glands) that is surgically removed from one area of the body and attached to another area. Necrosis contributes to skin graft failure. The "subject" referred to herein may be a vertebrate, a mammal, or a domestic animal. The subject may be any animal of veterinary interest, for instance, a cat, a dog, a horse, a sheep or a cow. However, it is preferred that the subject is a mammal, such as a human. The term *"epilepsy"* can be defined as a condition where sudden bursts of electrical activity in the brain cause seizures or fits. The term *"neurodegenerative disease"* can be defined as disease characterized by neuron loss. The term "*Alzheimer's disease (AD)"* can be defined as a progressive, degenerative disease of the brain marked especially by confusion, disorientation, memory failure, speech disturbances, and eventual dementia. The term "*Parkinson's disease (PD)*" can be defined as a chronic progressive neurological disease marked especially by tremor of resting muscles, rigidity, slowness of movement, impaired balance, and a shuffling gait. The term "*prion diseases"* can be defined as transmissible brain diseases of mammals. The term "*amyotrophic lateral sclerosis (ALS)"* can be defined as a neurological disorder that affects motor neurons, the nerve cells in the brain and spinal cord that control voluntary muscle movement and breathing. The term *"Huntington's disease"* can be defined as a hereditary brain disorder that is a progressive, neurodegenerative condition marked especially by impairments in thinking and reasoning, disturbances of emotion and behaviour, and the involuntary spasmodic movements of chorea and that is associated with the loss or atrophy of nerve cells. The term *"spinal muscular atrophy"* can be defined as a group of hereditary diseases that can damage and kill specialized nerve cells in the brain and spinal cord (motor neurons). The term *"spinocerebellar ataxia"* can be defined as a group of dominantly inherited, predominately late-onset, cerebellar ataxias. The term *"ataxia"* can be defined as one of a group of genetic disorders characterized by slowly progressive incoordination of gait and is often associated with poor coordination of hands, speech, and eye movements. The term *"motor neuron disease"* can be defined as a group of progressive neurological disorders that destroy motor neurons. The term *"multiple system atrophy"* can be defined as a rare condition of the nervous system that causes gradual damage to nerve cells in the brain. The term *"progressive supranuclear palsy"* can be defined as a rare neurological condition that can cause problems with balance, movement, vision, speech and swallowing. The term *"spinal cord injury"* can be defined as damage to the bundle of nerves and nerve fibres that sends and receives signals from the brain. The term *"acute liver disease*/*injury"* can be defined as a loss of liver function that progresses quickly in days to a few months. The term "acute *kidney disease*/*injury"* can be defined as a loss of kidney function that progresses quickly in days to a few months. The term *"chronic liver disease*/*injury"* can be defined as a condition where kidney function gets worse over time and includes associated conditions like non-alcoholic fatty liver disease (NAFLD), non-alcoholic steatohepatitis (NASH) and cirrhosis. The term *"chronic kidney disease*/*injury"* can be defined as a condition where kidney function gets worse over time. The term *"pancreatic disease"* can be defined as a condition that results in loss of pancreatic function (e.g. due to alcohol or inflammation). The term *"metabolic syndrome"* can be defined as a condition that includes a cluster of risk factors specific for cardiovascular disease. The cluster of metabolic factors include abdominal obesity, high blood pressure, impaired fasting glucose, high triglyceride levels, and low HDL cholesterol levels. The term *"autoimmune disease"* can be defined as a condition associated with a hyperactive immune system. The term *"inflammatory bowel disease"* can be defined as an autoimmune condition affecting part or all of the gastrointestinal system, and includes conditions like Crohn's disease, inflammatory bowel syndrome and ulcerative colitis. The term "*arthritis*" can be defined as a condition of joint inflammation. The term "*lupus*" can be defined as a long-term autoimmune condition that causes joint pain, skin rashes and tiredness. The term "*diabetes*" can be defined as a chronic, metabolic disease characterized by elevated levels of blood glucose (or blood sugar). The term "*multiple sclerosis (MS)"* can be defined as a disorder in which the body's immune system attacks the protective covering of the nerve cells. The term *"traumatic brain injury (TBI)"* can be defined as a form of acquired brain injury which occurs when a sudden trauma causes damage to the brain. The term *"head injury"* can be any injury that occurs to the scalp, skull, brain, and underlying tissue and blood vessels in the head. The term *"concussion"* can be defined as the sudden but short-lived loss of mental function that occurs after a blow or other injury to the head. The term *"closed head injury"* can be defined as an injury that occurs where there is impact without breaking the skull. The term *"penetrating head injury"* can be defined as an injury that occurs when an object fractures the skull and enters brain tissue. The term *"diffuse brain injury"* can be defined as an injury that occurs when the brain moves within the skull when the head is shaken, and damage might be caused in several areas where the brain hits the skull. The term *"brain contusion"* can be defined as bruised or swollen brain tissue that occurs when the skull cracks or breaks. The term *"depressed skull fracture"* can be defined as fragments of a broken skull pressing against the brain. The term *"penetrating skull fracture"* can be defined as bone fragments entering the brain tissue. The term *"cerebral palsy"* can be defined as a group of lifelong conditions that affect movement and co-ordination. The term *"intracranial hematoma (ICH)"* can be defined as a collection of blood within the skull. The term *"hematoma"* can be defined as a solid swelling of clotted blood within the tissues. The term *"stroke"* can be defined as a condition when the blood supply to part of the brain is interrupted or reduced. The term *"brain aneurysm"* can be defined as a bulge in a blood vessel caused by a weakness in the blood vessel wall. The term *"hypoxic brain injury"* can be defined as a condition when part of the brain receives reduced oxygen. The term *"anoxic brain injury"* can be defined as a condition when part of the brain receives no oxygen. The term *"haemorrhage"* can be defined as an escape of blood from a ruptured blood vessel. The term *"meningitis"* can be defined as an infection of the protective membranes that surround the brain and spinal cord (meninges). The term *"encephalitis"* can be defined as inflammation of the brain, caused by infection or an allergic reaction. The term "cancer" can be defined as disease caused by an uncontrolled division of abnormal cells in a part of the body. The term *"tumours"* can be defined as groups of abnormal cells that form lumps or growths. The term *"infectious disease"* can be defined as an illness due to a pathogen or its toxic product. The term *"cardiac arrest"* can be defined as a, sometimes temporary, cessation of the heart's functioning. The term *"ischemia"* can be defined as an inadequate blood supply to an organ or other part of the body.

In certain embodiments, the term *"comprising"* can refer to *"consisting of"* or *"consisting essentially of".*

All of the embodiments and features described herein (including any accompanying clauses, claims, abstract and drawings), and/or all of the steps of any method or process so disclosed, may be combined with any of the above aspects or embodiments in any combination, unless stated otherwise with reference to a specific combination, for example, combinations where at least some of such features and/or steps are mutually exclusive.

For a better understanding of the invention, and to show embodiments of the invention may be put into effect, reference will now be made, by way of example, to the accompanying drawings, in which:-
**Figure 1** **is an illustration of the *predicted weak spots* in cell physiology that make cells susceptible to cellular necrosis.** The weak spots include: increase in cytosolic calcium via (i) movement of calcium ions into the cytosol via plasma membrane calcium ion channels; (ii) movement of calcium ions from within organelles with the cell, especially the endoplasmic/sarcoplasmic (ER/SR) reticulum via ER/SR calcium ion channels; and (iii) movement of calcium ions and other damaging agents from cell to cell via gap junctions. Other weak spots include calpain hyper-activation, cathepsin hyper-activation and the opening of store-operated calcium channels (also called calcium release-activated calcium channels) post ER calcium depletion. The most pertinent of these steps are the calcium ion entry via ER/SR calcium ion channels and gap junctions, and followed by entry via the plasma membrane calcium ion channels, as discussed in further detail in Example 1 below.

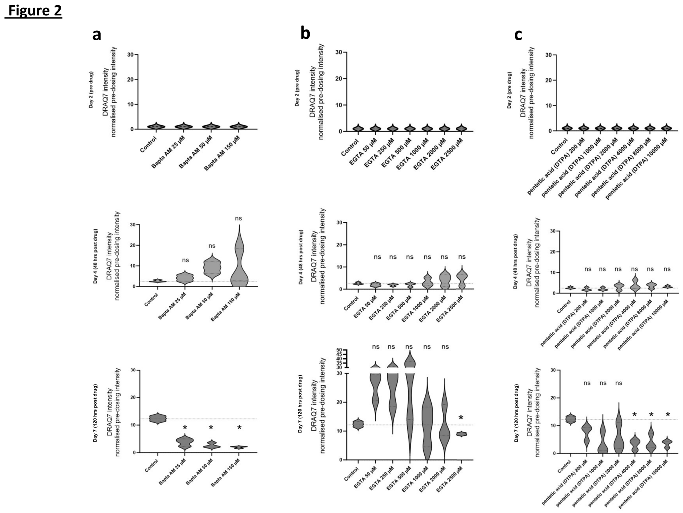

**Figures 2-28** **show necrotic core formation in Hepg2s spheroids** under different stressors and treatment conditions, as discussed below. Spheroids are spherical cellular aggregates supporting cell-cell and cell-matrix interactions in an environment that mimics the real-world situation. Spheroids cannot be supported for long periods of time or grown large due to necrotic core formation (cells in the middle of the spheroid are starved of oxygen and adequate nutrients, with primarily hypoxia causing cell death via necrosis).
**Figure 2** **shows that Hepg2s spheroids show reduced necrotic core formation, induced by hypoxic and nutrient deprivation stress, after 7 days with the addition of relatively high doses of calcium chelators.** Treatment is different chelating agents at varying dosages. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

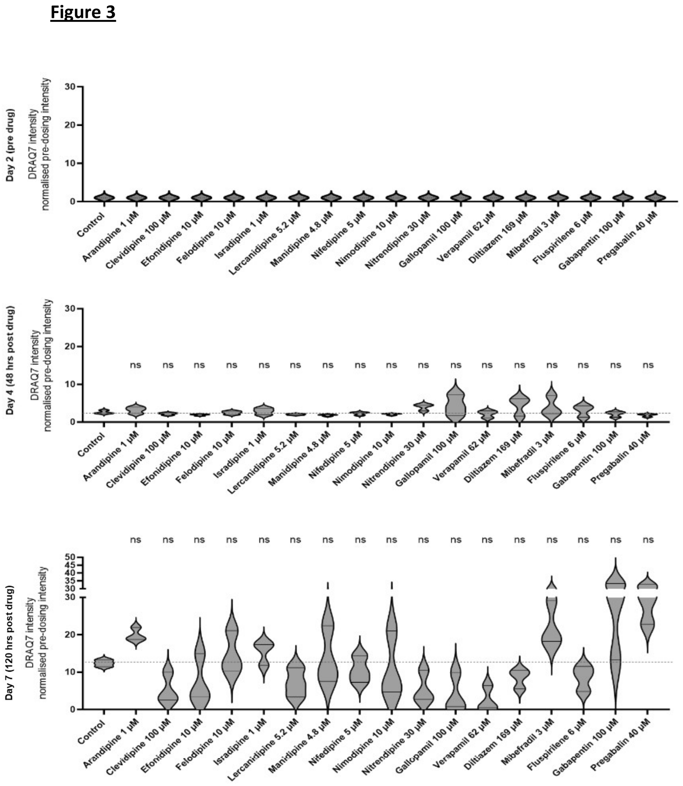

**Figure 3** **shows that Hepg2s spheroids do not show reduced necrotic core formation, induced by hypoxic and nutrient deprivation stress, with the addition of a range of plasma membrane calcium channel inhibitors.** Treatment is different plasma membrane calcium channel inhibitors. The inhibitors were added at doses which were expected to be the maximum doses showing minimal toxicity. Red dotted line: mean of control. Three trials (n=2 per trial). Violin plot showing the distribution of the data with quartiles as dotted lines.*P<0.05, **P<0.01, ***P<0.001, ****P<0.0001 by two-way ANOVA (Dunnett's correction) relative to control.

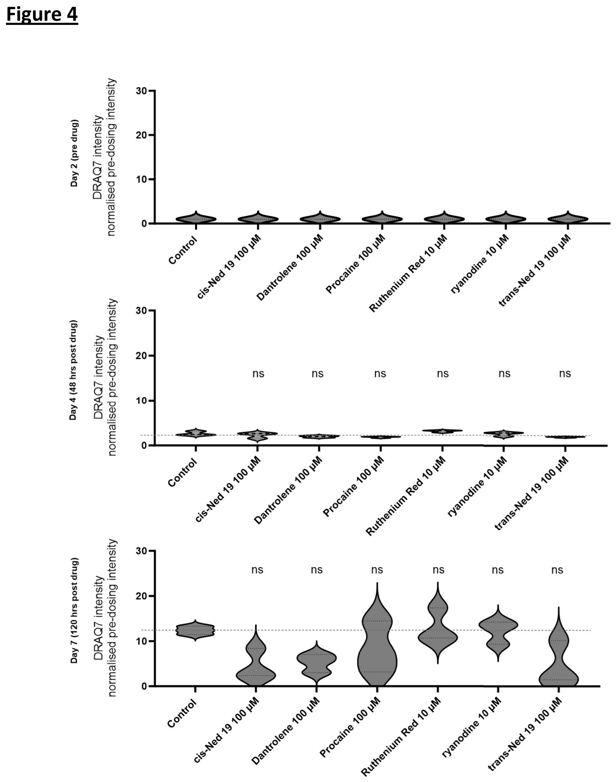

**Figure 4** **shows that Hepg2s spheroids do not show reduced necrotic core formation, induced by hypoxic and nutrient deprivation stress, with the addition of a range of endoplasmic reticulum /sarcoplasmic reticulum (ER/SR) calcium channel inhibitors.** Treatment is endoplasmic reticulum /sarcoplasmic reticulum (ER/SR) calcium channel inhibitors at doses which were expected to be the maximum doses showing minimal toxicity. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

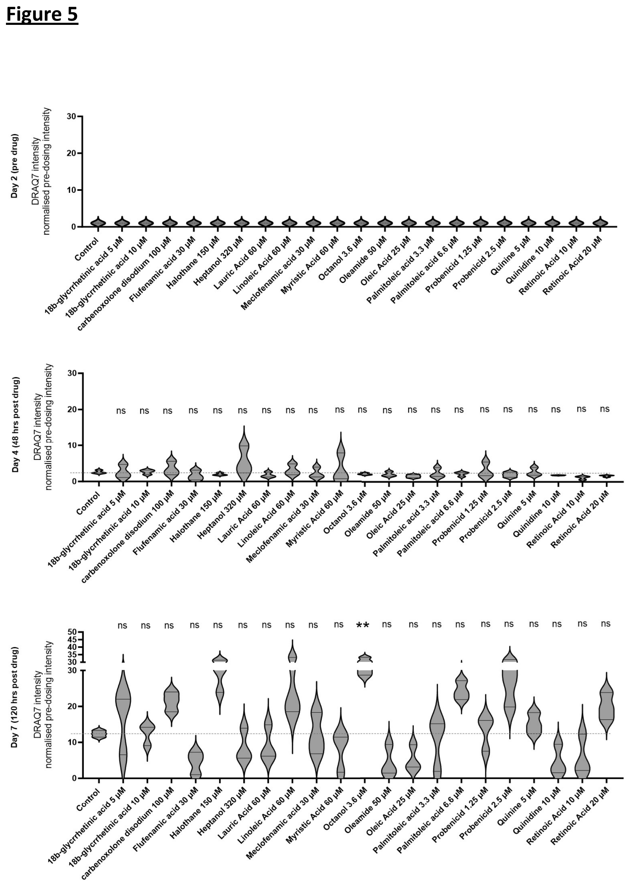

**Figure 5** **shows that Hepg2s spheroids do not show reduced necrotic core formation, induced by hypoxic and nutrient deprivation stress, with the addition of a range of gap junction inhibitors.** Treatment is gap junction inhibitors at doses which were expected to be the maximum doses showing minimal toxicity. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

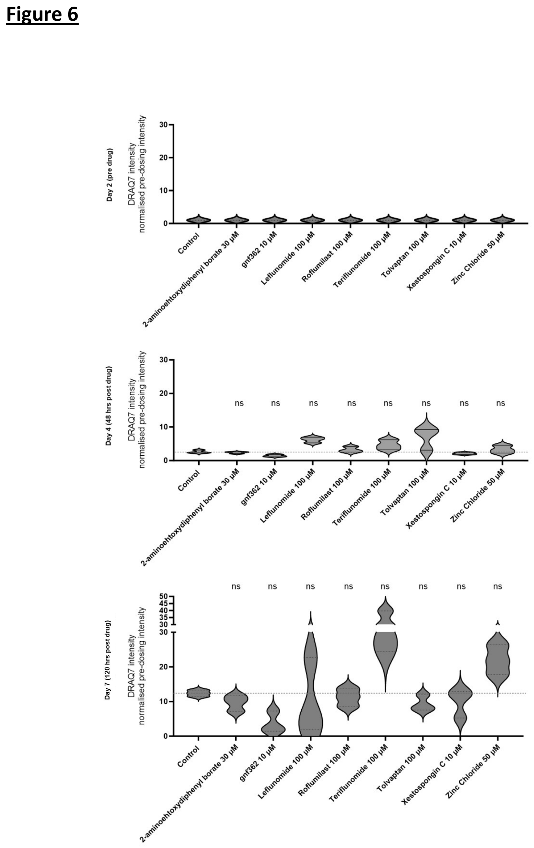

**Figure 6** **shows that Hepg2s spheroids do not show reduced necrotic core formation, induced by hypoxic and nutrient deprivation stress, with the addition of a range of store-operate calcium channels (SOCC) inhibitors.** Treatment is SOCC inhibitors at doses which were expected to be the maximum doses showing minimal toxicity. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

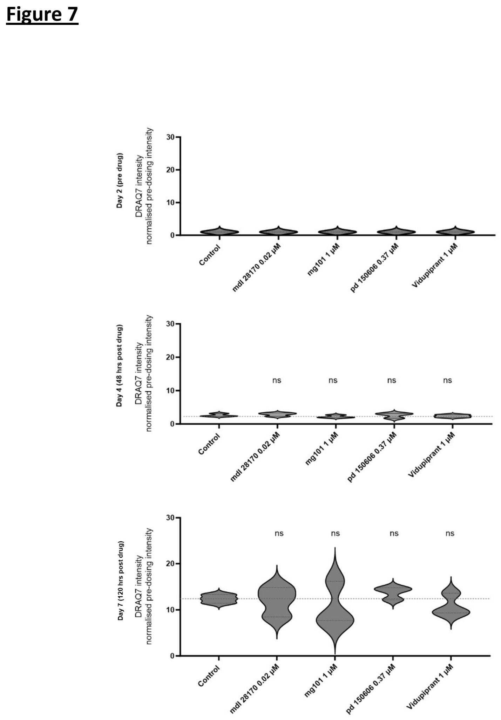

**Figure 7** **shows that Hepg2s spheroids do not show reduced necrotic core formation, induced by hypoxic and nutrient deprivation stress, with the addition of a range of calpain inhibitors.** Treatment is calpain inhibitors at doses which were expected to be the maximum doses showing minimal toxicity. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

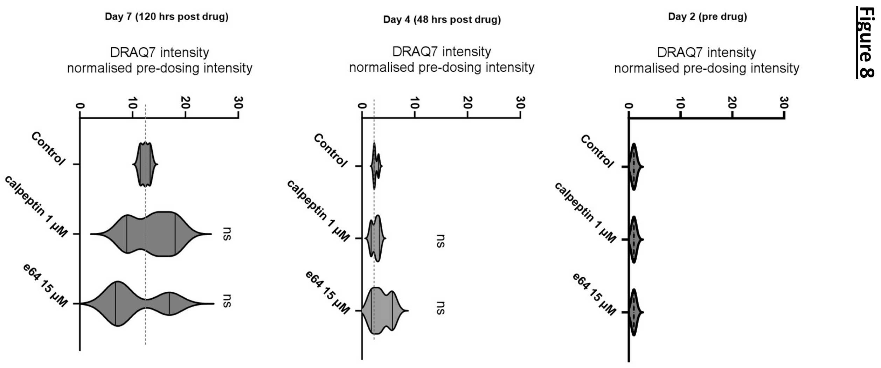

**Figure 8** **shows that Hepg2s spheroids do not show reduced necrotic core formation, induced by hypoxic and nutrient deprivation stress, with the addition of calpain/cathepsin non-specific inhibitors.** Treatment is calpain/cathepsin non-specific inhibitors at doses which were expected to be the maximum doses showing minimal toxicity. Red dotted line: mean of control. Three trials (n=2 per trial). Violin plot showing the distribution of the data with quartiles as dotted lines.*P<0.05, **P<0.01, ***P<0.001, ****P<0.0001 by two-way ANOVA (Dunnett's correction) relative to control.

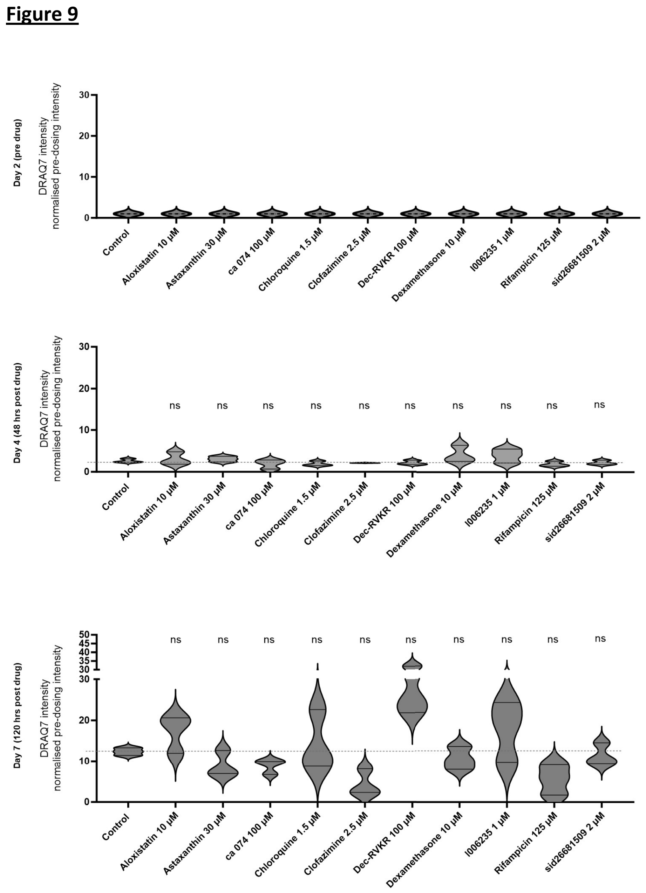

**Figure 9** **shows that Hepg2s spheroids do not show reduced necrotic core formation, induced by hypoxic and nutrient deprivation stress, with the addition of a range of cathepsin inhibitors.** Treatment is cathepsin inhibitors at doses which were expected to be the maximum doses showing minimal toxicity. Red dotted line: mean of control. Three trials (n=2 per trial). Violin plot showing the distribution of the data with quartiles as dotted lines.*P<0.05, **P<0.01, ***P<0.001, ****P<0.0001 by two-way ANOVA (Dunnett's correction) relative to control.

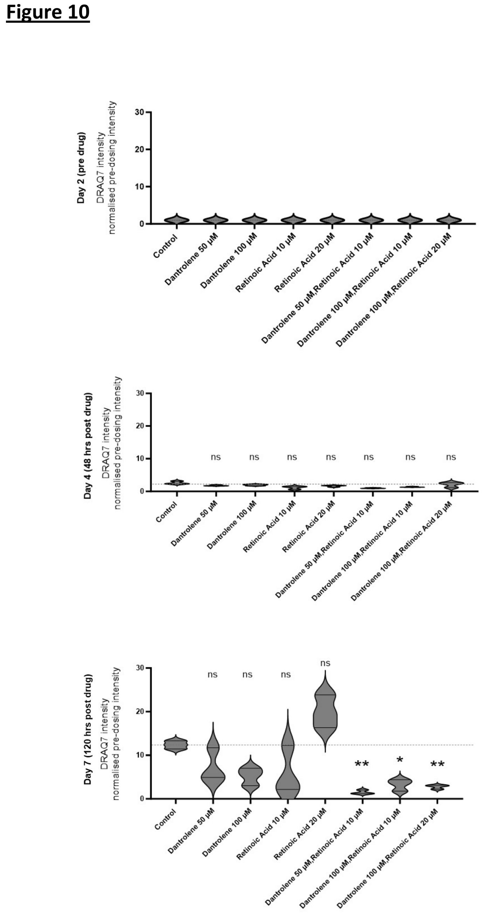

**Figure 10** **shows that Hepg2s spheroids show greatly reduced necrotic core formation, induced by hypoxic and nutrient deprivation stress, with the addition of a combination of the endoplasmic reticulum/sarcoplasmic reticulum (ER/SR) calcium channel inhibitor Dantrolene and the gap junction inhibitor retinoic acid.** Treatment is Dantrolene in different dosages individually, retinoic acid in different dosages individually, and the combination of Dantrolene and retinoic acid in different dosages. The combination has a more than additive effect compared to treatment with the individual components of the combination that show no significant difference from the control. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

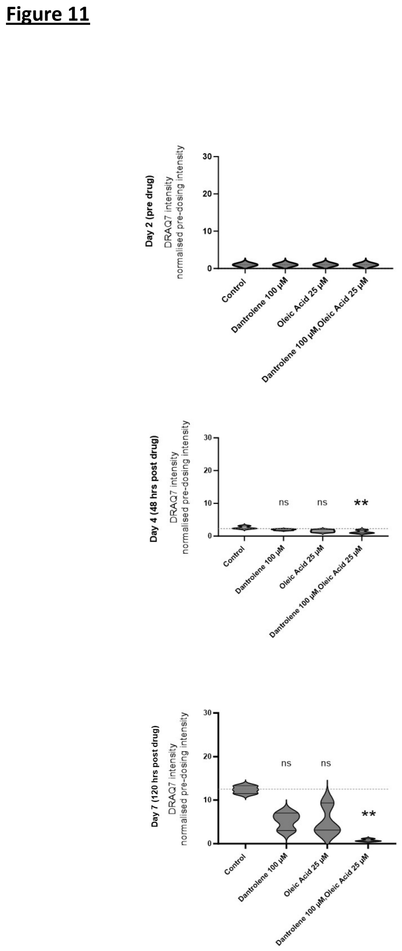

**Figure 11** **shows that Hepg2s spheroids show greatly reduced necrotic core formation, induced by hypoxic and nutrient deprivation stress, with the addition of a combination of the endoplasmic reticulum/sarcoplasmic reticulum (ER/SR) calcium channel inhibitor Dantrolene and the gap junction inhibitor oleic acid.** Treatment is Dantrolene and oleic acid used individually and in combination. The combination has a more than additive effect compared to treatment with the individual components of the combination that show no significant difference from the control. Red dotted line: mean of control. Three trials (n=2 per trial). Violin plot showing the distribution of the data with quartiles as dotted lines..*P<0.05, **P<0.01, ***P<0.001, ****P<0.0001 by two-way ANOVA (Dunnett's correction) relative to control.

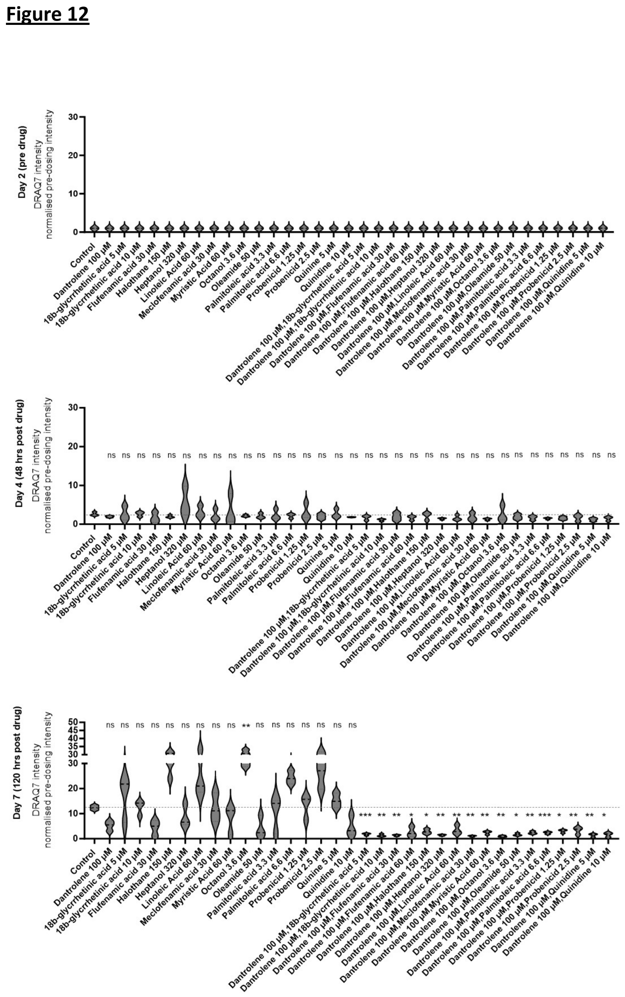

**Figure 12** **shows that Hepg2s spheroids show greatly reduced necrotic core formation, induced by hypoxic and nutrient deprivation stress, with the addition of a combination of the endoplasmic reticulum/sarcoplasmic reticulum (ER/SR) calcium channel inhibitor Dantrolene and a range of gap junction inhibitors.** Treatment is Dantrolene and a range of gap junction inhibitors used individually and in combination. The combination has a more than additive effect compared to treatment with the individual components of the combination that show no significant difference from the control. Red dotted line: mean of control. Three trials (n=2 per trial). Violin plot showing the distribution of the data with quartiles as dotted lines..*P<0.05, **P<0.01, ***P<0.001, ****P<0.0001 by two-way ANOVA (Dunnett's correction) relative to control.

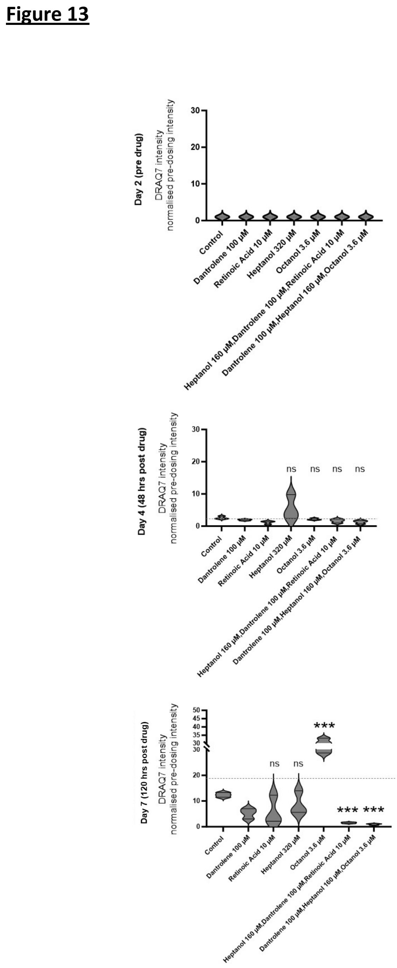

**Figure 13** **shows that Hepg2s spheroids show greatly reduced necrotic core formation, induced by hypoxic and nutrient deprivation stress, with the addition of the endoplasmic reticulum /sarcoplasmic reticulum (ER/SR) calcium channel inhibitor Dantrolene combined with more than one gap junction inhibitor.** Treatment is Dantrolene and more than one gap junction inhibitor combined, as well as the individual components of the combination tested separately. The combination has a more than additive effect compared to treatment with the individual components of the combination that show no significant difference from the control. Red dotted line: mean of control. Three trials (n=2 per trial). Violin plot showing the distribution of the data with quartiles as dotted lines.*P<0.05, **P<0.01, ***P<0.001, ****P<0.0001 by two-way ANOVA (Dunnett's correction) relative to control.

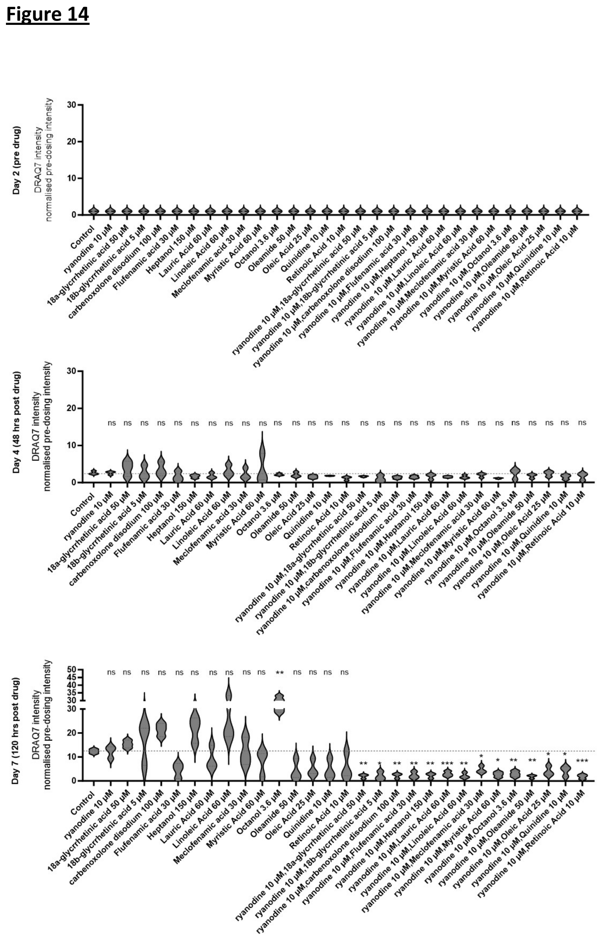

**Figure 14** **shows that Hepg2s spheroids show greatly reduced necrotic core formation, induced by hypoxic and nutrient deprivation stress, with the addition of a combination of the endoplasmic reticulum/sarcoplasmic reticulum (ER/SR) calcium channel inhibitor ryanodine and a range of gap junction inhibitors.** Treatment is ryanodine and a range of gap junction inhibitors used individually and in combination. The combination has a more than additive effect compared to treatment with the individual components of the combination that show no significant difference from the control. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

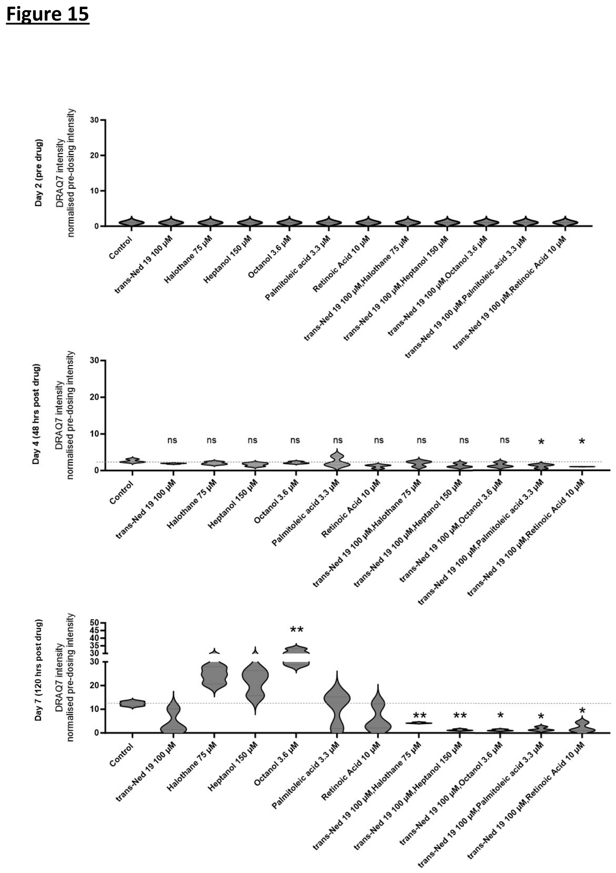

**Figure 15** **shows that Hepg2s spheroids show greatly reduced necrotic core formation, induced by hypoxic and nutrient deprivation stress, with the addition of a combination of the endoplasmic reticulum/sarcoplasmic reticulum (ER/SR) calcium channel inhibitor trans-Ned 19 and a range of gap junction inhibitors.** Treatment is trans-Ned 19 and a range of gap junction inhibitors used individually and in combination. The combination has a more than additive effect compared to treatment with the individual components of the combination that show no significant difference from the control. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

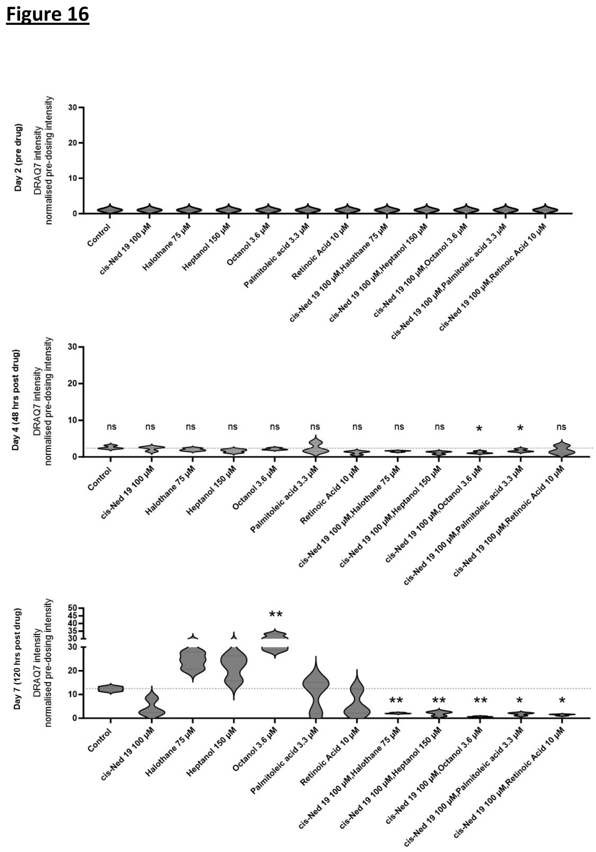

**Figure 16** **shows that Hepg2s spheroids show greatly reduced necrotic core formation, induced by hypoxic and nutrient deprivation stress, with the addition of a combination of the endoplasmic reticulum/sarcoplasmic reticulum (ER/SR) calcium channel inhibitor cis-Ned 19 and a range of gap junction inhibitors.** Treatment is cis-Ned 19 and a range of gap junction inhibitors used individually and in combination. The combination has a more than additive effect compared to treatment with the individual components of the combination that show no significant difference from the control. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

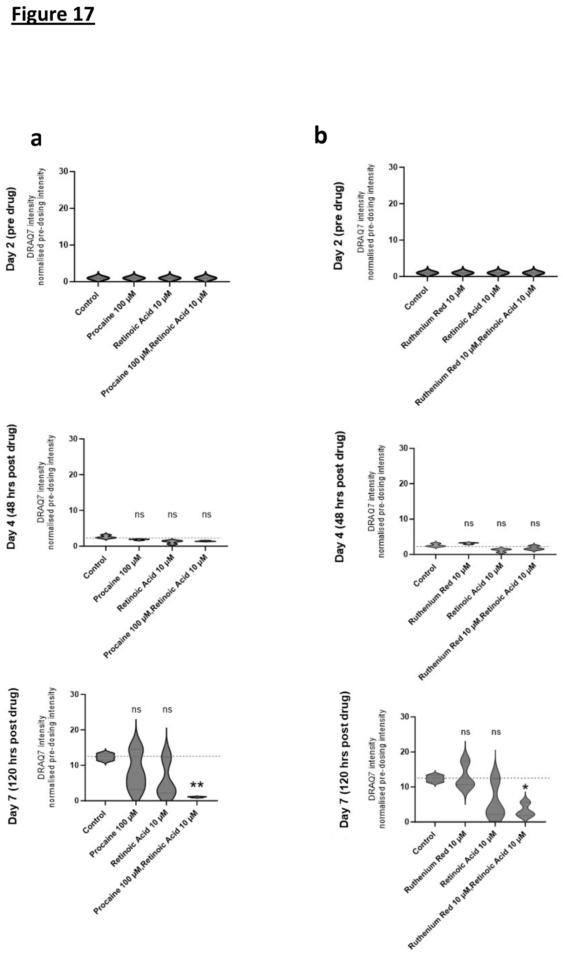

**Figure 17** **shows that Hepg2s spheroids show greatly reduced necrotic core formation, induced by hypoxic and nutrient deprivation stress, with the addition of a combination of either the endoplasmic reticulum/sarcoplasmic reticulum (ER/SR) calcium channel inhibitor procaine or the endoplasmic reticulum/sarcoplasmic reticulum (ER/SR) calcium channel inhibitor ruthenium red, with the gap junction inhibitor retinoic acid. (a)** Treatment is procaine and the gap junction inhibitor retinoic acid used individually and in combination. **(b)** Treatment is ruthenium red and the gap junction inhibitor retinoic acid used individually and in combination. The combinations (of both (a) and (b)) have a more than additive effect compared to treatment with the individual components of the combination that show no significant difference from the controls. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

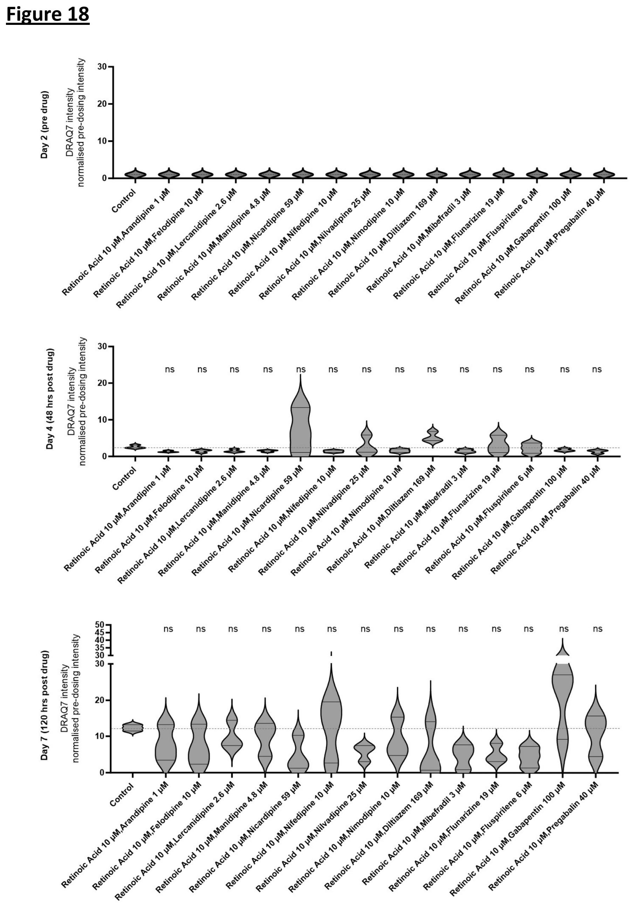

**Figure 18** **shows that Hepg2s spheroids show no reduced necrotic core formation, induced by hypoxic and nutrient deprivation stress, with the addition of a combination of the gap junction inhibitor retinoic acid and a range of plasma membrane calcium channel inhibitors.** Treatment is a combination of the gap junction inhibitor retinoic acid and a range of plasma membrane calcium channel inhibitors. The combinations show no significant difference from the control. Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

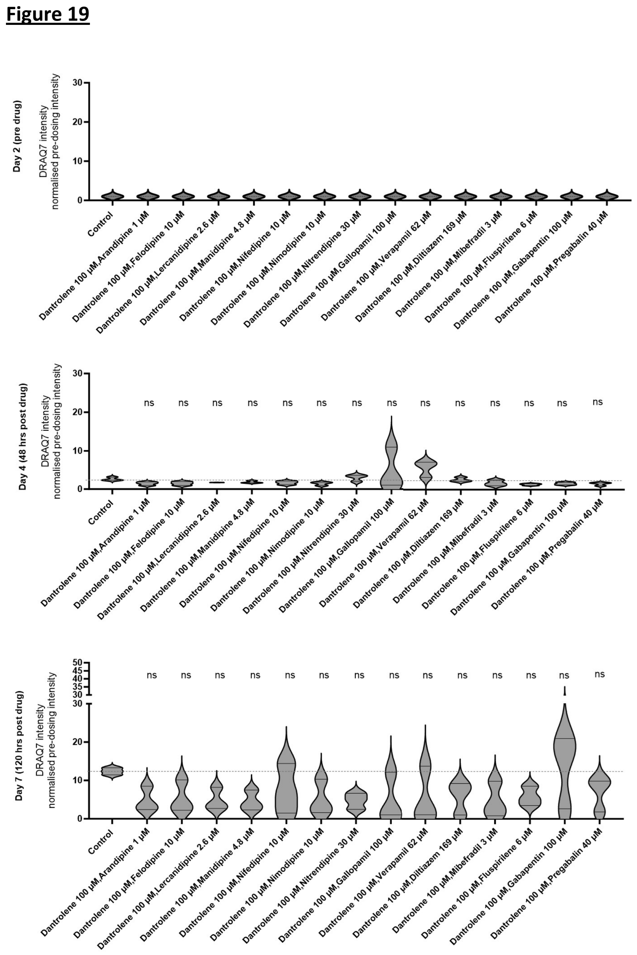

**Figure 19** **shows that Hepg2s spheroids show no reduced necrotic core formation, induced by hypoxic and nutrient deprivation stress, with the addition of a combination of the endoplasmic reticulum/sarcoplasmic reticulum (ER/SR) calcium channel inhibitor Dantrolene and a range of plasma membrane calcium channel inhibitors.** Treatment is a combination the Endoplasmic reticulum/Sarcoplasmic reticulum (ER/SR) calcium channel inhibitor Dantrolene and a range of plasma membrane calcium channel inhibitors. The combinations show no significant difference from the control. Red dotted line: mean of control. Three trials (n=2 per trial). Violin plot showing the distribution of the data with quartiles as dotted lines. *P<0.05, **P<0.01, ***P<0.001, ****P<0.0001 by Two-way ANOVA (Dunnett's correction) relative to control.

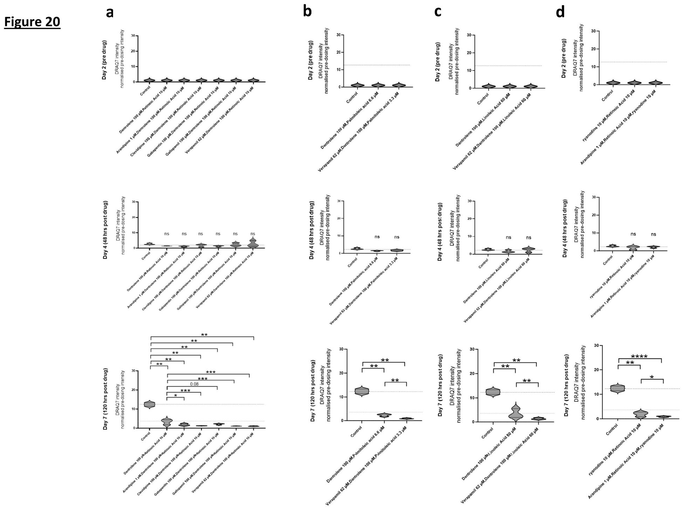

**Figure 20** **shows that reduction in Hepg2s spheroid necrotic core formation, induced by hypoxic and nutrient deprivation stress, is even greater when the combination of an endoplasmic reticulum /sarcoplasmic reticulum (ER/SR) calcium channel inhibitor and a gap junction inhibitor is further combined with a plasma membrane calcium channel inhibitor. (a)** Treatment is the endoplasmic reticulum/sarcoplasmic reticulum (ER/SR) calcium channel inhibitor Dantrolene and the gap junction inhibitor retinoic acid, used alone and in combination with a range of different plasma membrane calcium channel inhibitors. **(b)** Treatment is the endoplasmic reticulum/sarcoplasmic reticulum (ER/SR) calcium channel inhibitor Dantrolene and the gap junction inhibitor palmitoleic acid, used alone and in combination with the plasma membrane calcium channel inhibitor Verapamil. **(c)** Treatment is the endoplasmic reticulum/sarcoplasmic reticulum (ER/SR) calcium channel inhibitor Dantrolene and the gap junction inhibitor linoleic acid, used alone and in combination with the plasma membrane calcium channel inhibitor Verapamil. **(d)** Treatment is the Endoplasmic reticulum/sarcoplasmic reticulum (ER/SR) calcium channel inhibitor ryanodine and the gap junction inhibitor retinoic acid, used alone and in combination with the plasma membrane calcium channel inhibitor Arandipine. The combination of the 3 compounds (i.e., the ER/SR calcium channel inhibitor, the gap junction inhibitor and the plasma membrane calcium channel inhibitor) has a greater effect than the combination of only the endoplasmic reticulum/sarcoplasmic reticulum (ER/SR) calcium channel inhibitor with the gap junction inhibitor. However, it will be appreciated that both the combination of 2 compounds, and the combination of 3 compounds, have a more than additive effect compared to the individual components of the combination relative to the control (c.f. Figures 3,4 and 5 of the individual components that show no significant difference to the control; Figures 3,4 and 5 and this figure are directly comparable with trials done alongside each other). Red dotted line: mean of control. Grey dotted line: mean of the combination of 2 compounds, where one is the gap junction inhibitor and the other the endoplasmic reticulum/sarcoplasmic reticulum (ER/SR) calcium channel inhibitor. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control and relative to the combination of 2 compounds, i.e., to the endoplasmic reticulum/sarcoplasmic reticulum (ER/SR) calcium channel inhibitor with the gap junction inhibitor.

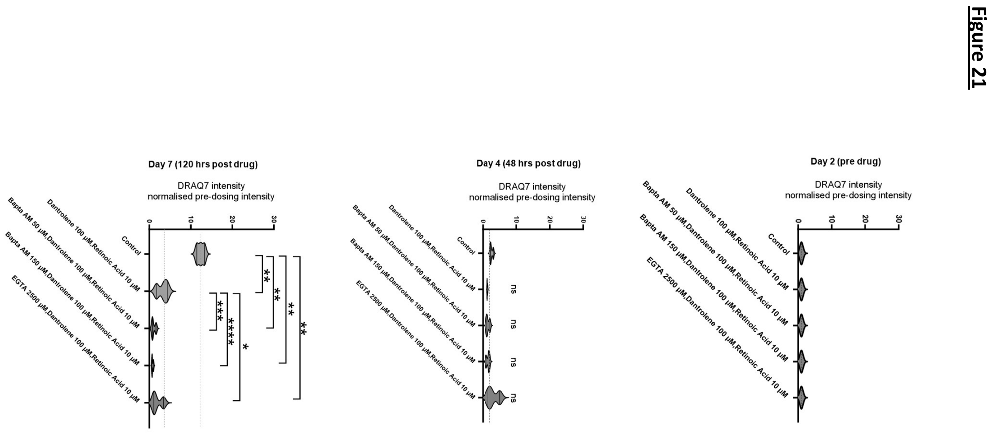

**Figure 21** **shows that reduction in Hepg2s spheroid necrotic core formation, induced by hypoxic and nutrient deprivation stress, is even greater when the combination of an endoplasmic reticulum /Sarcoplasmic reticulum (ER/SR) calcium channel inhibitor and a gap junction inhibitor is further combined with a calcium chelator.** Treatment is the endoplasmic reticulum/sarcoplasmic reticulum (ER/SR) calcium channel inhibitor Dantrolene and the gap junction inhibitor retinoic acid, used alone and in combination with a range of different calcium chelators. The combination of the 3 compounds has a greater effect than the combination of only the endoplasmic reticulum/sarcoplasmic reticulum (ER/SR) calcium channel inhibitor with the gap junction inhibitor. It will be appreciated that both the combination of 2 compounds, and the combination of 3 compounds, have a more than additive effect compared to the individual components of the combination relative to the control (c.f. Figures 2,4 and 5 of the individual components that show no significant difference to control; Figures 2,4 and 5 and this figure are directly comparable with trials done alongside each other). Red dotted line: mean of control. Grey dotted line: mean of the combination of 2 compounds, where one is the gap junction inhibitor and the other the endoplasmic reticulum/sarcoplasmic reticulum (ER/SR) calcium channel inhibitor. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control and relative to the combination of 2 compounds, i.e., to the endoplasmic reticulum/sarcoplasmic reticulum (ER/SR) calcium channel inhibitor with the gap junction inhibitor.

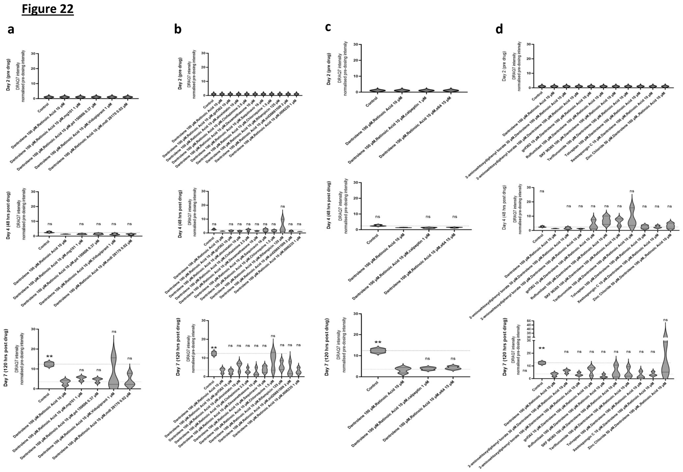

**Figure 22** **shows that there is no further reduction in Hepg2s spheroid necrotic core formation, induced by hypoxic and nutrient deprivation stress, when the combination of an endoplasmic reticulum/sarcoplasmic reticulum (ER/SR) calcium channel inhibitor and a gap junction inhibitor is further combined with drugs associated with other targets of weak spots in cell physiology that make cells susceptible to necrosis (including calpain inhibitors, cathepsin inhibitors, calpain/cathepsin non-specific protease inhibitors and SOCC inhibitors). (a)** Treatment is the endoplasmic reticulum/sarcoplasmic reticulum (ER/SR) calcium channel inhibitor Dantrolene and the gap junction inhibitor retinoic acid, used alone and in combination with a range of different calpain inhibitors. **(b)** Treatment is the endoplasmic reticulum/sarcoplasmic reticulum (ER/SR) calcium channel inhibitor Dantrolene and the gap junction inhibitor retinoic acid, used alone and in combination with a range of different cathepsin inhibitors. **(c)** Treatment is the endoplasmic reticulum/sarcoplasmic reticulum (ER/SR) calcium channel inhibitor Dantrolene and the gap junction inhibitor retinoic acid, used alone and in combination with a range of different calpain/cathepsin non-specific protease inhibitors. **(d)** Treatment is the endoplasmic reticulum/sarcoplasmic reticulum (ER/SR) calcium channel inhibitor Dantrolene and the gap junction inhibitor retinoic acid, used alone and in combination with a range of different SOCC inhibitors. The combination of 3 has the same, if not a worse, effect than the combination of only the endoplasmic reticulum/sarcoplasmic reticulum (ER/SR) calcium channel inhibitor with the gap junction inhibitor. Red dotted line: mean of control. Grey dotted line: mean of the combination of 2 compounds, where one is the gap junction inhibitor and the other is the endoplasmic reticulum/sarcoplasmic reticulum (ER/SR) calcium channel inhibitor. Three trials (n=2 per trial). Violin plot showing the distribution of the data with quartiles as dotted lines. *P<0.05, **P<0.01, ***P<0.001, ****P<0.0001 by Two-way ANOVA (Dunnett's correction) relative to the combination of the 2 compounds, i.e., to the endoplasmic reticulum/sarcoplasmic reticulum (ER/SR) calcium channel inhibitor with the gap junction inhibitor.

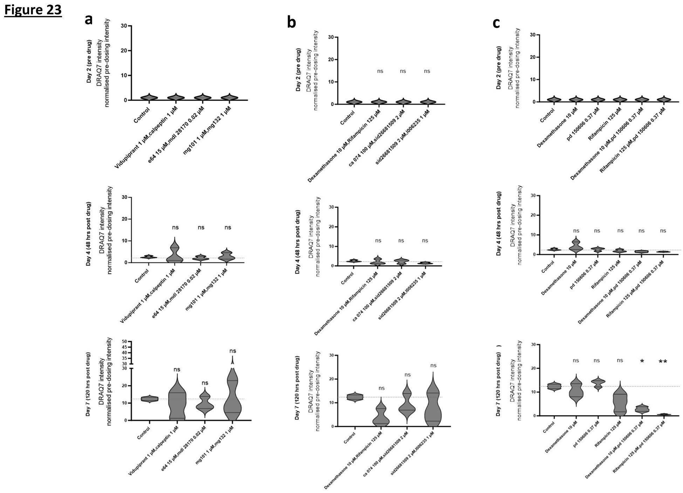

**Figure 23** **shows that Hepg2s spheroids show reduced necrotic core formation, induced by hypoxic and nutrient deprivation stress, with the addition of a combination of a calpain inhibitor with a cathepsin inhibitor, but not other combinations such as a combination of 2 or more calpain inhibitors, a combination of 2 or more calpain inhibitors or combinations with non-specific calpain/cathepsin inhibitors. (a)** Treatment is a combination of different (i) calpain inhibitors, and (ii) calpain inhibitors combined with non-specific calpain/cathepsin inhibitors. **(b)** Treatment is a combination of different cathepsin inhibitors. **(a,b)** The combination has no significant effect relative to the control. **(c)** Treatment is a combination of different calpain inhibitors combined with cathepsin inhibitors; the combination has a significant beneficial effect relative to the control, and the effect is more than compared of the individual components of the combination that show no significant difference from control. The combination of 2 has a more than additive effect compared to the individual components of the combination relative to the control, (c.f. Figures 7 and 9 of the individual components that show no significant difference to control; Figures 7 and 9 and this figure are directly comparable with trials done alongside each other). Red dotted line: mean of control. Three trials (n=2 per trial). Violin plot showing the distribution of the data with quartiles as dotted lines. *P<0.05, **P<0.01, ***P<0.001, ****P<0.0001 by two-way ANOVA (Dunnett's correction) relative to control.

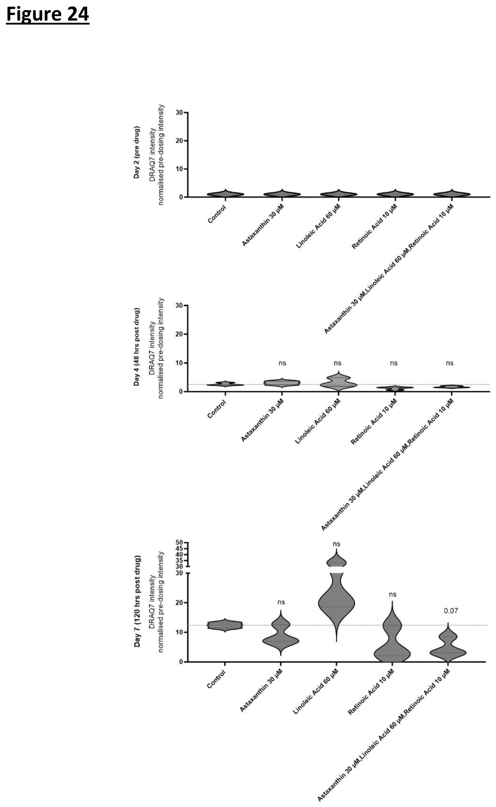

**Figure 24** **shows that Hepg2s spheroids show reduced necrotic core formation, induced by hypoxic and nutrient deprivation stress, when combinations of over-the-counter compounds are used that inhibit targets associated with the *predicted weak spots* in cell physiology that make cells susceptible to cellular necrosis.** Treatment is over the counter compounds that inhibit targets associated with the predicted weak spots in cell physiology (c.f. Figure 1) that make cells susceptible to cellular necrosis. Notably, the low toxicity of these compounds allows them to be mixed in combinations without a severe increase in cell death from chemical toxicity being observed. Spheroids are spherical cellular aggregates supporting cell-cell and cell-matrix interactions in an environment that mimics the real-world situation. Spheroids cannot be supported for long periods of time or grown large due to necrotic core formation (cells in the middle of the spheroid are starved of oxygen and adequate nutrients, with primarily hypoxia causing cell death via necrosis). The combination has a more than additive effect compared of the individual components of the combination that show no significant difference from control. Red dotted line: mean of control. Three trials (n=2 per trials). Violin plot showing the distribution of the data with quartiles as dotted lines. P=0.07 showing marginal reduced core formation by two-way ANOVA (Dunnett's correction) relative to control.

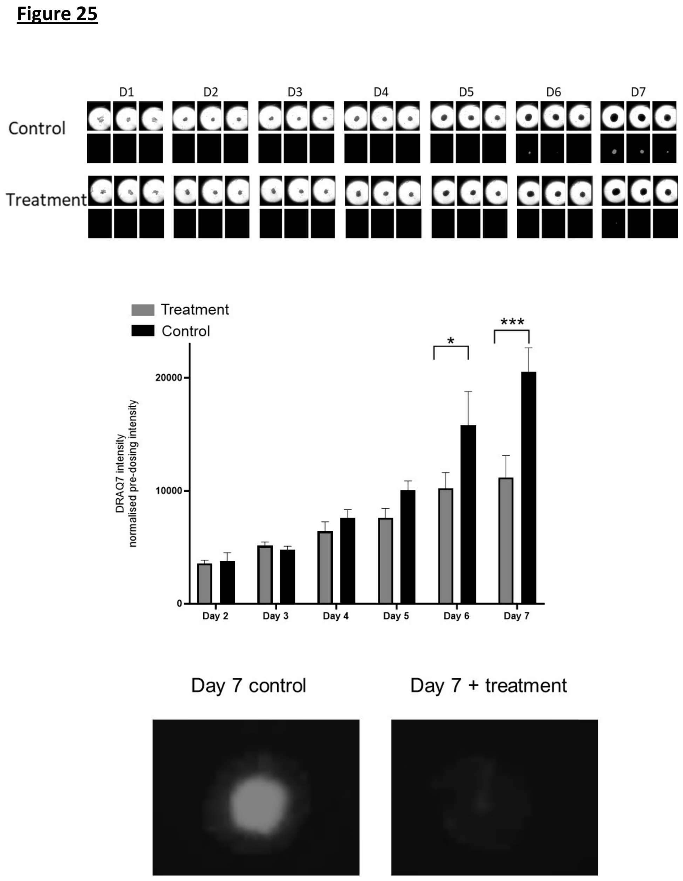

**Figure 25** **is a time series of Hepg2s spheroids with the addition of a combination of the endoplasmic reticulum /sarcoplasmic reticulum (ER/SR) calcium channel inhibitor Dantrolene and the gap junction inhibitor retinoic acid, and shows greatly reduced necrotic core formation, induced by hypoxic and nutrient deprivation stress, through time.** Treatment (added on day 2) is Dantrolene and retinoic acid. The combination is followed through time up to 7 days and shows robust necrotic core suppression in all 3 trials. Top: Bright field images above fluorescence (DRAQ7^{™}; viability dye that stains necrotic cells; red) images of spheroids in the well of multi-welled plates. Middle: Bar graphs with s.t.d showing cell death stain DRAQ7^{™} intensity through time in the treatment and control group. The left-hand bar shows the treatment group, and the right-hand bar shows the control group. Bottom: example fluorescence (DRAQ7^{™}; viability dye that stains necrotic cells; red) images of spheroids in the well of multi-welled plates. Three trials (n=2 per trial). *p<0.05, **P<0.01, ***P<0.001, ****P<0.0001 by two-way ANOVA (Bonferroni correction).

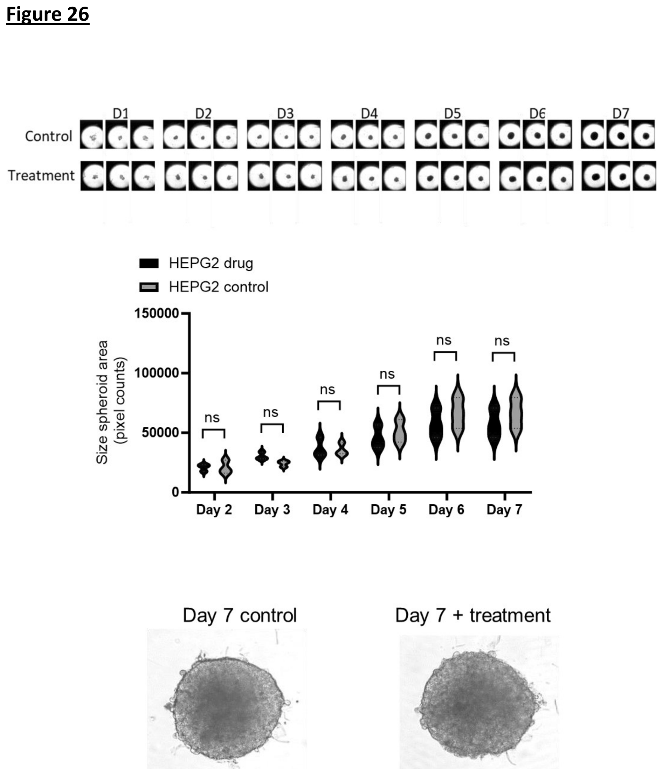

**Figure 26** **is a time series of Hepg2s spheroids with the addition of a combination of the endoplasmic reticulum /sarcoplasmic reticulum (ER/SR) calcium channel inhibitor Dantrolene and the gap junction inhibitor retinoic acid, and shows no significant toxic effect of the combination; spheroid formation, growth, size and appearance appears normal.** Treatment (added on day 2) is Dantrolene and retinoic acid. The combination is followed through time up to 7 days and shows no significant effect on spheroid formation, growth, size or appearance. Top: Bright field images of spheroids in the well of multi-welled plates. Middle: Violin plot of spheroid size through time, a marker of formation and growth, showing the distribution of the data with quartiles as dotted lines. The left-hand plot shows the treatment ("drug") group, and the right-hand plot shows the control group. Bottom: example bright filed images of spheroids in the well of multi-welled plates, showing a normal morphology and appearance and no loss of integrity of the spheroid with the treatment. Three trials (n=2 per trial). *P<0.05, **P<0.01, ***P<0.001, ****P<0.0001 by two-way ANOVA (Bonferroni correction).

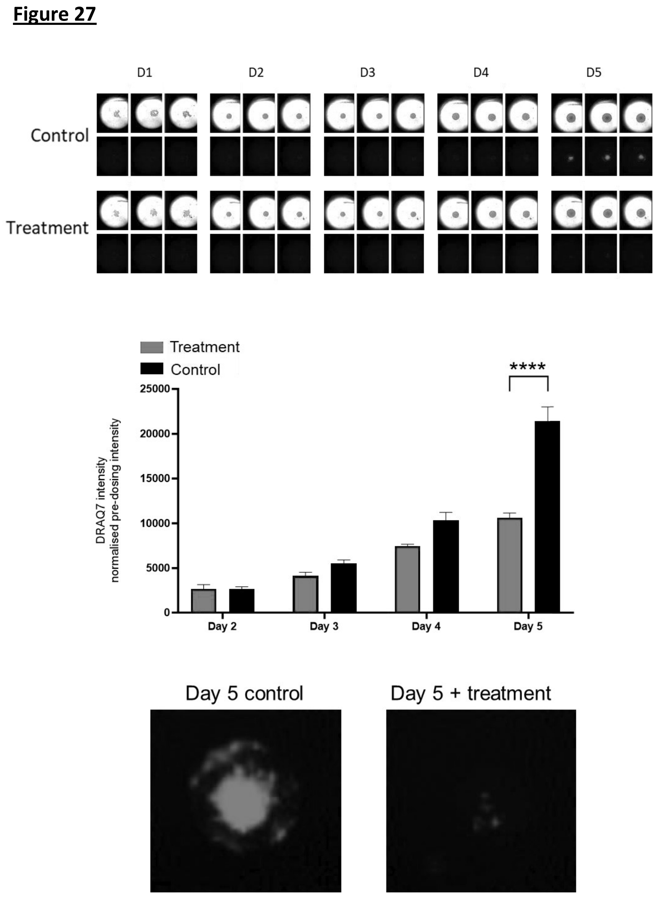

**Figure 27** **is a time series of HEK293 spheroids with the addition of a combination of the endoplasmic reticulum /sarcoplasmic reticulum (ER/SR) calcium channel inhibitor Dantrolene and the gap junction inhibitor retinoic acid, and shows greatly reduced necrotic core formation, induced by hypoxic and nutrient deprivation stress, through time.** Treatment (added on day 2) is Dantrolene and retinoic acid. The combination is followed through time up to 5 days and shows robust necrotic core suppression in all 3 trials. Top: Bright field images above fluorescence (DRAQ7^{™}; viability dye that stains necrotic cells; red) images of spheroids in the well of multi-welled plates. Middle: Bar graphs with s.t.d showing cell death stain DRAQ7^{™} intensity through time in the treatment and control group. The left-hand bar shows the treatment group, and the right-hand bar shows the control group. Bottom: example fluorescence (DRAQ7^{™}; viability dye that stains necrotic cells; red) images of spheroids in the well of multi-welled plates. Three trials (n=2 per trial). *p<0.05, **P<0.01, ***P<0.001, ****P<0.0001 by two-way ANOVA (Bonferroni correction).

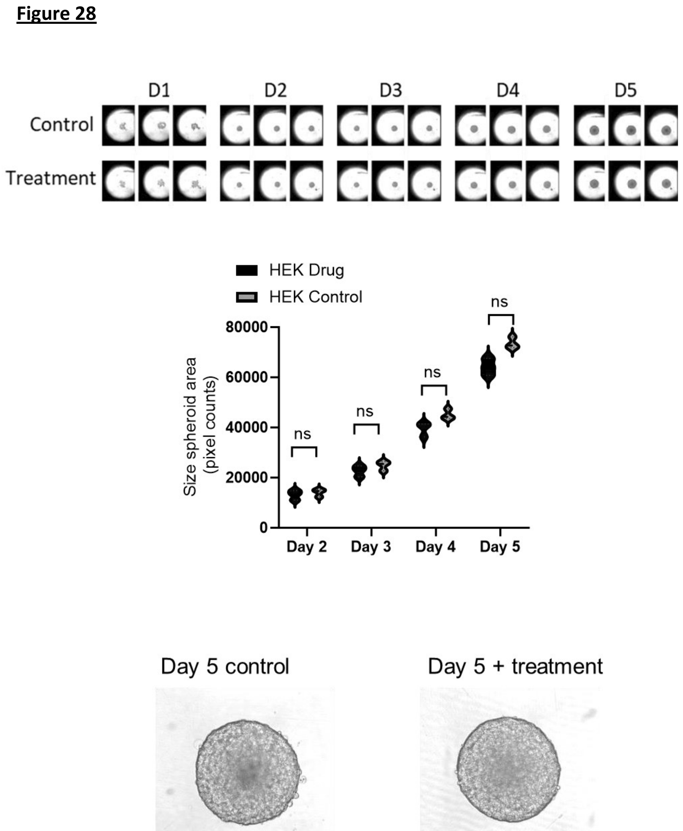

**Figure 28** **is a time series of HEK293 spheroids with the addition of a combination of the endoplasmic reticulum /sarcoplasmic reticulum (ER/SR) calcium channel inhibitor Dantrolene and the gap junction inhibitor retinoic acid, and shows no significant toxic effect of the combination; spheroid formation, growth, size and appearance appears normal.** Treatment (added on day 2) is Dantrolene and retinoic acid. The combination is followed through time up to 5 days and shows no significant effect on spheroid formation, growth, size or appearance. Top: Bright field images of spheroids in the well of multi-welled plates. Middle: Violin plot of spheroid size through time, a marker of formation and growth, showing the distribution of the data with quartiles as dotted lines. The left-hand plot shows the treatment ("drug") group, and the right-hand plot shows the control group. Bottom: example bright filed images of spheroids in the well of multi-welled plates, showing a normal morphology and appearance and no loss of integrity of the spheroid with the treatment. Three trials (n=2 per trial). *p<0.05, **P<0.01, ***P<0.001, ****P<0.0001 by two-way ANOVA (Bonferroni correction).

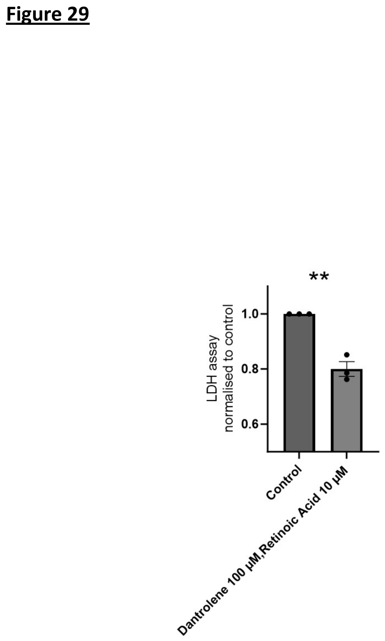

**Figure 29** **shows that Hepg2s spheroids show greatly reduced necrosis as measured by an lactate dehydrogenase LDH-Glo^{™} assay, induced by necrotic cores from hypoxic and nutrient deprivation stress, with the addition of a combination of the endoplasmic reticulum /sarcoplasmic reticulum (ER/SR) calcium channel inhibitor Dantrolene and the gap junction inhibitor retinoic acid.** Treatment is Dantrolene and retinoic acid used in combination. Three trials (n=2 per trial). Bar graphs with s.e.m. showing cell death. *P<0.05, **P<0.01, ***P<0.001, ****P<0.0001 by unpaired two-tailed t-test.

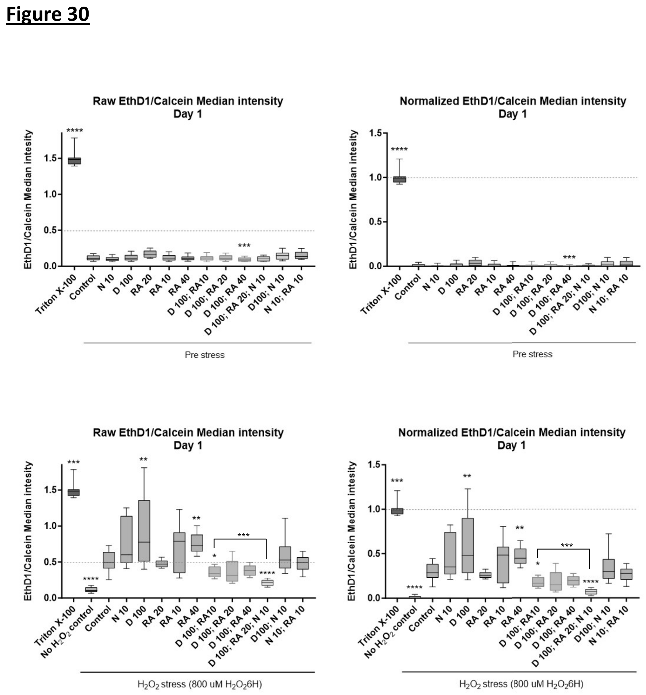

**Figure 30** **shows human intestinal fibroblast primary cell spheroids show a (more than additive) large reduction in necrosis, as measured by the standard viability stain Calcein AM/Ethidium homodimer-1, following oxidative stress when treated with either (i) a combination of an Endoplasmic reticulum /Sarcoplasmic reticulum (ER/SR) calcium channel inhibitor with a gap junction inhibitor, or (ii) a combination of an Endoplasmic reticulum /Sarcoplasmic reticulum (ER/SR) calcium channel inhibitor with a gap junction inhibitor and a plasma membrane calcium channel inhibitor as a preventative measure, but not other combinations.** Red: a combination of an Endoplasmic reticulum /Sarcoplasmic reticulum (ER/SR) calcium channel inhibitor with a gap junction inhibitor. Specifically, the combinations in red are those labelled "D 100; RA 10", "D 100; RA 20" and "D 100; RA 40". D refers to Dantrolene, and RA refers to retinoic acid. The numerical value refers to the dosage, e.g., 100 refers to treatment with 100µM of the compound. Blue: a combination of an Endoplasmic reticulum /Sarcoplasmic reticulum (ER/SR) calcium channel inhibitor with a gap junction inhibitor and a plasma membrane calcium channel inhibitor. Specifically, the combination in blue is that labelled "D 100; RA 20; N 10". N refers to Nifedipine. Stress is exposure to hydrogen peroxide (800µM) for 6 hrs. Top: pre stress data and Bottom: post-stress data. Left: raw data of the ratio of Ethidium homodimer-1 over Calcein AM intensity. Right: data normalised to Triton X-100 that kills all cells, so the percentage of necrosis can be gauged. A combination of (i) an Endoplasmic reticulum /Sarcoplasmic reticulum (ER/SR) calcium channel inhibitor with a gap junction inhibitor, and (ii) a combination of an Endoplasmic reticulum /Sarcoplasmic reticulum (ER/SR) calcium channel inhibitor with a gap junction inhibitor and a plasma membrane calcium channel inhibitor have a significant and more than additive effect on necrosis, compared to the single compounds in the combination that show either no significant difference or a worse effect to control. The combination of an Endoplasmic reticulum /Sarcoplasmic reticulum (ER/SR) calcium channel inhibitor with a gap junction inhibitor and a plasma membrane calcium channel inhibitor is better than the combination of an Endoplasmic reticulum /Sarcoplasmic reticulum (ER/SR) calcium channel inhibitor with a gap junction inhibitor. Other combinations, including (i) the combination an Endoplasmic reticulum /Sarcoplasmic reticulum (ER/SR) calcium channel inhibitor with a plasma membrane calcium channel inhibitor, and (ii) the combination a gap junction inhibitor with a plasma membrane calcium channel inhibitor, show no significant difference to stressed control. Box and whiskers plots (10-90 percentile). Two trials (n=31 per trial). *P<0.05, **P<0.01, ***P<0.001, ****P<0.0001 by Kruskal-Wallis test (Dunn's multiple comparisons correction).

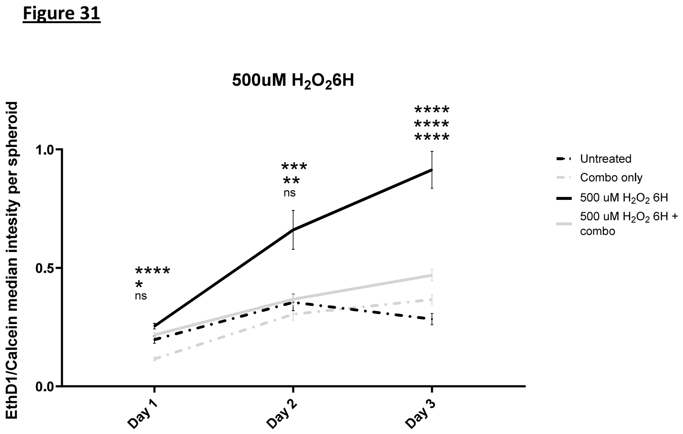

**Figure 31** **shows human intestinal fibroblast primary cell spheroids show a large reduction in necrosis, as measured by the standard viability stain Calcein AM/Ethidium homodimer-1, following mild oxidative stress over a long duration when treated with a combination of an Endoplasmic reticulum /Sarcoplasmic reticulum (ER/SR) calcium channel inhibitor with a gap junction inhibitor as a preventative and treatment measure.** The ER/SR calcium channel inhibitor used was Dantrolene, and the gap junction inhibitor used was retinoic acid. Stress is exposure to hydrogen peroxide (500µM) for 6 hrs. Line graphs showing mean and s.e.m. Two trials (n=31 per trial). *P<0.05, **P<0.01, ***P<0.001, ****P<0.0001 by Two-way ANOVA (Dunn's multiple comparisons correction). At each data point (i.e., at day 1, day 2 and day 3), the stars are as follows: top, stressed control vs stressed treatment with combo (i.e., **** at day 1, *** at day 2, and **** at day 3); middle, stressed control vs untreated control (i.e., * at day 1, ** at day 2, and **** at day 3); bottom, stressed treatment vs untreated control (i.e., ns at day 1, ns at day 2 and **** at day 3).

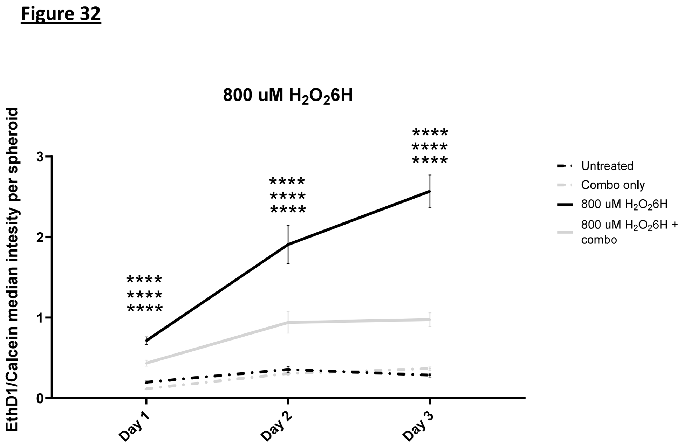

**Figure 32** **shows human intestinal fibroblast primary cell spheroids show a large reduction in necrosis, as measured by the standard viability stain Calcein AM/Ethidium homodimer-1, following moderate oxidative stress over a long duration when treated with a combination of an Endoplasmic reticulum /Sarcoplasmic reticulum (ER/SR) calcium channel inhibitor with a gap junction inhibitor as a preventative and treatment measure.** The ER/SR calcium channel inhibitor used was Dantrolene, and the gap junction inhibitor used was retinoic acid. Stress is exposure to hydrogen peroxide (800µM) for 6 hrs. Line graphs showing mean and s.e.m. Two trials (n=31 per trial). *P<0.05, **P<0.01, ***P<0.001, ****P<0.0001 by Two-way ANOVA (Dunn's multiple comparisons correction). At each data point (i.e., at day 1, day 2 and day 3), the stars are as follows: top, stressed control vs stressed treatment with combo (i.e., **** at day 1, **** at day 2, and **** at day 3); middle, stressed control vs untreated control (i.e., **** at day 1, **** at day 2, and **** at day 3); bottom, stressed treatment vs untreated control (i.e., **** at day 1, **** at day 2, and **** at day 3).

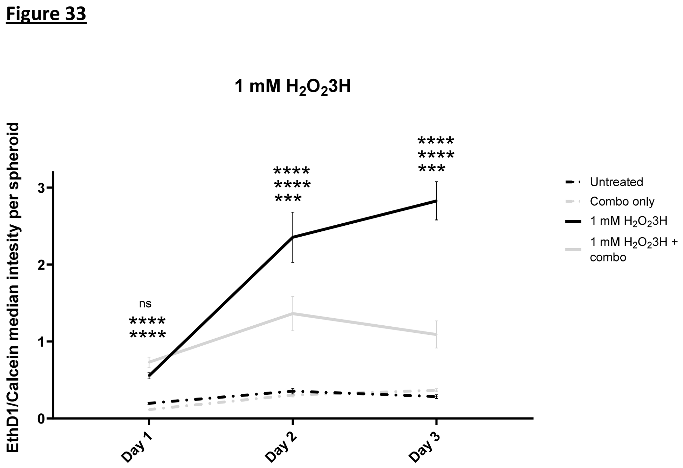

**Figure 33** **shows human intestinal fibroblast primary cell spheroids show a large reduction in necrosis, as measured by the standard viability stain Calcein AM/Ethidium homodimer-1, following severe oxidative stress over a moderate 3 hr duration when treated with a combination of an Endoplasmic reticulum /Sarcoplasmic reticulum (ER/SR) calcium channel inhibitor with a gap junction inhibitor as a preventative and treatment measure.** The ER/SR calcium channel inhibitor used was Dantrolene, and the gap junction inhibitor used was retinoic acid. Stress is exposure to hydrogen peroxide (1 mM) for 3 hrs. Line graphs showing mean and s.e.m. Two trials (n=31 per trial). *P<0.05, **P<0.01, ***P<0.001, ****P<0.0001 by Two-way ANOVA (Dunn's multiple comparisons correction). At each data point (i.e., at day 1, day 2 and day 3) the stars are as follows: top, stressed control vs stressed treatment with combo (i.e., ns at day 1, **** at day 2, and **** at day 3); middle, stressed control vs untreated control (i.e., **** at day 1, **** at day 2, and **** at day 3); bottom, stressed treatment vs untreated control (i.e., **** at day 1, *** at day 2, and *** at day 3).

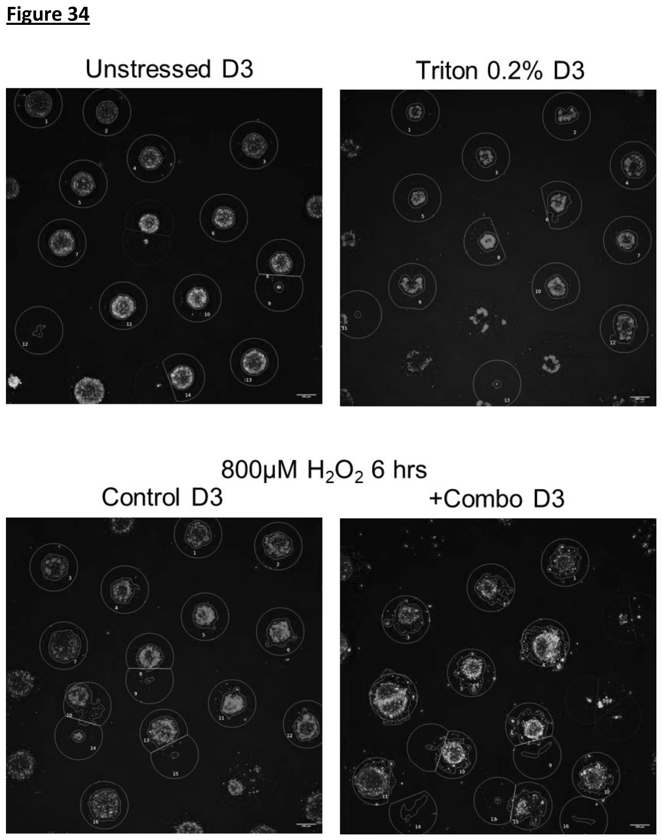

**Figure 34** **shows exemplary images of human intestinal fibroblast primary cell spheroids showing a large reduction in necrosis, as measured by the standard viability stain Calcein AM/Ethidium homodimer-1, following moderate oxidative stress over a long duration when treated with a combination of an Endoplasmic reticulum /Sarcoplasmic reticulum (ER/SR) calcium channel inhibitor with a gap junction inhibitor as a preventative and treatment measure.** The ER/SR calcium channel inhibitor used was Dantrolene, and the gap junction inhibitor used was retinoic acid. Stress is exposure to hydrogen peroxide (800µM) for 6 hrs. Green: viable cells stained with Calcein AM; Red: dead necrotic cells stained with Ethidium homodimer-1. Around 16 spheroids can be seen per image. Triton X-100 that kills all cells images displayed, so the percentage of necrosis can be gauged. All images taken on day 3 of the experiment (c.f. Figure 32 that these images relate to).

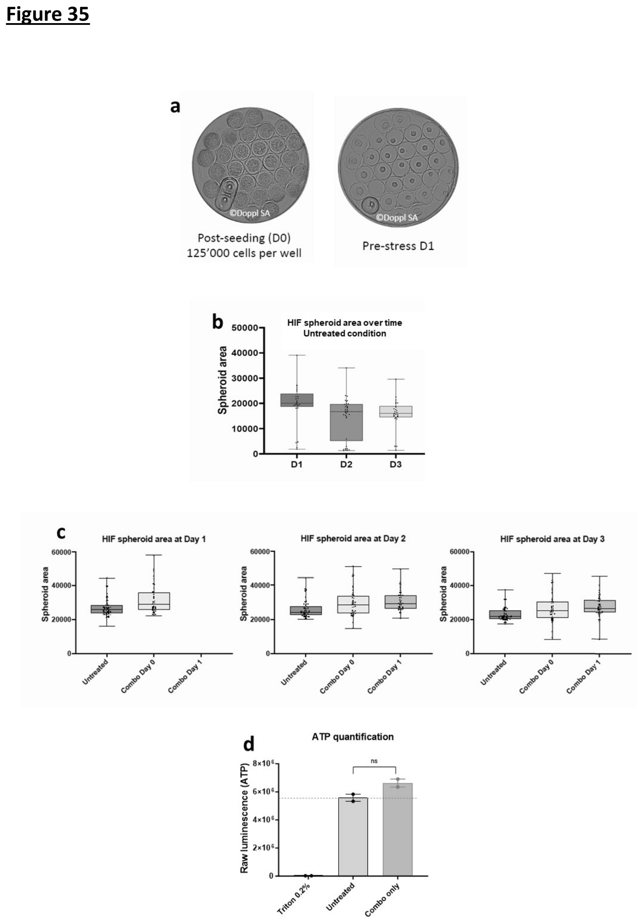

**Figure 35** **showing human intestinal fibroblast primary cell spheroids with the addition of a combination of the Endoplasmic reticulum /Sarcoplasmic reticulum (ER/SR) calcium channel inhibitor Dantrolene and the gap junction inhibitor retinoic acid, and shows no significant toxic effect of the combination; spheroid formation, growth and size, as well as ATP levels are maintained. (a)** Image of cells aggregating to form spheroids by day 1. Spheroids are spherical cellular aggregates supporting cell-cell and cell-matrix interactions in an environment that mimics the real-world situation. **(b)** Spheroid size in the control shows no major change from day 1 to day 3. **(c)** Treatment added on day 0 or day 1 is Dantrolene and retinoic acid. The combination is followed through time up to 3 days and shows no significant effect on spheroid size or growth, i.e. change in size with time. Three trials (n=30 per trial). (d) ATP presence measured on day 3 using a CellTiter-Glo^{®} 3D Cell Viability Assay shows no reduction in ATP levels with treatment added on day 1. Bar graph with raw data points displayed. Two trials (n=31 per trial). *p<0.05, **P<0.01, ***P<0.001, ****P<0.0001 by One-way ANOVA (Šídák's multiple comparisons correction).

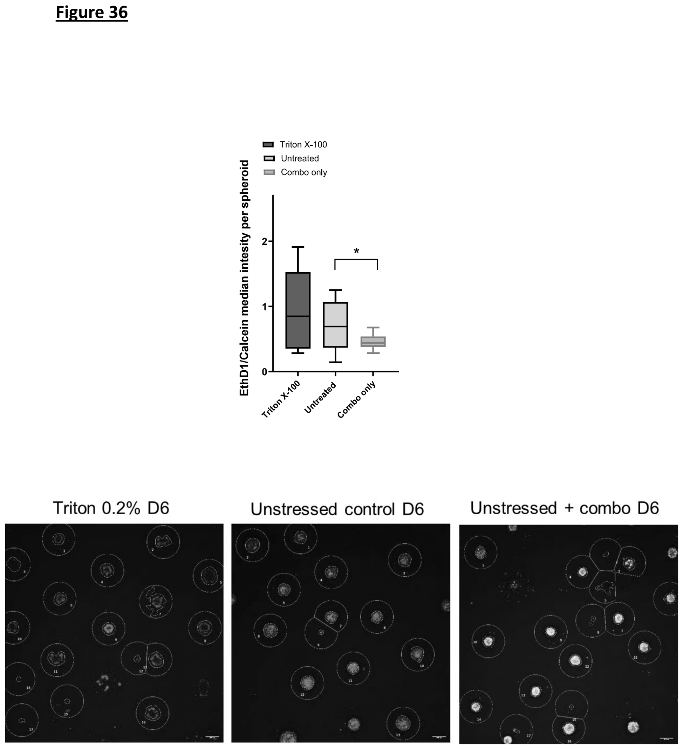

**Figure 36** **shows images of human intestinal fibroblast primary cell spheroids showing a large reduction in necrotic core formation, induced by hypoxic and nutrient deprivation stress, on day 6, as measured by the standard viability stain Calcein AM/Ethidium homodimer-1, when treated with a combination of an Endoplasmic reticulum /Sarcoplasmic reticulum (ER/SR) calcium channel inhibitor with a gap junction inhibitor.** The ER/SR calcium channel inhibitor used was Dantrolene (100 µm), and the gap junction inhibitor used was retinoic acid (10 µm). Top: Box and whiskers plots (10-90 percentile); data normalised to Triton X-100 that kills all cells. Two trials (n=31 per trial). *P<0.05, **P<0.01, ***P<0.001, ****P<0.0001 by Kruskal-Wallis test (Dunn's multiple comparisons correction). Bottom: Example images. Green: viable cells stained with Calcein AM; Red: dead necrotic cells stained with Ethidium homodimer-1. Around 16 spheroids can be seen per image.

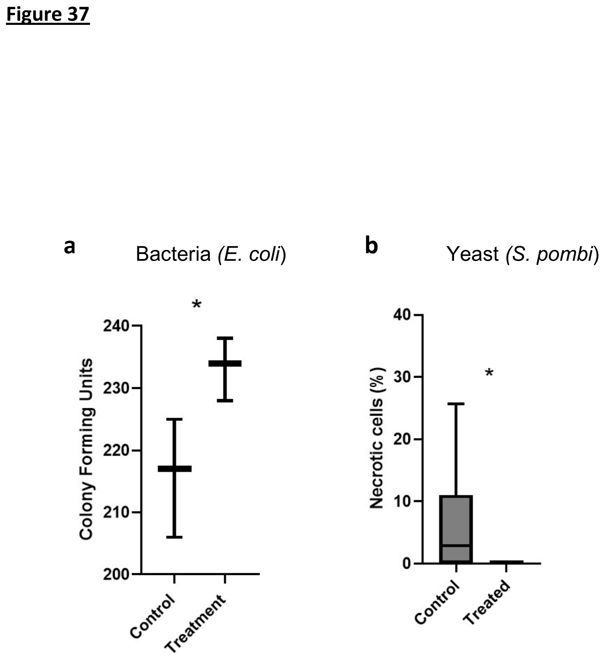

**Figure 37** **shows that *E. coli* and *S. pombi* death is prevented with drug that supresses necrosis following oxidative stress (stress is for 50 min 1 mM H₂O₂). (a)** *E. coli* bacteria. Treatment is EGTA 2500 µM during stress. Following stress, the bacteria is washed in buffer, diluted 1:500 in MilliQ water, seeded onto agar plates containing bactopeptone and then left at 37 °C overnight, followed by the counting of colony forming units (CFU). N=3 trials. **(b)** *S. pombi* yeast*.* Treatment is EGTA 2500 µM during stress. Following stress, the yeast is washed and stained with *Propidium iodide* (PI; red) which enters necrotic cells. Box and whiskers (Tukey) of % of necrotic cells. *(N=6-7 images, combined data of 2 trials).* *P<0.05 by unpaired two-tailed t-test.

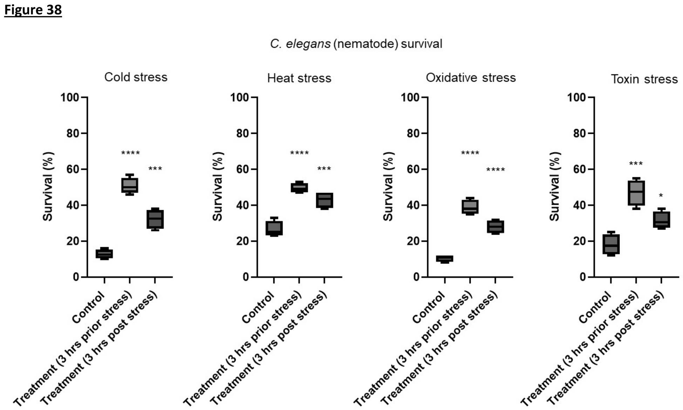

**Figure 38** **shows that *C. elegans* death is prevented following a variety of stressors including heat, cold/freeze, oxidative and toxin stress, when treated with drug that supresses necrosis.** From left to right: Cold stress (-80°C for 1.5 min); Heat stress (37°C for 4.5 hrs); Oxidative stress (2M hydrogen peroxide for 5 min); and toxin stress (1.3%(v/v) *tert-butylhydroperoxide (tBOOH)* for 5 min). Treatment is EGTA 2500 µM in liposomes given as a preventative measure 3 hrs prior to stress or as a treatment 3 hrs post stress. Box and whiskers (Tukey) plots. 3 trails (n=10 per trial). *P<0.05, ***P<0.001, ****P<0.0001, by One-way ANOVA (Dunnett correction).

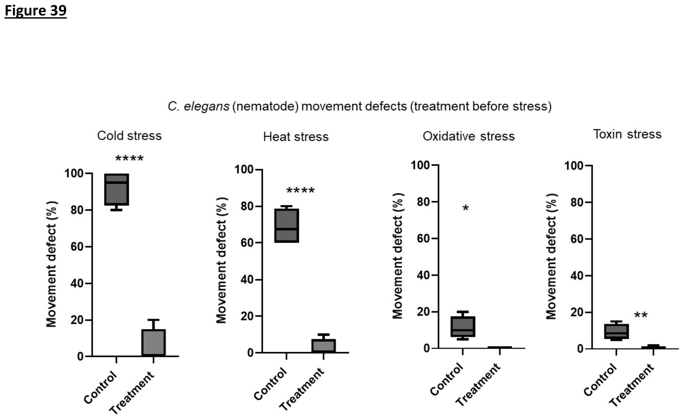

**Figure 39** **shows that necrosis mutants show reduced movement defects post a variety of stressors including heat, cold/freeze, oxidative and toxin stress, when treated with drug that supresses necrosis.** From left to right: Cold stress (-80°C for 1.5 min); Heat stress (37°C for 4.5 hrs); Oxidative stress (2M hydrogen peroxide for 5 min); and toxin stress (1.3%(v/v) *tert-butylhydroperoxide (tBOOH)* for 5 min). Treatment is EGTA 2500 µM in liposomes given 3 hrs prior stress. Box and whiskers (Tukey) plots. 3 trails (n=10 per trial). *P<0.05, ***P<0.001, ****P<0.0001 by One-way ANOVA (Dunnett correction). Worms that show slowed movement (are typically still and only move when poked with a worm pick or may not move at all), are scored and the percentage of animals with a movement defect displayed (y-axis).

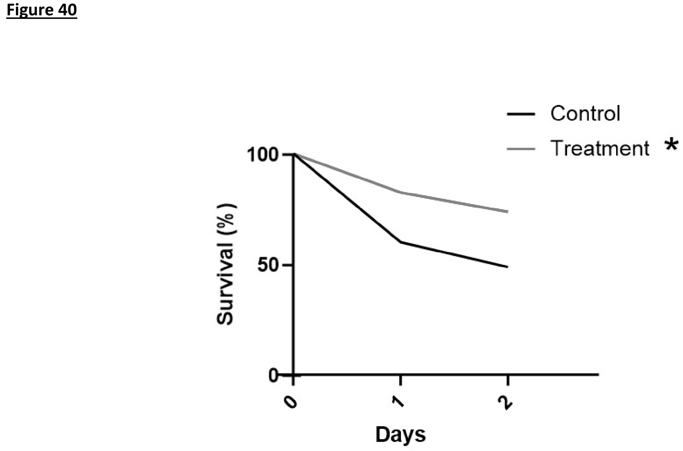

**Figure 40** **shows that *D. melanogaster death* is prevented following cold/freeze stress, when treated with drug that supresses necrosis.** Stress is keeping animals at 0 degrees for 10 min. *D. melanogaster* W1118 animals are aged to day 7, starved for 8 hrs and then fed for 12 hrs with food containing 0 or 2500µM EGTA. This is followed by the cold/freeze stress and then survival measured every 12 hrs following. Treatment showed a significant difference to control. *P<0.05 by log-rank analysis

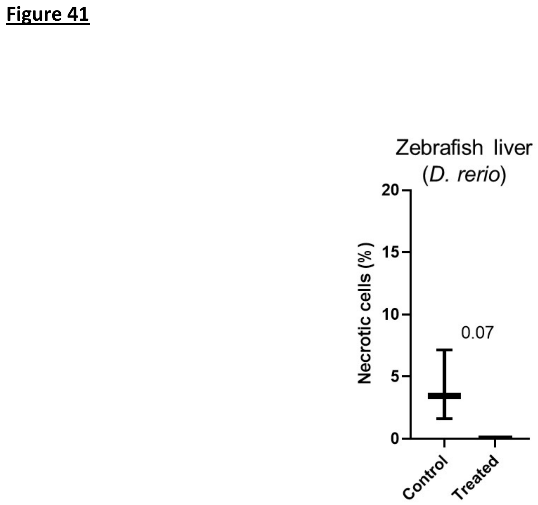

**Figure 41** **shows that Zebra fish *(Danio rerio)* liver shows necrosis cell death is prevented by drug that supress necrosis following oxidative stress (7 min in 1 mM H₂O₂).** Treatment is 2500µM EGTA during stress. Following stress, liver is washed and stained with *Propidium iodide* (Pl; red)which enters necrotic cells. Tissue is also stained with DAPI (4'6-diamidino-2-phenylindole) that enters all cells (blue) so total number of cells present in the image can be analysed. % of necrotic cells (PI stain), relative to all cells seen (DAPI stain); Box and whiskers plot (Tukey). 3 trials. P = 0.07 by unpaired two-tailed t-test.

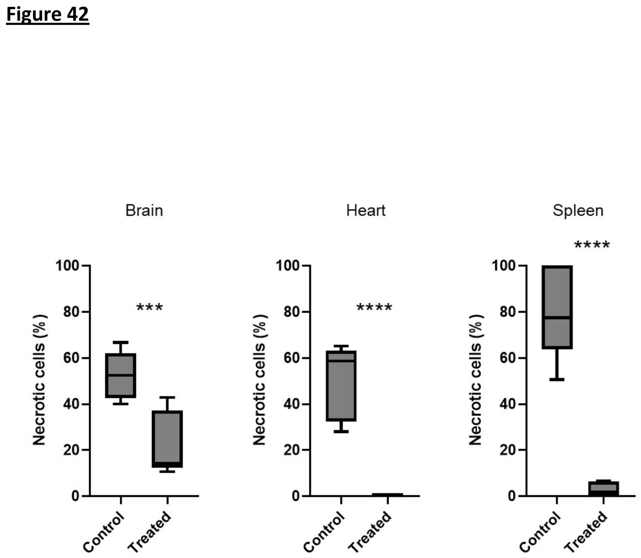

**Figure 42** **shows that mouse organs show necrosis cell death is prevented by drug that supress necrosis following hypoxia stress (2.5 hrs in N₂ gas bubbled buffer).** Organotypic slices of tissue are prepared and placed in buffer mimicking Cerebrospinal fluid (CSF) for brain tissue or Ringer's buffer for other tissue. Thickness of slices is as follows: heart: 0.210 mm; brain: 0.300 mm; Spleen: 300 mm. Treatment is 2500µM EGTA during stress. Following stress, slices are washed and stained with *Propidium iodide* (Pl; red) which enters necrotic cells. Tissue is also stained with DAPI that enters all cells (blue) so total number of cells present in the image can be analysed. % of necrotic cells (PI stain), relative to all cells seen (DAPI stain). Box and whiskers plot (Tukey). Combined data of 2 trials. ***P<0.001, **** P<0.0001 by unpaired two-tailed t-test.

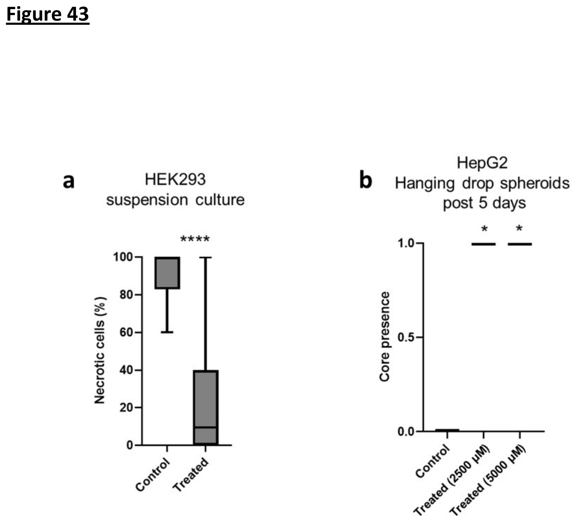

**Figure 43** **shows that regardless of the type of cell, type of cell culture or stress, necrosis cell death is prevented by drugs that supress necrosis. (a) HEK293 suspension culture following oxidative stress (20 see 1M H₂O₂).** Treatment is 2500µM EGTA 2 hrs prior as well as during stress. Following stress, cells are washed and stained with *Propidium iodide* (Pl; red) which enters necrotic cells. Cells are also stained with DAPI that enters all cells (blue) so total number of cells present in the image can be analysed. % of necrotic cells (PI stain), relative to all cells seen (DAPI stain). Box and whiskers plot (Tukey). Combined data of 2 trials. **** P<0.0001 by unpaired two-tailed t-test. **(b) Hepg2s spheroids made by hanging drop technique and placed on agarose pads show no necrotic core even after 5 days with the addition of drug that supress necrosis.** Treatment is 2500µM EGTA or 5000µM EGTA in spheroid culture. Spheroids, used in tissue engineering (spherical cellular aggregate supporting cell-cell and cell-matrix interactions in an environment that mimics the real-world situation), cannot be supported for long periods of time or grown large due to necrotic core formation (cells in the middle of the spheroid are starved of oxygen and nutrients and this causes cell death via necrosis). Box and whiskers plot (Tukey). Combined data of 3 trials. *P<0.05, ***P<0.001, **** P<0.0001 by unpaired two-tailed t-test.

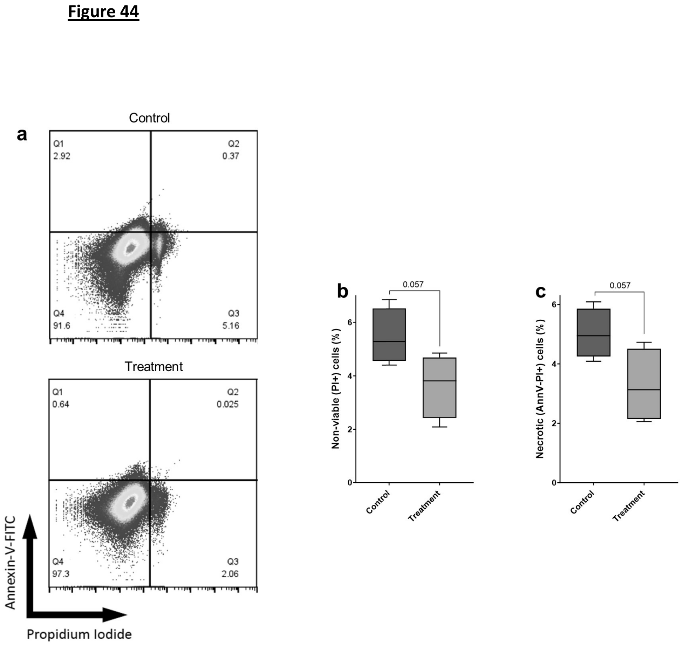

**Figure 44****. Drugs to supress necrosis (a combination of the Endoplasmic reticulum /Sarcoplasmic reticulum (ER/SR) calcium channel inhibitor Dantrolene and the gap junction inhibitor retinoic acid) can be added to the gold standard organ/graft preservation media, University of Wisconsin (UW) media to increase cell viability and reduce cell necrosis during warm and cold storage and so increase organ/graft quality.** Following acclimation, mice were anesthetized with isoflurane and cardiac perfusion was performed with 50mL UW buffer at 5mL/min containing either no treatment (i.e. control group) or treatment: 100µM Dantrolene and 10 µM Retinoid Acid. Kidneys were incubated in 5mL 4°C for 24h followed by 2h at 37°C prior to single cell isolation from whole kidney. Following washing, cells were stained with Annexin V and Propidium Iodide (PI) to determine the ratio of necrotic (Pl+ Annexin V-) and viable (PI-) cells and flow cytometry performed. Left (Fig 44a): example images of flow cytometry. Middle and right (Figs 44b and 44c): Non-viable dead cells and necrotic cells in control and treatment groups. 4 trials (n=1 kidney per trial). *P=0.057 by unpaired two-tailed t-test.

### Examples

### Materials and methods

### HEK293 and HepG2 cell spheroid production and drug screen in U-bottom plates: hypoxic stress.

Stand cell culture of HEK293T and HepG2 cells was performed (10% Fetal bovine serum in Dulbecco's Modified Eagle Medium). Cells were then transferred to either 96-well Nunc Sphera or 384-well PrimeSurface^{®} 3D Culture Spheroid plates (ultra-low attachment (ULA) plates) using an initial cell density of 500 cells. Following spheroid formation in the plates, treatment compound(s) were solubilised in DMSO (dimethylsulfoxide) to get a final assay DMSO concentration of 1 % v/v and then added to the ULA plate wells. Media changes were on alternative days. Brightfield phase contrast and fluorescent image acquisition was performed at regular intervals up until 6 days post-treatment using an Incucyte^{®} S3 live-cell analysis system. The standard viability dye DRAQ7^{™} (that only stains the nuclei in dead and permeabilised cells) was used as a marker of cell death. All conditions were tested in duplicate on three separate occasions (i.e. three independent trials were performed). DMSO controls were added on each plate. Necrotic core size was quantified as the percentage change from the average of DMSO control wells based on fluorescence area normalised to spheroid size. As a secondary indicator of cell death, a lactate dehydrogenase LDH-Glo^{™} assay was performed on supernatants from the treated well and control wells.

### Human fibroblast primary cells cell spheroid production and drug screen in U-bottom plates: oxidative stress and hypoxic stress.

Stand cell culture of Human intestinal fibroblast primary cells was performed. Cells were then transferred to 384-multi well plates (Gri3D product number Gri3D-96P-S-96-800). Seeding density per well was 125,000 cells, with 4000 cells per spheroid within the wells forming. Following spheroid formation in the plates, treatment compounds were solubilised in DMSO to get a final assay DMSO concentration of 0.5% v/v and then added to the plate wells. Media changes were on alternative days. Triton X-100 was used as a positive control marker of cell death. Drug treatment was done overnight prior stress. Brightfield phase contrast and fluorescent image acquisition was performed at regular intervals immediately post stress, and on subsequent days post stress up to day 3 using a Tecan Spark instrument for brightfield (model number Spark Cyto 600) and a GE Healthcare IN Cell instrument for fluorescence (model number IN Cell Analyzer 2200); images were processed using Doppl's proprietary pipelines. Stress was exposure to varying levels of hydrogen peroxide for different time durations as stated in the figure legends. The standard viability Calcein AM/Ethidium homodimer-1 staining kit (Thermo Molecular Probes) was used as a marker of cell death (3 hrs incubation at 37°C prior stress). To determine the presence of metabolically active viable cells in 3D cell culture, a quantitation of the level of ATP present was performed using the CellTiter-Glo^{®} 3D Cell Viability Assay. For this, 150µl of supernatant is collected per well, followed by the addition of 50µl CTG and mixing for 5 minutes. Luminescence was then measured after 30 minutes of incubation at r.t.p. on day 1 and day 3 or 6. Two repeats of all conditions were performed. Each repeat used 31 organoids per condition. Readouts were resistance against hydrogen peroxide stress and necrotic core formation (i.e. hypoxic stress) through time; and cell viability gauged from metabolic activity, i.e. ATP levels. DMSO controls were assessed in parallel on each plate. Level of dead organoid tissue was quantified as percentage change from DMSO control wells based on fluorescence area normalised to spheroid size.

### Viability Analysis of Perfused Mouse Kidneys by Flow Cytometry.

8 animals were acclimated to a vivarium 7 days prior to the study initiation. After acclimation, animals were randomized by body weight, and culled and cardiac perfused with UW (University of Wisconsin) preservation media with and without treatment compounds added. Intact right kidneys were then extracted from the animal and stored in UW with or without treatment compounds in hypothermic (4°C) conditions for 24 hrs, followed by 2 hrs at 37°C before processing for analysis. Kidney Processing was done by dissociation manually by mincing and digestion into a single cell suspension using Liberase TM, collagenase, dispase, and Dnase I. Cells were then passed through a 70 µM filter to remove debris and generate a single cell suspension suitable for flow cytometry. For flow cytometry analysis, kidney cells were stained with FITC-Annexin V (AnnV) and propidium iodide (PI) and analyzed for the following: (i) AnnV-PI+ for necrotic cells; and (ii) PI+ for non-viable dead cells. Cells were then acquired on a BD Accuri C6 Flow Cytometer, on FL-1 for Annexin V-FITC and FL-3 for PI.

### HEK293 suspension culture oxidative stress

HEK293 cells were detached using trypsin, washed and placed in cell culture medium. Cells were pretreated for 2 hrs before and during stress with or without (control) EGTA 2500 µM. Cells were stressed by placing them in a 1M hydrogen peroxide solution (in cell culture medium) for 20 sec. Cells were then washed in cell culture medium and stained with PI as well as DAPI to visualise all cell nuclei (including intact cell nuclei), and placed on a microscope slide under a coverslip and imaged.

### Preparation of 3D HepG2 cell culture spheroids

Multi-cellular spheroids were prepared using a hanging drop method. A stock solution of methylcellulose (MC) was prepared by dissolving 1.2 g of MC powder (4000 cP, Sigma-Aldrich) in 100 mL of DMEM. The solution was magnetically stirred overnight at 4°C and subsequently centrifuged at 4,000 g for 3 hrs. HepG2s cells were re-suspended in cell medium containing 20 % MC stock solution at a concentration of 20,000 cells per 50 µL spheroid. 50 µL drops were subsequently pipetted onto a non-adherent petri dish (Greiner) and, replacing the lid, the dish was turned upside-down and incubated overnight at 37°C. Multi-cellular spheroids were harvested individually with a wide bore pipette or collectively by adding 5 mL cell medium to the dish, collecting and centrifuging the suspension at 300 g for 5 min and resuspending the spheroids in cell medium. Spheroids were then cultured for 5 days in 48 well plates containing 2.5 % agarose gel and 400 uL cell medium. EGTA was added at 0 and 2500 µM to both the spheroid pre-cursor solution and the cell culture medium. At day 3 and day 5 the spheroids were imaged and assessed.

### Mouse organotypic slice preparation and culture

For organotypic cultures, sagittal hippocampal brainslices were taken from wild type mice. Following decapitation, all organs were quickly removed, placed in ice-cold buffer (artificial cerebrospinal fluid (ACSF) [in mmol/L]:NaCl 125, KCl 2.4, NaHCO₃ 26, NaH₂PO₄ 1.25, glucose 25, CaCl₂ 2 [0.5 mmol/L for dissection], MgCl₂ 1 [5 mmol/L for dissection]; bubbled with carbogen) for brain, and for all other organs Ringers buffer. For brain, to reduce the rate of cell death most dissections were carried out with high-magnesium (5 mmol/L)/low-calcium (0.5 mmol/L) ACSF. The brain was hemisected and a section was cut away by hand that was approximately 100° from the midline surface. The hemispheres were then mounted with superglue on to a slicing stage and 300 µm hippocampal slices were cut with a vibroslicer and transferred to an incubating chamber with circulating ACSF (bubbled with carbogen). All other organs were treated similarly but Ringer's buffer was used instead of ACSF. For the heart and spleen, cross sectional slices going fully across the organ were taken. The slices were incubated at 35°C for approximately 1 hrs before the incubator was switched off and the bath was allowed to cool to room temperature.

For stress, slices were transferred to beakers (containing ACSF or Ringers buffer, for brain and other organs, respectively) that had N₂ bubbled through for 3 hrs prior rather than carbogen. They were left in the beakers with no gas bubbling to mimic hypoxia. Treated beakers had drug solubilised at the given concentration in the N₂ bubbled buffer. Following this, the slices were stained with PI as well as DAPI to visualise all cell nuclei (including intact cell nuclei). Slices were then placed on a microscope slide under a coverslip and imaged.

### Preparation of Zebra fish liver

Freshly culled zebrafish (*Danio rerio*) was dissected to quickly remove the liver which was then placed in PBS buffer and immediately stressed with 1 mM hydrogen peroxide for 7 min in with or without 2500 µM EGTA. The liver was then washed in PBS to stop the stress and stained with PI as well as DAPI to visualise all cell nuclei (including intact cell nuclei). The liver was then placed on a microscope slide under a coverslip and imaged.

### C. elegans culture methods

*C*. *elegans* was maintained on nematode growth medium (NGM) agar plates seeded with *Escherichia coli* OP50, using standard culture methodologies (S. Brenner, 1974). A *C*. *elegans* (N2H) hermaphrodite stock was used as wild-type. Day 1 hermaphrodites were used in experiments and all maintenance and trials were conducted at 20°C unless otherwise stated.

### C. elegans stress assays

### • Cold/freeze stress

Per trial, 100 animals were placed in 1.5 ml Eppendorf tubes (30 animals per tube) containing 100 µl M9 buffer, and then transferred to a -80°C incubator for 1.5 min. Following the stress, animals were transferred to a 20°C incubator and maintained as usual on OP50 NGM plates.

### • Heat stress

Per trial, 100 animals were placed in a 37°C incubator for 4.5 hrs on NGM plates. Following the stress, animals were transferred to a 20°C incubator and maintained as usual on OP50 NGM plates.

### • Oxidative stress

Per trial, 100 animals were transferred into 1.5 ml Eppendorf tubes (20 animals per tube) containing 300 µL of 2M hydrogen peroxide for 5 min. Following the stress, animals were transferred to a 20°C incubator and maintained as usual on OP50 NGM plates.

### • Toxin stress

Per trial, 100 animals were transferred into 1.5 ml Eppendorf tubes (20 animals per tube) containing 300 µL of 1.3%(v/v) *tert-butylhydroperoxide (tBOOH)* for 5 min. Following the stress, animals were transferred to a 20°C incubator and maintained as usual on OP50 NGM plates.

### Movement analysis post stress in C. elegans

Movement was scored according to an adapted methodology (from Herndon et al., 2002). Briefly, animals were marked as either: normal movement; or defective movement. Defective movement was defined as worms that are stationary unless touched with a pick, in which case they may only move a few centimetres forward before stopping again or they may not move at all. 100 animals were used per trial and the percentage of worms with defective movement was scored.

### Liposome mediated drug delivery in C. elegans

According to the protocol in Shibamura et al., 2009, drug was dissolved in MilliQ water and encapsulated in liposomes. Drug used was EGTA, at a final concentration of 2500 µM. Briefly, L-α-phosphatidylcholine (LaPC) was used to prepare liposomes by first dissolving LaPC in distilled water at the concentration of 96 mg/ml. A x2 concentration of drug solution was mixed with LaPC to a 1:1 ratio and heated to 65°C. Drug-LaPC solution was then converted into liposomes using a mini-Avanti extruder a 100 nm pore size polycarbonate membrane. MilliQ water encapsulated in liposomes was used a as the empty vector control. Following preparation, 25 µl of the liposome drug solution was spread onto a 25 µl spot OP50 lawn on NGM plates and worms were then picked onto the plates.

### Drosophila culture and stress.

Animals were under standard culture conditions. On day 1 of adulthood, they were starved for 8 hrs, then fed 12 hrs +/- EGTA 2500 µM. Following this, the animals were moved to 0°C for 10 min and then recovered and survival measured.

### E. coli oxidative stress.

2 loops of *E. coli* stationary-phase bacterial cells collected post seeding (80 µL from a culture grown for 12 hrs) on agar plates containing bactopeptone and allowing to grow for 24 hrs was collected in buffer. Bacterial cells were then stressed for 50 min using 1 mM hydrogen peroxide. Treatment is EGTA 2500 µM during stress. Cells were then washed in buffer. Following stress, bacteria was washed in buffer and then diluted 1:500 in MilliQ water and seeded onto agar plates containing bactopeptone and left at 37 °C overnight. This was followed by counting colony forming units (CFU) counted. Three repeats were performed.

### S. pombi oxidative stress.

A culture of *S*. *pombi* was exposed to 1 mM hydrogen peroxide for 50 min. Following this the yeast cells were washed in buffer and stained with the dye Propidium iodide (PI) and placed on a microscope slide under a coverslip and imaged using Nomarski and epifluorescence microscopy to identify the percentage of yeast cells that underwent necrosis. Treatment is EGTA 2500 µM during stress. Two repeats were performed.

### Examples

Doppl SA, (affiliated with EPFL, the Swiss Federal Institute of Technology in Lausanne); Domainex, Cambridge UK; and ICHOR life sciences NY, US performed data collection.

### Example 1. Necrosis can be prevented and/or treated by blocking the "predicted weak spots" of cell physiology. One such means is using relatively high dosages of calcium chelating agents.

Traditionally necrosis is viewed as a chaotic process that cannot be intervened in. It has been assumed that necrosis is the consequence of numerous random events that cause loss of cell homeostasis.

As discussed above, the inventors believe that: following any stress, many random events follow that may culminate in loss of cell homeostasis and necrosis. However, there are certain initiated events that make a cell more vulnerable to stress (i.e., cellular weak spots that cause more damage than other processes). Suppression of these *predicted weak spots* should make a cell more resistant to stress, i.e., necrosis can be prevented, and/or treated; a much greater amount of stress would then be required for any other random events to induce cell death. Downstream conditions, such as tissue damage and organ damage, can therefore also be prevented and/or treated. The *predicted weak spots,* illustrated in Figure 1, are: an increase in intracellular cytoplasmic concentration of free calcium ions due to movement of calcium (1a) across the cell plasma membrane, (1b) and from internal compartments where it is stored, primarily the endoplasmic reticulum (ER)/sarcoplasmic reticulum (SR). Following calcium entry, many proteins are activated in an unwanted manner. As part of this futile protein activation, (2) calpain proteins are activated. Calpains then destroy internal compartments including lysosomes, resulting in the activation and release of hazardous components of internal compartments like the lysosomes, including release of (3) cathepsin proteins. Finally, (4) movement of calcium ions across to neighbouring cells via gap junctions, results in the disruption of calcium homeostasis and triggering of necrosis in those neighbouring cells. Store-Operated Calcium Channels (SOCC), also called Calcium release-activated calcium (CRAC) channels, are another source of calcium entry into the cells. SOCC channels, however, only become relevant post ER/SR calcium depletion, and hence SOCC is less of a focus because SOCC is expected to be less of a weak spot than steps (1) - (4) discussed herein.

The inventors have discovered that a critical step in the occurrence of necrosis is the calcium ion increase in the cytosol, which may be due to any one of the 3 main points of calcium ion entry of into a cell: i.e., via cell surface membrane channels, gap junctions, and the endoplasmic reticulum (ER)/sarcoplasmic reticulum (SR). It is believed that, because plasma membranes are in contact with the environment surrounding the cell, they will show greater levels of re-enforcement compared to ER/SR calcium channels and gap junctions. Moreover, for cells in certain tissue types that are very tightly packed, calcium movement via ER/SR calcium channels and gap junctions will pose a greater threat during a stress.

Therefore, the inventors have made the surprising discovery that using a combination of compounds to block the ER/SR calcium channels and gap junctions is particularly advantageous for preventing and/or treating necrosis. This combination can be further improved by also blocking calcium ion entry via plasma membranes. Thus, the invention provides the combination therapy of an endoplasmic/sarcoplasmic reticulum calcium channel inhibitor (such as a ryanodine receptor antagonist), a gap junction inhibitor, and a plasma membrane calcium channel inhibitor. Additionally, or alternatively, the combination can be further improved by the use of other means to mimic reduction of cytosolic calcium ion concentration, such as through use of calcium chelating agents that sequester calcium (or use competitive inhibitors of calcium ions, such as other cations of a similar charge and size such as magnesium ions). In other words, the inventors made the surprising discovery that the most efficacious combination to prevent and/or treat necrosis is the simultaneous inhibition of ER/SR calcium channels and gap junctions, and this combination can be enhanced with the addition of plasma membrane calcium channel inhibitors and/or calcium chelating agents (or competitive inhibitors of calcium ions). Thus, the invention provides the combination therapy of an endoplasmic/sarcoplasmic reticulum calcium channel inhibitor (such as a ryanodine receptor inhibitor or antagonist) and a gap junction inhibitor. The invention also provides the combination therapy of an endoplasmic/sarcoplasmic reticulum calcium channel inhibitor (such as a ryanodine receptor inhibitor or antagonist), a gap junction inhibitor, and a plasma membrane calcium channel inhibitor. The invention also provides the combination therapy of an endoplasmic/sarcoplasmic reticulum calcium channel inhibitor (such as a ryanodine receptor inhibitor or antagonist), a gap junction inhibitor, and a calcium chelator. The invention also provides the combination therapy of an endoplasmic/sarcoplasmic reticulum calcium channel inhibitor (such as a ryanodine receptor inhibitor or antagonist), a gap junction inhibitor, a plasma membrane calcium channel inhibitor and a calcium chelator.

The rationale of the combination therapy of the present invention can be further illustrated by considering what may happen when other combinations of calcium ion channel blockers are used, as follows. The inventors believe that, if only calcium plasma membrane channels are blocked, necrosis may still occur to a large degree because calcium ions may still enter via the ER/SR and via gap junctions. If only ER/SR channels are blocked, necrosis may still occur to a large degree because calcium ions may still enter primarily via gap junctions. If only gap junctions are blocked, necrosis may still occur to a large degree because calcium ions may still enter primarily via ER/SR channels. If a combination of ER/SR channels and plasma membrane calcium ion channels are blocked, necrosis may still occur to a large degree because calcium ions still enter primarily via gap junctions. If a combination of gap junctions and plasma membrane calcium ion channels are blocked, necrosis may still occur to a large degree as calcium ions still enter primarily via ER/SR channels.

Blocking calpains and cathepsins (Figure 1) represents another therapeutic target for treating and/or preventing necrosis. Thus, a combination of a calpain inhibitor and a cathepsin inhibition is provided by the present invention. However, these are only two of the many downstream mediators of destruction following a rise in cytosolic calcium ion during cell necrosis. Inhibition of calpains and cathepsins may therefore not be the most efficacious form of intervention.

In some of the following Examples, human 3D cell culture was employed using liver Hepg2 cells. 3D cell culture is known to accurately replicate a cell's native environment. During embryonic development, cells aggregate and self-assemble into complex multi-cellular structures. The same aggregation process can likewise occur in *in vitro* culture systems if cell-cell cohesion is favoured over cell attachment to a substrate. The resulting 3D cell culture aggregates are known as spheroids. Spheroids exhibit extensive cell-cell adhesion, typically retain their endogenous extracellular matrix, and have properties that closely mimic their *in vivo* tissue counterparts in both structure and function. A standard method of spheroid formation in U-bottom low adhesion plates was used in the Examples herein.

Notably, spheroids typically cannot be grown to large sizes. This is because, as the spheroids grow, only the peripheral cells obtain oxygen and nutrients from the cell culture medium. The inner cells die from cell death necrosis, with the formation of large necrotic cores (observable though use of standard live dead stains). Thus, necrotic core formation represents a naturally occurring hypoxic and nutrient stress. This resembles multiple forms of shock in humans where blood flow carrying nutrients and oxygen is disrupted to certain cells, tissues and/or organs. To show that necrosis can be prevented and/or treated by the compositions and compounds of the present invention, some of the Examples herein make use of these naturally forming necrotic cores, in order to show that treatment results in pharmacological inhibition of necrotic core formation.

For instance, Hepg2 cell spheroids made in U-bottom low adhesion plates with an initial seeding cell density of 500 cells proliferate and form spheroids and by the 7th day show prominent necrotic cores, as seen with the standard viability dye DRAQ7^{™} that is a marker of cell death as it only stains the nuclei of dead and permeabilised cells (Figure 25, see control).

An increase in the level of cytosolic calcium ions is a key weak spot that induces necrosis. The inventors have found that relatively high doses of chelating agents (i.e. agents that sequester cytosolic calcium) are effective agents at blocking cell death, such as necrosis, during stress. The chelating agents EGTA (ethylene glycol-bis(β-aminoethyl ether)-N,N,N',N'-tetraacetic acid), DTPA (diethylenetriaminepentaacetic acid) and BAPTA AM (2-[N-[2-(acetyloxymethoxy)-2-oxoethyl]-2-[2-[2-[bis[2-(acetyloxymethoxy)-2-oxoethyl]amino]phenoxy]ethoxy]anilino]acetic acid acetyloxymethyl ester) were tested in this example. It is known in the art that cell culture dosages of around 50 µM, 50 µM and 1-3 µM, respectively are appropriate because they are considered maximum dosages which result in minimum toxicity. When a x8 dosage of BAPTA-AM (i.e., 25 µM) was used, significant necrosis suppression was seen on day 7 (Figure 2a). However, this dosage has some milder toxicity in some trials at earlier time points (Figure 2a, middle row). For EGTA, a x50 dosage (i.e., 2500 µM) was needed (Figure 2b) and for DTPA a x80 dosage (i.e., 4000 µM) was needed (Figure 2c) to supress necrosis. These latter two chelators also showed some mild, but not statistically significant, toxicity at earlier time points.

Therefore, use of relatively high doses of chelating agents confirms that necrosis can be inhibited. However, given the potential toxicity side-effects, such relatively high doses of chelating agents may not be preferred for *in vivo* treatment. However, this discovery of the benefits of using high dosages of chelating agents to treat or prevent necrosis presents advantages for uses outside of the body. Such uses include *in vitro* cell culture, such cell culture growth in laboratories and in tissue engineering, as well as graft (including cell, tissue and/or organ) preservation.

It will be appreciated that the discussion in Example 1, and in the examples below, apply generally to the invention as a whole.

### Example 2. Blocking individual steps associated with weak spots in cell physiology does not significantly prevent and/or treat necrosis.

Individual steps associated with the predicted weak spots in cell physiology that make cells susceptible to cellular necrosis (Figure 1) were blocked. Necrotic core formation was analysed, in line with the discussion above regarding the Hepg2 cells spheroids.

It was found that inhibition of individual steps did not significantly reduce the level of cell necrosis relative to control. The dosages used in Example 2 (as shown in the associated figures) was based on dosage which were expected to be the maximum doses showing minimal toxicity in cell culture based on the literature. No significant necrosis prevention and/or treatment was seen relative to control when:
1.) A whole range of individual plasma membrane calcium channel inhibitors were tested, including dihydropyridines, nondihydropyridines (phenylalkylamines and benzothiazepines), nonselective calcium channel inhibitors and gabapentinoids. And these covered inhibition of L,N, P, Q and/or T-type voltage gated ion channels (Figure 3).
2.) A range of ER/SR channel inhibitors was tested (Figure 4).
3.) A range of gap junction inhibitors were tested (Figure 5). This included products of vitamin A like retinoic acid, glycyrrhetinic acid and its derivative carbenoxolone, long-chain alcohols like heptanol and octanol, halogenated general volatile anaesthetics like halothane, fatty acids like linoleic acid, arachidonic acid and oleic acid, fatty acid amides like oleamide, fenamates (arylaminobenzoates) like flufenamic acid, niflumic acid and meclofenamic acid, quinine, quinidine and quinine-derivatives like mefloquine as well as other inhibitors.
4.) A range of SOCC inhibitors was tested (Figure 6).
5.) A range of calpain inhibitors was tested (Figure 7).
6.) Calpain/cathepsin non-selective protease inhibitors was tested (Figure 8).
7.) A range of cathepsin inhibitors was tested (Figure 9).

### Example 3. The best method to prevent and/or treat necrosis, in relation to strong efficacy and minimal toxic side effects, is using a combination of a gap junction inhibitor with an endoplasmic reticulum (ER)/sarcoplasmic reticulum (SR) calcium channel inhibitor.

Next combinations of compounds, where multiple targets in (Figure 1) are inhibited simultaneously were tested. An inhibition of gap junctions along with inhibition of ER/SR calcium channels surprisingly led to significant necrosis suppression.

In particular, the combination of the gap junction inhibitor retinoic acid was tested in combination with the ER/SE calcium channel inhibitor Dantrolene. Various doses of these two compounds were used in combination, as well as used individually. Individually, the compounds have no significant effect on necrosis inhibition (see Figure 10). In fact, the mean DRAQ7^{™} intensity value exhibited by some of the individual compound doses were above that of the control, suggesting possible toxicity (e.g. retinoic acid 20 µM at Day 7). In combination, however, a surprisingly strong inhibition of cell necrosis was seen, for each of the dosages used in the combination (Figure 10). The effects seen are synergistic, and are more than the simple additive effects of the single compounds which had no significant effect (Figure 10). Moreover, time points earlier than day 7 showed no increased toxicity for the combination (Figure 10).

As with Dantrolene and retinoic acid, a similar synergistic effect was surprisingly seen for the combination of Dantrolene with oleic acid. In this example, oleic acid was used as a gap junction inhibitor instead of retinoic acid. The combination of Dantrolene with oleic acid led to a significant decrease in cellular necrosis (Figure 11). This effect was more than additive. Indeed, the individual compound of Dantrolene had no significant effect on cell necrosis relative to control, and the individual compound of oleic acid had no significant effect on cell necrosis relative to control (Figure 11).

To further illustrate this advantageous combination of an ER/SR inhibitor with a gap junction inhibitor, a whole range of gap junction inhibitors were used in combination with Dantrolene (Figure 12). Gap junction inhibitors included glycyrrhetinic acid; long-chain alcohols like heptanol and octanol; halogenated general volatile anaesthetics like halothane; fatty acids; fatty acid amides; fenamates (arylaminobenzoates); as well as other inhibitors. As shown in Figure 12, individual gap junction inhibitors and individual compound Dantrolene had no significant effect. In many cases, administration of individual gap junction inhibitors exhibited a higher level of cell death than the control. However, a surprisingly strong, and more than additive, necrosis inhibition was achieved by administration of a combination of a gap junction inhibitor and the ER/SR calcium channel inhibitor Dantrolene was used. This was, in all cases, a striking effect (see Figure 12).

Notably, this beneficial effect on necrosis suppression (i.e., this synergistic effect), was also seen when multiple gap junction inhibitors were used in combination with the ER/SR inhibitor Dantrolene. In fact, when multiple gap junction inhibitors were used in combination with Dantrolene, almost no cell death necrosis was seen, even at day 7 (Figure 13). Thus, the combination of two or more gap junction inhibitors with an ER/SR calcium channel blocker is a particularly effective therapeutic strategy.

Next, the ER/SR calcium channel inhibitor Dantrolene was changed for the ER/SR calcium channel inhibitor ryanodine. Ryanodine was tested alongside, and in combination with, different gap junction inhibitors. As with dantrolene, ryanodine in isolation showed no significant difference to control, in fact most trials of ryanodine showed a greater level of cell death than the control (Figure 14). Likewise gap junction inhibitors in isolation showed no significant difference to control. However, the combination of ryanodine with any gap junction inhibitor showed a surprisingly strong, more than additive, suppression of cell death necrosis (Figure 14). Notably, even for compounds that demonstrated greater toxicity relative to control in isolation in most trials, like ryanodine and octanol or linoleic acid, the combination of compounds where one is an ER/SR calcium channel inhibitor and the other a gap junction inhibitor, showed significant suppression of cell death necrosis relative to control (Figure 14).

Next a range of other ER/SR calcium channel inhibitors, including trans-Ned 19 (Figure 15), cis-Ned 19 (Figure 16), procaine (Figure 17a) and ruthenium red (Figure 17b), were used in combination with a variety of different gap junction inhibitors. Again, regardless of the exact gap junction inhibitor and regardless of the exact ER/SR calcium channel inhibitor, when used individually these compounds had no significant effect on level of cell death necrosis. But when the compounds were combined, i.e., when an ER/SR channel inhibitor and a gap junction inhibitor were used in combination, a surprisingly robust and more than additive effect was observed on necrosis suppression (Figures 15, 16 and 17).

### Example 4. Other combinations of two compounds including plasma membrane calcium channel inhibitors, gap junction inhibitors and (ER)/sarcoplasmic reticulum (SR) inhibitors do not have a significant effect on the prevented and/or treatment of cell necrosis.

Other combinations of plasma membrane inhibitors, gap junction inhibitors and (ER)/SR inhibitors were explored. Compounds with high levels of efficacy and good safety profiles were used - the best choice compounds being Dantrolene as an ER/SR calcium channel inhibitor and retinoic acid as a gap junction inhibitor. As shown herein, specifically combining a gap junction inhibitor with an ER/SR inhibitor had a strong effect on necrosis suppression. However, it was found that other combinations did not have a beneficial effect (i.e. it was found that, to see a significant effect on necrosis inhibition, it is advantageous to simultaneously block ER/SR calcium ion channels and gap junctions, and that treatments which do not block these two targets simultaneously may exhibit cell necrosis when a stress is experienced by the cell). This is shown in the Examples using the ER/SR calcium ion channel inhibitors and gap junction inhibitors in combination (Figures 10-17).

When the gap junction inhibitor retinoic acid was used in combination with a range of different plasma membrane inhibitors (including dihydropyridines, nondihydropyridines (phenylalkylamines and benzothiazepines), nonselective calcium channel inhibitors and gabapentinoids, and including inhibition of L,N, P, Q and/or T-type voltage gated ion channels), no significant effect on necrosis inhibition was seen relative to control (Figure 18).

When the ER/SR calcium channel inhibitor Dantrolene was used in combination with a range of different plasma membrane inhibitors (including dihydropyridines, nondihydropyridines (phenylalkylamines and benzothiazepines), nonselective calcium channel inhibitors and gabapentinoids, and including inhibition of L,N, P, Q and/or T-type voltage gated ion channels), no significant effect on necrosis inhibition was seen relative to control (Figure 19).

### Example 5. Enhancing the combination of a gap junction inhibitor and (ER)/sarcoplasmic reticulum (SR) inhibitor that can supress necrosis, using plasma membrane calcium channel inhibitor.

The inventors have found that, in order to see a significant effect on necrosis inhibition, it is advantageous to simultaneously block ER/SR calcium ion channels and gap junctions. This is because it has been discovered that a critical step in the occurrence of necrosis is the calcium ion increase in the cytosol, due to any one of the 3 main points of calcium ion entry of into a cell: cell surface membrane channels, gap junctions, ER/SR calcium channels. The most susceptible points of entry are, in particular, entry of calcium ions via gap junctions and the endoplasmic ER/SR. It is believed that, because plasma membranes are in contact with the environment surrounding the cell, plasma membrane calcium ion channels will show greater levels of re-enforcement compared to ER/SR calcium channels and gap junctions.

Based on this, the most important therapeutic combination for necrosis suppression is a combination of one or more ER/SR calcium channel inhibitors and one or more gap junction inhibitors. The inventors have found that this combination can be further enhanced by adding to one or more plasma membrane inhibitors. This is confirmed herein. A gap junction inhibitor and an ER/SR calcium channel inhibitor, i.e. Dantrolene combined with retinoic acid, were further combined with a variety of plasma membrane calcium channel inhibitors. A further surprisingly strong inhibition of necrosis was seen by this combination. The effect was statistically significant when compared to control, as well as when compared to the combination of only Dantrolene and retinoic acid (Figure 20a). Notably the combination of the 3 classes, i.e. a gap junction inhibitor with an ER/SR calcium channel inhibitor and with a plasma membrane calcium channel inhibitor, had a more than additive effect of any of the three compounds given in isolation (see Figure 20a of the combination and Figures 3, 4 and 5 where the individual compounds have no significant effect relative to control; all figures are comparable with three independent trials of each done as part of the same experiment).

These advantages were further shown by using other gap junction inhibitors and a different ER/SR calcium channel inhibitor. The same effects were seen (Figure 20 b,c,d). This robustly demonstrates that, no matter the exact compounds, the combination of a gap junction inhibitor with an ER/SR calcium channel inhibitor and with a plasma membrane calcium channel inhibitor, results in a strong, statistically significant and more than additive effect of necrosis suppression. In fact, the effect is so strong, that even by day 7 when significant necrosis and a necrotic core was seen in control, almost no necrosis increase was seen in the combination of 3 of a gap junction inhibitor with an ER/SR calcium channel inhibitor and with a plasma membrane calcium channel inhibitor (Figure 20).

### Example 6. Enhancing the combination of a gap junction inhibitor and (ER)/sarcoplasmic reticulum (SR) inhibitor that can supress necrosis, using calcium chelating agents.

Another means to reduce cytosolic calcium ion concentrations is via use of an agent that sequesters calcium ions, i.e. calcium chelating agents. Adding calcium chelating agents to the combination of a gap junction inhibitor with an ER/SR calcium channel inhibitor was shown to have a further beneficial effect. The inventors believe that this is because, by working at a different target, efficacy is increased, while toxicity is unlikely to be increased.

When this was tested with different chelating agents, it was found that the addition of a calcium chelating agent to the combination of a gap junction inhibitor with an ER/SR calcium channel inhibitor resulted in a further beneficial effect on necrosis suppression, and had no additional toxic effects (Figure 21). In fact, earlier toxic effects causing variation between trials for the chelating agents (Figure 2) was not seen in this combination of a gap junction inhibitor with an ER/SR calcium channel inhibitor and a calcium chelating agent.

### Example 7. Other compounds including calpain inhibitors, cathepsin inhibitors and SOCC inhibitors, do not improve the combination of a gap junction inhibitor with an (ER)/sarcoplasmic reticulum (SR) inhibitor.

To further examine our hypothesis, we looked at other combinations of three compounds based on the key targets (see Figure 1) associated with weak spots in cell physiology that make cells susceptible to cell death necrosis when a stress is experienced and the combination of a gap junction inhibitor and an ER/SR calcium channel inhibitor, i.e. Dantrolene combined with retinoic acid. The remaining targets to combine and test here are calpain inhibitors, cathepsin inhibitors, and SOCC inhibitors.

Data were as follows:
1.) When a range of calpain inhibitors were added to Dantrolene combined with retinoic acid, no further reduction in cell death necrosis was observed relative to the combination of only Dantrolene combined with retinoic acid. In fact, the combination of three here became more variable, with many trials showing a higher level of cell death than the maximum level of cell death seen when the combination of only Dantrolene with retinoic acid (Figure 22a). This is in contrast to the addition of plasma membrane inhibitors and chelating agents that improved the effect of the combination of only Dantrolene with retinoic acid (Figure 20,21).
2.) The same trend was seen when a range of cathepsin inhibitors were added to the combination of Dantrolene with retinoic acid (Figure 22b), with the combination of 3 not showing any significant improvement over the combination of only Dantrolene with retinoic acid. This again is in contrast to the addition of plasma membrane inhibitors and chelating agents that improved the effect of the combination of only Dantrolene with retinoic acid (Figure 20,21).
3.) The same trend was seen when calpain/cathepsin non-specific protease inhibitors were added to the combination of Dantrolene with retinoic acid (Figure 22c), with the combination of 3 not showing any significant improvement over the combination of only Dantrolene with retinoic acid. This again is in contrast to the addition of plasma membrane inhibitors and chelating agents that improved the effect of the combination of only Dantrolene with retinoic acid (Figure 20,21).
4.) Finally, the same trend was seen when a range of SOCC inhibitors were added to the combination of Dantrolene with retinoic acid (Figure 22d), with the combination of 3 not showing any significant improvement over the combination of only Dantrolene with retinoic acid. This again is in contrast to the addition of plasma membrane inhibitors and chelating agents that improved the effect of the combination of only Dantrolene with retinoic acid (Figure 20,21).

### Example 8. Combinations of cathepsin inhibitors with calpain inhibitors, but not combinations of 2 or more calpain only or cathepsin only inhibitors, have a more than additive beneficial effect.

Calpains and cathepsins are only two of the many downstream mediators of destruction which follow a rise in cytosolic calcium ion rise during cell necrosis. Thus, inhibition of either only calpains or only cathepsins may have limited efficacy, and shown by this Example wherein no significant effect on cell death necrosis relative to control was seen (Figures 7 and 9). Additionally, calpain/cathepsin non-specific inhibitors risk off target toxic effects. Indeed, when tested, calpain/cathepsin non-specific inhibitors showed no significant beneficial suppression of cell death necrosis relative to control (Figure 8).

Data were as follows:
1.) When calpain only inhibitors were combined, no beneficial additive effect was seen, with the combination showing no significant difference from control (Figure 23a).
2.) When cathepsin only inhibitors were combined, no beneficial additive effect was seen, with the combination showing no significant difference from control (Figure 23b).
3.) When calpain inhibitors were combined with cathepsin inhibitors, a surprising beneficial and more than additive effect was seen, with the combination showing a significant difference from control (Figure 23c; data can be compared to Figure 7 and 9 of the compounds tested in isolation, and showing no significant difference from control).

### Example 9. Making use of safety profiles in combinations of compounds that can be purchased over-the-counter

It may be advantageous to use combinations of compounds that are known to have minimal toxicity when used in isolation. This can be particularly relevant when compounds are available over-the-counter. Examples of such compounds include linoleic acid and retinoic acid amongst other over the counter compounds such as Astaxanthin (which exhibits dual inhibitory effect as a gap junction inhibitor and a cathepsin inhibitor) and other similar carotenoids. By combining such over-the-counter compounds associated with the different targets in Figure 1, it is possible to see necrosis suppression with minimal toxicity. This Example shows that the combination of Astaxanthin with retinoic acid and linoleic acid resulted in a surprising beneficial, albeit mild, suppression of cell death necrosis (Figure 24). This has special utility *in vivo* where toxicity is a large concern, and may be especially useful when considering supplements to enhance general health as well as performance and reduce injury for individuals at risk of cell death necrosis, such as individuals undergoing strenuous exercise or with other underlying health conditions associated with cell death necrosis.

### Example 10. The combination of a gap junction inhibitor with an endoplasmic reticulum (ER)/sarcoplasmic reticulum (SR) calcium channel inhibitor used to block cell death necrosis shows robust safety and efficacy across cell types

To show that the combinations described herein are effective across multiple cell types and robustly blocks cell death necrosis with no major toxic side effects, further analysis was performed in this Example.

First, an analysis in Hepg2 spheroids was performed. This analysis looked at cell death levels through time, as well as the variation in spheroid size and the overall morphology and appearance of the spheroids, following the addition of the combination of a gap junction inhibitor and an ER/SR calcium channel inhibitor. Here, the combination used was Dantrolene combined with retinoic acid. As described hereinabove, the standard viability dye DRAQ7^{™} (a marker of cell death) was used to identify necrotic cores because it only stains the nuclei of dead and permeabilised cells.

Necrotic cores formed in the control spheroids. In contrast, no necrotic core was seen when the combination was added to the spheroids on day 2 of spheroid formation, even up to 7 days post spheroid formation when large dead necrotic core areas were seen in the control (Figure 25).

Notably, when the spheroid size was analysed through time, (wherein spheroid size is an indication of spheroid growth), no significant difference was observed with the combination added relative to the control (Figure 26). Moreover, there was no significant effect on spheroid morphology with the combination added relative to control, i.e. there was no disruption of spheroid integrity or formation or growth (Figure 26). Thus, no significant toxicity was caused by the combination of a gap junction inhibitor and an ER/SR calcium channel inhibitor.

Next, the same experiments were repeated using a different cell line, namely HEK293 cells that grow more quickly in culture. With HEK293 cells, necrotic cores were prominently seen by day 5 of culture in the control (Figure 27). A surprisingly strong suppression of necrosis was seen even in this different cell type with the combination of a gap junction inhibitor and an ER/SR calcium channel inhibitor relative to control (Figure 27).

Together, these findings demonstrate that the addition of a combination of gap junction inhibitor and an ER/SR calcium channel inhibitor results in a significant suppression of cell death necrosis across cell types.

Again, when spheroid size was analysed through time, no significant difference was observed with the combination added relative to control in this different cell type (Figure 28). Moreover, there was no significant effect on spheroid morphology with the combination added relative to control, i.e. there was no disruption of spheroid integrity or formation or growth (Figure 28). Together this demonstrated that significant toxicity was not caused by the combination of a gap junction inhibitor and an ER/SR calcium channel inhibitor across cell types.

Finally, as an additional experiment, and as a secondary indicator of cell death, a lactate dehydrogenase LDH-Glo^{™} assay was performed on supernatants from wells on day 7 treated with the combination vs controls in Hepg2 spheroids. The spheroids treated with the combination of a gap junction inhibitor and an ER/SR calcium channel inhibitor showed significantly lower levels of cell death relative to control (Figure 29). This demonstrates that regardless of marker of cell death, the same effects are seen. Thus, a composition comprising one or more endoplasmic/sarcoplasmic reticulum calcium channel inhibitors and one or more gap junction inhibitors is effective for use in methods of treating or preventing necrosis and cell death, as well as downstream conditions such as tissue damage or organ damage, in a subject.

### Example 11. Combinations of a gap junction inhibitor with an endoplasmic reticulum (ER)/sarcoplasmic reticulum (SR) calcium channel inhibitor +/- a plasma membrane inhibitor have robust preventative and treatment effects against cell death necrosis across different stressors.

Thus far, the Examples have focused on hypoxic and nutrient deprivation stressors. To test other stressors, this Example uses an oxidative stress/toxin stress model, where human intestinal fibroblast (HIF) primary cell spheroids were exposed to hydrogen peroxide. The standard viability Calcein AM/Ethidium homodimer-1 staining was used as a marker of cell death. Following 800µM hydrogen peroxide exposure for 6 hrs, just over 25% of cells were dead in the control (i.e. relative to Triton X-100 that kills all cells). The addition of a combination of a gap junction inhibitor and an ER/SR calcium channel inhibitor resulted in a surprisingly large and statistically significant reduction in level of cell death necrosis relative to control (Figure 30). Notably, this effect was more than additive of the individual components of the combination alone (Figure 30).

An even better effect was observed when the combination of a gap junction inhibitor with an ER/SR calcium channel inhibitor was further enhanced by adding a plasma membrane calcium channel inhibitor to the combination. Following stress, this combination of three compounds showed almost no increase in necrosis relative to unstressed control. A surprisingly large and statistically significant reduction in cell death necrosis was achieved relative to the stressed control (Figure 30). The effect is also clearly more than additive (Figure 30).

In contrast, other combinations tested showed no significant preventative effect on necrosis suppression. This included a combination of an ER/SR calcium channel inhibitor with a plasma membrane calcium channel inhibitor that showed no significant improvement over control (Figure 30), and a combination of a gap junction inhibitor with a plasma membrane calcium channel inhibitor that showed no significant improvement over control (Figure 30). In other words, an ER/SR calcium channel inhibitor must be combined and used with a gap junction inhibitor to achieve the most effective treatment.

To further highlight the benefit of the combination of an ER/SR calcium channel inhibitor with a gap junction inhibitor as a treatment post-stress (as well as a preventative measure), stress cell death was followed through time for up to three days in spheroids. A variety of stress conditions were used including 500µM hydrogen peroxide exposure for 6 hrs (Figure 31), 800µM hydrogen peroxide exposure for 6 hrs (Figure 32) and 1mM hydrogen peroxide exposure for 3 hrs (Figure 33). With concentrations of over 800µM hydrogen peroxide for 6 hours exposure on day 1, the majority of cells were dead by day 3 in the control (Figure 34). In all cases, a surprising reduction in cell death necrosis that was conserved through time was seen with the addition of the combination of an ER/SR calcium channel inhibitor combined with a gap junction inhibitor during and post stress (Figures 31,32,33 and 34).

Together these results demonstrate that combinations of a gap junction inhibitor with an endoplasmic reticulum ER/SR calcium channel inhibitor (+/- a plasma membrane inhibitor) have robust preventative and treatment effects against cell death necrosis across different stressors.

### Example 12. The combination of a gap junction inhibitor with an endoplasmic reticulum (ER)/sarcoplasmic reticulum (SR) calcium channel inhibitor used to block cell death necrosis shows robust safety profiles primary human cells

Potential toxic effects of the combination of a gap junction inhibitor with an ER/SR inhibitor were assessed in HIF primary cell spheroids. This was done by assessing (i) growth of spheroids through time when the combination was added on either day 0 prior to spheroid formation or on day 1 post spheroid formation and (ii) via ATP level quantification using a standard CellTiter-Glo^{®} 3D Cell Viability Assay.

Following cell seeding on day 0, spheroids formed with 4000 cells per spheroid by day 1. From day 1 to day 3, there was little variation in spheroid size, apart from some compacting of the spheroid that is more or less offset by low levels of cell proliferation (Figure 35a and b). Notably, with the addition of the combination of a gap junction inhibitor with an ER/SR inhibitor (given on either day 0 prior spheroid formation or on day 1 of spheroid formation), no significant decrease in spheroid size was seen. This indicates a lack of any severe toxic effects of the combination (Figure 35c).

ATP levels are a marker of cell viability, where the higher the level, the more viable the cells. When ATP levels were assessed on day 3 in cells treated with the combination on day 1 of spheroid formation, no significant decrease in the level of ATP was observed (in fact the mean ATP level was higher in the treatment relative to control) (Figure 35d).

In summary, these results suggest a robust safety profile for the combination of a gap junction inhibitor with an ER/SR inhibitor in human primary cells.

### Example 13. A combination of a gap junction inhibitor with an endoplasmic reticulum (ER)/sarcoplasmic reticulum (SR) calcium channel inhibitor can inhibit cell death necrosis and necrotic cores following nutrient deprivation and hypoxic stress in human primary cells

HIF primary cells showed natural necrotic core formation 6 days post spheroid formation. These cores formed due to the inner part of the spheroid being starved of oxygen and nutrients (Figure 36). Notably, the combination of a gap junction inhibitor with an endoplasmic reticulum (ER)/sarcoplasmic reticulum (SR) calcium channel inhibitor inhibited this necrotic core formation (Figure 36). This Example highlights a robust beneficial effect of this combination against multiple stressors.

### Example 14. Benefits of blocking the "predicted weak spots" of cell physiology to prevent cellular necrosis, increase survival and decrease movement defects is seen across species

It was tested whether this protective effect on necrosis from inhibition of the *predicted weak spots* was conserved across species, ranging from simple single celled bacteria and yeast to roundworms, fruit flies, fish and other mammals.

Bacteria, *E. coli,* and yeast, S. *pombi,* were exposed to toxin hydrogen peroxide stress (stress is for 50 min 1 mM H₂O₂) with or without high doses (2500 µM; c.f. Figure 2) of the chelating agent EGTA (inhibitor of the predicted weak spot of loss of calcium ion homeostasis in the cytosol) delivered during the stress.

Following stress, bacteria cells were then placed on agar plates and allowed to grow at 37°C overnight and colony forming units (CFU) were then counted.

For yeast, cells were stained with the standard viability dye Propidium iodide (PI) that only enters necrotic cells, placed on a microscope slide under a coverslip, and imaged using Nomarski and epifluorescence microscopy to identify the percentage of cells that undergo necrosis (all cells present could be counted using Nomarski microscopy).

Notably, with use of high levels of the calcium chelating agent, a reduction in the level of necrosis was observed in both bacteria and yeast. In bacteria, this was observed as an increase in CFU, which represents a larger percentage of viable cells (Figure 37a). In yeast this benefit was seen as a decrease in the percentage of cell that were stained with PI (Figure 37b).

Overall, these results indicate that use of high doses of chelating agents, as in mammalian cells, can inhibit cell necrosis during stress in single celled organisms like yeast and bacteria via suppression of the targeted primary predicted weak spot in cell physiology.

Next the model *Caenorhabditis elegans* was used and animal survival following stress was scored. As it is difficult to directly deliver free drugs via feeding to this model (*C. elegans* has a filter feeder that preferentially ingests particulate material), liposomes were used as a vector. Animals underwent a variety of stresses including heat stress where animals were placed at 37°C for 4.5 hrs (the optimal temperature of this species is around 18-20°C, and temperatures over this are more stressful with even 25°C having a negative effect on reproductive output); cold/freeze stress where animals were placed at -80°C for 1.5 min; oxidative stress where animals were placed in 2M hydrogen peroxide for 5 min.; and toxin stress where animals were placed in 1.3%(v/v) *tert-butylhydroperoxide (tBOOH)* for 5 min.

Animals were administered with high doses of chelating agents in liposomes either 3 hrs prior stress, i.e., as a preventative measure, or 3 hrs post stress as a late treatment. In all cases, across all stresses and regardless of the timing of treatment administration, a significant increase in animal survival was observed with high doses of chelating agents relative to control (Figure 38). These results indicate a robust reduction in overall necrosis during stress, regardless of the stress, and no significant toxic effects were caused by the treatment, i.e. therefore inhibiting agents described herein are effective at preventing necrosis and increasing survival following stress. These results also indicate a robust reduction in overall necrosis when treatment is administered post stress, regardless of the stressor, and no significant toxic effects result from the treatment, i.e. inhibiting agents described herein are effective at treatments of necrosis and effective at increasing survival following stress. The results also demonstrate that the inhibitors described herein can be administered via different routes, including use of vectors like liposomes.

Notably, following stress, *C*. *elegans* demonstrated movement defects, with animals showing slowed and less-coordinated movement. The percentage of animals with this defective movement were scored post stress. Animals that received high levels of chelating agents prior stress, showed marked and statistically significant improvements in movement regardless of the stressor (Figure 39). These results, therefore, demonstrate a robust reduction in overall movement defects following stress when the inhibiting agents described herein are administered.

Fruit flies, *Drosophila melanogaster,* were likewise exposed to heat and cold stress. Heat stress was exposure to the toxin hydrogen peroxide. Flies were starved for a short period of 8 hrs and then fed for 12 hrs yeast containing high doses of a calcium chelating agent to ensure an adequate amount of drug entered the animals; doses used were: 0 or 2500 µM EGTA. This was followed by cold/freeze stress, where animals were placed at 0°C for 10 min, and then animals were scored for survival every 12 hrs. With 2500 µM calcium chelator EGTA, a significant protective effect was seen (Figure 40). Thus, like with single celled yeast and bacteria and *C*. *elegans,* a full organism level protective effect of blocking the *predicted weak spots* associated with cell death necrosis was also seen in fruit flies.

Moving to more complex animals, the intact liver from freshly culled and dissected zebrafish (*Danio rerio*) was placed in PBS buffer and immediately stressed with the toxin hydrogen peroxide with or without EGTA. The liver was then washed in PBS and stained with PI as well as DAPI to visualise all cell nuclei (including intact cell nuclei). The liver was then placed on a microscope slide under a coverslip and imaged, and the percentage of PI-stained cells scored (relative to all cells seen in the image, i.e., DAPI stained). Livers treated with EGTA showed a reduction in the percentage of necrotic (PI stained) cells (Figure 41).

Moving to mammals, organotypic slices were prepared from various organs including the brain (hippocampal slices), spleen and heart of mice and placed in appropriate oxygenated buffer. Following this, the oxygen supply was replaced with nitrogen gas to induce hypoxia stress, with or without the presence of EGTA in the buffer. Cells were then stained with PI and DAPI to score the percentage of necrotic (PI stained) cells. Presence of high concentrations of the calcium chelating agent EGTA showed marked improvement in all 3 tissue types (Figure 42), highlighting a protective effect at the cell, tissue and organ level.

### Example 15. Further evidence in human cells and uses in tissue engineering of blocking the "predicted weak spots" of cell physiology to inhibit cellular necrosis

The protective role of inhibiting the *predicted weak spots* that make a cell susceptible to necrosis further in cell culture was explored in other forms of cell culture. Here, (i) HEK293 suspension culture was used, as well as (ii) a different technique used to make Hepg2 spheroids, i.e. a hanging drop method and culture on agarose.

For HEK293 suspension culture, cells were treated with a high dosage of EGTA 2 hrs prior to stress as well as during the stress. Stress was exposure to 1M hydrogen peroxide alongside untreated controls. Cells were then stained with DAPI and PI and Normarski and epifluoresence imaging was performed. The percentage of necrotic cells (PI-stained vs all cells DAPI stained) was significantly reduced in the EGTA treated cells (Figure 43a) showing that the protective effect is conserved regardless of form of cell culture.

Next, 3D cell culture was employed using Hepg2 cells and a standard hanging drop protocol of spheroid production. Notably a challenge in tissue engineering is the formation of necrotic cores in 3D cell culture, as it means organoids, organs etc. cannot currently be grown to relatively large sizes in a lab. Necrotic cores disrupt physiology, and a challenge is vasculature formation faster than necrotic core formation. To explore the utility of our compounds in tissue engineering, 20,000 cells were plated with or without the presence of EGTA (0, 250 and 500 mM) and necrotic core formation was then analysed by imaging the spheroids after 5 days; with 20,000 cells spheroids are at risk of cores as early as post 1 day. Presence of EGTA at 250 and 500 mM showed a marked improvement to the point of no necrotic cores being visible in treated spheroids even by day 5 (Figure 43b). Thus, along with the data in (Figures 2-36), the utility of necrosis inhibition in tissue engineering can be gauged.

### Example 16. Cell, tissue and organ preservation in University of Wisconsin (UW) organ preservation media supplemented with the combination of a gap junction inhibitor with an endoplasmic reticulum (ER)/sarcoplasmic reticulum (SR) calcium channel inhibitor shows reduced cell death necrosis.

UW solution is a gold standard organ preservation media. Mouse kidneys were removed from culled mice and perfused with UW with and without the combination of a gap junction inhibitor with an endoplasmic reticulum ER/SR calcium channel inhibitor, i.e. Dantrolene and retinoic acid, added. Kidneys were then stored for 36 hrs at 4°C to mimic cold storage, and then rewarmed to 37°C to mimic warm storage/ reperfusion. Following this, levels of viable cells (i.e. not apoptotic or necrotic) and levels of necrotic cells were measured using flow cytometry. A sample size of only 4 kidneys were used per condition, and even here a weakly significant difference was observed (pval=0.057) with a 35% reduction in necrosis percentage in the mean of the treatment and a 33% reduction of non-viable dead cells with the treatment (Figure 44).

### Exemplary Clauses

The invention is further described by the following numbered clauses:
1. A calcium activity inhibitor, a calpain inhibitor, a cathepsin inhibitor and/or a gap junction inhibitor for use in treating or preventing (i) necrosis, (ii) tissue damage, or (iii) organ damage, in a subject.
2. A method of treating or preventing (i) necrosis, (ii) tissue damage, or (iii) organ damage in a subject, the method comprising:
   administering a calcium activity inhibitor, a calpain inhibitor, a cathepsin inhibitor and/or a gap junction inhibitor to the subject to treat or prevent or necrosis, tissue damage or organ damage.
3. A method of preventing necrosis in a cell or tissue, the method comprising:
   contacting the cell or tissue with a calcium activity inhibitor, a calpain inhibitor, a cathepsin inhibitor and/or a gap junction inhibitor to prevent necrosis in the cell or tissue.
4. The calcium activity inhibitor, a calpain inhibitor, a cathepsin inhibitor and/or a gap junction inhibitor for use according to clause 1, or the method of clause 2, wherein the tissue damage is a selection of one, more or all of the group consisting of: burns, multiple organ dysfunction syndrome, organ failure, surgical trauma, bedsores, chemical burns, radiation burn, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure.
5. The calcium activity inhibitor, a calpain inhibitor, a cathepsin inhibitor and/or a gap junction inhibitor for use according to clause 1 or 4, or the method according to any one of clauses 2 to 4, wherein the tissue may be one or more tissues selected from the group comprising or consisting of: epithelial tissue, connective tissue, and muscle tissue.
6. A composition comprising a selection of one or more of a calcium activity inhibitor, a calpain inhibitor, a cathepsin inhibitor and a gap junction inhibitor.
7. The composition according to clause 6, wherein the composition is a pharmaceutical composition comprising a pharmaceutically acceptable carrier, and a selection of one or more of a calcium activity-inhibitor, a calpain inhibitor, a cathepsin inhibitor and a gap junction inhibitor.
8. A cosmetic composition comprising a calcium activity inhibitor, a calpain inhibitor, a cathepsin inhibitor and/or a gap junction inhibitor; and a cosmetically acceptable carrier.
9. The composition according to clause 8, wherein the composition comprises two or more of a calcium activity inhibitor, a calpain inhibitor, a cathepsin inhibitor and a gap junction inhibitor.
10. A method of making a cosmetic composition, the method comprising:
   contacting a selection of one or more of a calcium activity inhibitor, a calpain inhibitor, a cathepsin inhibitor and/or a gap junction inhibitor with a cosmetically acceptable carrier to create a cosmetic composition.
11. A method of using a cosmetic composition, the method comprising:
   applying a cosmetic composition according to clause 8 or clause 9 to the skin of a subject.
12. A food preservative comprising a selection of one or more of a calcium activity inhibitor, a calpain inhibitor, a cathepsin inhibitor and a gap junction inhibitor.
13. A food product comprising a food preservative according to clause 12.
14. A method of storing a food product, the method comprising contacting a food product with a food preservative according to clause 12 to store the food product.
15. A method of preserving a live tissue or organ, the method comprising culturing or storing the tissue or organ in a culture media comprising a calcium activity inhibitor, a calpain inhibitor, a cathepsin inhibitor and/or a gap junction inhibitor to preserve the tissue or organ.
16. A method of engineering a tissue or an organ, the method comprising contacting the organ or tissue with a culture media comprising a calcium activity inhibitor, a calpain inhibitor, a cathepsin inhibitor and/or a gap junction inhibitor.
17. A method of preserving or culturing blood cells, the method comprising contacting a culture media comprising blood cells with a calcium activity inhibitor, a calpain inhibitor, a cathepsin inhibitor and/or a gap junction inhibitor.
18. The method of clause 17, wherein the culture media is blood, plasma or serum.
19. A calcium activity inhibitor, a calpain inhibitor, a cathepsin inhibitor and/or a gap junction inhibitor for use according to any one of clauses 1, 4 and 5, the method according to any one of clauses 2-5, 10, 11, 14-18, the composition according to any one of clauses 6 to 9, the food preservative according to clause 12, and the food product according to clause 13, wherein the calcium activity inhibitor is calcium chelator, a calcium channel blocker or antagonist, a ryanodine receptor antagonist, and/or an InsP3R antagonist.
20. A calcium activity inhibitor, a calpain inhibitor, a cathepsin inhibitor and/or a gap junction inhibitor for use according to clause 19, the method according to clause 19, the composition according to clause 19, the food preservative according to clause 19, and the food product according to clause 19, wherein the calcium channel blocker prevents a rise in the intracellular concentration of free calcium ions by blocking voltage-dependent Ca²⁺ channels, orai channels and/or store-operated calcium channels.
21. A calcium activity inhibitor, a calpain inhibitor, a cathepsin inhibitor and/or a gap junction inhibitor for use according to clause 19, the method according to clause 19, the composition according to clause 19, the food preservative according to clause 19, and the food product according to clause 19, wherein the calcium channel blocker is a didihydropyridine, a non-didihydropyridine or a gabapentinoid.
22. A calcium activity inhibitor, a calpain inhibitor, a cathepsin inhibitor and/or a gap junction inhibitor for use according to clause 21, the method according to clause 21, the composition according to clause 21, the food preservative according to clause 21, and the food product according to clause 21, wherein the non-didihydropyridine is a phenylalkylamine or a benzothiazepine.

### NUMBERED EMBODIMENTS

1. A composition comprising one or more endoplasmic/sarcoplasmic reticulum calcium channel inhibitors and one or more gap junction inhibitors for use in a method of treating or preventing (i) necrosis, (ii) tissue damage, (iii) organ damage, or (iv) cell death, in a subject.
2. An endoplasmic/sarcoplasmic reticulum calcium channel inhibitor for use in a method of treating or preventing (i) necrosis, (ii) tissue damage, (iii) organ damage, or (iv) cell death in a subject, wherein said endoplasmic/sarcoplasmic reticulum calcium channel inhibitor is to be administered in combination with one or more gap junction inhibitors.
3. A gap junction inhibitor for use in a method of treating or preventing (i) necrosis, (ii) tissue damage, (iii) organ damage, or (iv) cell death in a subject, wherein said gap junction inhibitor is to be administered in combination with one or more endoplasmic/sarcoplasmic reticulum calcium channel inhibitors.
4. A method of treating or preventing (i) necrosis, (ii) tissue damage, (iii) organ damage, or (iv) cell death in a subject, the method comprising:
   administering one or more endoplasmic/sarcoplasmic reticulum calcium channel inhibitors and one or more gap junction inhibitors to the subject to treat or prevent necrosis, tissue damage, organ damage, or cell death,
   wherein the one or more endoplasmic/sarcoplasmic reticulum calcium channel inhibitors and the one or more gap junction inhibitors are administered simultaneously, separately or sequentially.
5. A method of preventing (i) necrosis or (ii) cell death in a cell or tissue, the method comprising:
   contacting the cell or tissue with one or more endoplasmic/sarcoplasmic reticulum calcium channel inhibitors and one or more gap junction inhibitors to prevent necrosis or cell death in the cell or tissue.
6. The composition for use according to embodiment 1, the endoplasmic/sarcoplasmic reticulum calcium channel inhibitor for use according to embodiment 2, the gap junction inhibitor for use according to embodiment 3, or the methods according to any one of embodiments 4-5, wherein there are one or more endoplasmic/sarcoplasmic reticulum calcium channel inhibitors and two or more gap junction inhibitors.
7. The composition for use according to embodiment 1 or 6, the endoplasmic/sarcoplasmic reticulum calcium channel inhibitor for use according to embodiment 2 or 6, the gap junction inhibitor for use according to embodiment 3 or 6, or the methods according to any one of embodiments 4-6, wherein there are two or more endoplasmic/sarcoplasmic reticulum calcium channel inhibitors and two or more gap junction inhibitors.
8. The composition for use according to any one of embodiments 1 or 6-7, the endoplasmic/sarcoplasmic reticulum calcium channel inhibitor for use according to any one of embodiments 2 or 6-7, the gap junction inhibitor for use according to any one of embodiments 3 or 6-7, or the methods according to any one of embodiments 4-7, wherein the endoplasmic/sarcoplasmic reticulum calcium channel inhibitor is a ryanodine receptor antagonist;
   optionally wherein the ryanodine receptor antagonist is selected from one or more of dantrolene, DHBP dibromide, cis-Ned 19, trans-Ned 19, ryanodine, SKF 86365 hydrochloride, ruthenium red, procaine, and tetracaine;
   optionally wherein the ryanodine receptor antagonist is selected from dantrolene and/or ryanodine.
9. The composition for use according to any one of embodiments 1 or 6-8, the endoplasmic/sarcoplasmic reticulum calcium channel inhibitor for use according to any one of embodiments 2 or 6-8, the gap junction inhibitor for use according to any one of embodiments 3 or 6-8, or the methods according to any one of embodiments 4-8, wherein the endoplasmic/sarcoplasmic reticulum calcium channel inhibitor is dantrolene.
10. The composition for use according to any one of embodiments 1 or 6-9, the endoplasmic/sarcoplasmic reticulum calcium channel inhibitor for use according to any one of embodiments 2 or 6-9, the gap junction inhibitor for use according to any one of embodiments 3 or 6-9, or the methods according to any one of embodiments 4-9, wherein the gap junction inhibitor is selected from (i) one or more of a fatty acid, a polyamine and a cyclodextrin, optionally wherein the fatty acid contains 13-21 carbons, or (ii) a connexin inhibitor and/or a pannexin inhibitor, preferably a connexin inhibitor.
11. The composition for use according to any one of embodiments 1 or 6-10, the endoplasmic/sarcoplasmic reticulum calcium channel inhibitor for use according to any one of embodiments 2 or 6-10, the gap junction inhibitor for use according to any one of embodiments 3 or 6-10, or the methods according to any one of embodiments 4-10, wherein the gap junction inhibitor is selected from one or more of 18α-glycyrrhetinic acid, 18β-glycyrrhetinic acid, carbenoxolone disodium, heptanol, octanol, halothane, oleic acid, oleamide, linoleic acid, arachidonic acid, palmitoleic acid, flufenamic acid, myristic acid, lauric acid, quinine, quinidine, dihydroquinidine, mefloquine, meclofenamic acid, probenecid, niflumic acid, flufenamic acid, astaxanthin, and retinoic acid.
12. The composition for use according to embodiment 11, the endoplasmic/sarcoplasmic reticulum calcium channel inhibitor for use according to embodiment 11, the gap junction inhibitor for use according to embodiment 11, or the method according to embodiment 11, wherein the gap junction inhibitor is one or more of retinoic acid, oleic acid, linoleic acid, octanol, heptanol, arachidonic acid, palmitoleic acid, flufenamic acid, myristic acid, halothane, astaxanthin and quinine.
13. The composition for use according to embodiment 12, the endoplasmic/sarcoplasmic reticulum calcium channel inhibitor for use according to embodiment 12, the gap junction inhibitor for use according to embodiment 12, or the method according to embodiment 12, wherein the gap junction inhibitor is one or more of retinoic acid, oleic acid, and linoleic acid.
14. The composition for use according to any one of embodiments 1 or 6-13, the endoplasmic/sarcoplasmic reticulum calcium channel inhibitor for use according to any one of embodiment 2 or 6-13, the gap junction inhibitor for use according to any one of embodiments 3 or 6-13, or the methods according to any one of embodiments 4-13, wherein the gap junction inhibitor is selected from two or more of oleic acid, linoleic acid, palmitoleic acid, quinine, astaxanthin, and retinoic acid.
15. The composition for use according to embodiment 14, the endoplasmic/sarcoplasmic reticulum calcium channel inhibitor for use according to embodiment 14, the gap junction inhibitor for use according to embodiment 14, or the methods according to embodiment 14, wherein the gap junction inhibitor is selected from (i) retinoic acid and linolic acid, (ii) retinoic acid and oleic acid, and (iii) linoleic acid and oleic acid.
16. The composition for use according to any one of embodiments 1 or 6-15, the endoplasmic/sarcoplasmic reticulum calcium channel inhibitor for use according to any one of embodiment 2 or 6-15, the gap junction inhibitor for use according to any one of embodiments 3 or 6-15, or the methods according to any one of embodiments 4-15, further comprising one or more plasma membrane calcium channel inhibitors.
17. The composition for use according to any one of embodiments 1 or 6-16, the endoplasmic/sarcoplasmic reticulum calcium channel inhibitor for use according to any one of embodiment 2 or 6-16, the gap junction inhibitor for use according to any one of embodiments 3 or 6-16, or the methods according to any one of embodiments 4-16, further comprising one or more calcium chelators.
18. The composition for use according to embodiment 16 or 17, the endoplasmic/sarcoplasmic reticulum calcium channel inhibitor for use according to embodiment 16 or 17, the gap junction inhibitor for use according to embodiment 16 or 17, or the methods according to embodiment 16 or 17, wherein the plasma membrane calcium channel inhibitor is selected from one or more of amlodipine, aranidipine, azelnidipine, barnidipine, benidipine, cilnidipine, clevidipine, efonidipine, felodipine, isradipine, lacidipine, lercanidipine, manidipine, nicardipine, nifedipine, nilvadipine, nimodipine, nisoldipine, nitrendipine, pranidipine, fendiline, gallopamil, verapamil, diltiazem, mibefradil, bepridil, flunarizine, fluspirilene, fendiline, gabapentin and pregabalin;
   preferably wherein the plasma membrane calcium channel inhibitor is selected from one or more of arandipine, clevidipine, efonidipine, felodipine, isradipine, lercanidipine, manidipine, nifedipine, nimodipine, nitrendipine, gallopamil, verapamil, diltiazem, mibefradil, fluspirilene, gabapentin or pregabalin.
19. The composition for use according to any one of embodiments 17-18, the endoplasmic/sarcoplasmic reticulum calcium channel inhibitor for use according to any one of embodiments 17-18, the gap junction inhibitor for use according to any one of embodiments 17-18, or the methods according to any one of embodiments 17-18, wherein the calcium chelator is selected from one or more of EDTA (Ethylenedioxy-diethylene-dinitrilo-tetraacetic acid); EGTA (Ethylene glycol-bis-(2-aminoethyl)-N,N,N',N'-tetraacetic acid); DTPA (diethylenetriaminepentaacetic acid); HEDTA (N-(2-Hydroxyethyl)ethylenediamine-N, N',N'-triacetic acid Trisodium salt); NTA (Nitrilotriacetic acid); BAPTA ((1,2-bis(o-aminophenoxy)ethane-N,N,N',N'-tetraacetic acid)); BAPTA AM (2-[N-[2-(acetyloxymethoxy)-2-oxoethyl]-2-[2-[2-[bis[2-(acetyloxymethoxy)-2-oxoethyl]amino]phenoxy]ethoxy]anilino]acetic acid acetyloxymethyl ester); citric acid; TPEN (N,N,N',N'-Tetrakis(2-pyridylmethyl)ethylenediamine); sodium citrate; and DMSA (dimercapto succinic acid);
   preferably wherein the calcium chelator is one or more of DTPA, BAPTA AM, sodium citrate, and citric acid.
20. The composition for use according to any one of embodiments 1 or 6-19, the endoplasmic/sarcoplasmic reticulum calcium channel inhibitor for use according to any one of embodiments 1 or 6-19, the gap junction inhibitor for use according to any one of embodiments 1 or 6-19, or the methods according to any one of embodiments 4-19, wherein the tissue damage is a selection of one, more or all of the group consisting of: burns, multiple organ dysfunction syndrome, organ failure, surgical trauma, bedsores, chemical burns, radiation burn, gangrene, alopecia, solar erythema, acute tubular necrosis, ulcers, physical injury, mechanical trauma, heat injury, cold injury, chilblain, trench foot, frostbite, avascular necrosis, pressure injury, and skin graft failure.
21. The composition for use according to any one of embodiments 1 or 6-20, the endoplasmic/sarcoplasmic reticulum calcium channel inhibitor for use according to any one of embodiments 1 or 6-20, the gap junction inhibitor for use according to any one of embodiments 1 or 6-20, or the methods according to any one of embodiments 4-20, wherein the tissue is one or more tissues selected from the group comprising or consisting of: epithelial tissue, connective tissue, muscle tissue, and neuronal tissue;
   preferably wherein the tissue is one or more tissues selected from the group comprising or consisting of: epithelial tissue, connective tissue, and muscle tissue.
22. The composition for use according to any one of embodiments 1 or 6-20, the endoplasmic/sarcoplasmic reticulum calcium channel inhibitor for use according to any one of embodiments 1 or 6-20, the gap junction inhibitor for use according to any one of embodiments 1 or 6-20, or the methods according to any one of embodiments 4-20, wherein the organ damage is in a neuronal organ or in an organ that is part of the central nervous system (CNS).
23. A food preservative comprising one or more endoplasmic/sarcoplasmic reticulum calcium channel inhibitors and one or more gap junction inhibitors;
   optionally wherein the food preservative further comprises one or more plasma membrane calcium channel inhibitors and/or one more calcium chelators.
24. A food product comprising a food preservative according to embodiment 23.
25. A method of storing a food product, the method comprising contacting a food product with a food preservative according to embodiment 23 to store the food product.
26. A method of preserving a live tissue or organ, the method comprising culturing or storing the tissue or organ in a culture media comprising one or more endoplasmic/sarcoplasmic reticulum calcium channel inhibitors and one or more gap junction inhibitors to preserve the tissue or organ;
   optionally wherein the culture media further comprises one or more plasma membrane calcium channel inhibitors and/or one or more calcium chelators.
27. The method of embodiment 26, wherein the culture media is the University of Wisconsin (UW) solution.
28. A method of engineering a tissue or an organ, the method comprising contacting the organ or tissue with a culture media comprising one or more endoplasmic/sarcoplasmic reticulum calcium channel inhibitors and one or more gap junction inhibitors;
   optionally wherein the culture media further comprises one or more plasma membrane calcium channel inhibitors and/or one or more calcium chelators.
29. A method of preserving or culturing blood cells, the method comprising contacting a culture media comprising blood cells with one or more endoplasmic/sarcoplasmic reticulum calcium channel inhibitors and one or more gap junction inhibitors;
   optionally wherein the contacting step further comprises contacting the culture media with one or more plasma membrane calcium channel inhibitors and/or one or more calcium chelators.
30. The method of embodiment 29, wherein the culture media is blood, plasma or serum.
31. A method of preserving blood, DNA or fertility samples, the method comprising contacting the samples with one or more endoplasmic/sarcoplasmic reticulum calcium channel inhibitors and one or more gap junction inhibitors to preserve the samples;
   optionally wherein the contacting step further comprises contacting the samples with one or more plasma membrane calcium channel inhibitors and/or one or more calcium chelators.
32. A method of preserving grafts, the method comprising contacting the graft with one or more endoplasmic/sarcoplasmic reticulum calcium channel inhibitors and one or more gap junction inhibitors to preserve the graft;
   optionally wherein the contacting step further comprises contacting the graft with one or more plasma membrane calcium channel inhibitors and/or one or more calcium chelators.
33. A method of preserving or culturing cells during cultivation of cells lines in a bioreactor, the method comprising contacting the cells with one or more endoplasmic/sarcoplasmic reticulum calcium channel inhibitors and one or more gap junction inhibitors to preserve or culture the cells;
   optionally wherein the contacting step further comprises contacting the cells with one or more plasma membrane calcium channel inhibitors and/or one or more calcium chelators.
34. A method of preserving or growing cell cultures, the method comprising contacting a culture media with one or more endoplasmic/sarcoplasmic reticulum calcium channel inhibitors and one or more gap junction inhibitors to preserve or grow the cell cultures;
   optionally wherein the contacting step further comprise contacting the culture media with one or more plasma membrane calcium channel inhibitors and/or one or more calcium chelators.
35. The food preservative according to embodiment 23, the food product according to embodiment 24, or the methods according to any one of embodiments 25-34, wherein the endoplasmic/sarcoplasmic reticulum calcium channel inhibitor, and/or the gap junction inhibitor, and/or the plasma membrane calcium channel inhibitor, and/or the calcium chelator is as recited in any one of embodiments 6-19.
36. A method of preserving a live tissue or organ, the method comprising culturing or storing the tissue or organ in a culture media comprising one or more calcium chelators to preserve the tissue or organ.
37. The method of embodiment 36, wherein the culture media is the University of Wisconsin (UW) solution.
38. A method of engineering a tissue or an organ, the method comprising contacting the organ or tissue with a culture media comprising one or more calcium chelators.
39. The methods according to any one of embodiments 36-38, wherein the culture media comprises a relatively high dose of the one or more calcium chelators, optionally wherein the culture media comprises the calcium chelator in an amount of about four times the dose typically used, optionally wherein the culture media comprises the one or more calcium chelators according to the amounts recited in Aspect A.
40. A composition comprising one or more calpain inhibitors and one or more cathepsin inhibitors for use in a method of treating or preventing (i) necrosis, (ii) tissue damage, (iii) organ damage, or (iv) cell death, in a subject.
41. The composition for use according to embodiment 40, wherein the one or more calpain inhibitors is selected from one or more members selected from the group comprising or consisting of: a calpastatin, MG101, MG132, vidupiprant, an α-mercaptoacrylate, a 5-azolone, a carboxamide and a α-helical cysteine protease inhibitor.
42. The composition for use according to embodiment 40 or 41, wherein the one or more cathepsin inhibitors is selected from one or more of dexamethasone, rifampicin, E64, E64d, calpeptin, CA 074, SID26681509, L006235, bafilomycin, aloxistatin, astaxanthin, chloroquine, clofazimine, dec-RVKR, and dexamethasone.

## Claims

1. A combination comprising dantrolene, or a pharmaceutically acceptable salt or a solvate thereof, and quinine, or a pharmaceutically acceptable salt or a solvate thereof.

2. The combination of claim 1, further comprising one or more additional endoplasmic/sarcoplasmic reticulum calcium channel inhibitors.

3. The combination of claim 2, wherein the additional endoplasmic/sarcoplasmic reticulum calcium channel inhibitor is a ryanodine receptor antagonist;
optionally wherein the additional ryanodine receptor antagonist is selected from one or more of DHBP dibromide, cis-Ned 19, trans-Ned 19, ryanodine, SKF 86365 hydrochloride, ruthenium red, procaine, and tetracaine;
optionally wherein the additional ryanodine receptor antagonist is ryanodine.

4. The combination of any one of claims 1-3, further comprising one or more additional gap junction inhibitors.

5. The combination of claim 4, wherein the additional gap junction inhibitor is selected from (i) one or more of a fatty acid, a polyamine and a cyclodextrin, optionally wherein the fatty acid contains 13-21 carbons, or (ii) a connexin inhibitor and/or a pannexin inhibitor, preferably a connexin inhibitor, or (iii) one or more of 18α-glycyrrhetinic acid, 18β-glycyrrhetinic acid, carbenoxolone disodium, heptanol, octanol, halothane, oleic acid, oleamide, linoleic acid, arachidonic acid, palmitoleic acid, flufenamic acid, myristic acid, lauric acid, quinidine, dihydroquinidine, mefloquine, meclofenamic acid, probenecid, niflumic acid, flufenamic acid, astaxanthin, and retinoic acid.

6. The combination of any one of claims 1-5, further comprising one or more plasma membrane calcium channel inhibitors.

7. The combination of claim 6, wherein the plasma membrane calcium channel inhibitor is selected from one or more of amlodipine, aranidipine, azelnidipine, barnidipine, benidipine, cilnidipine, clevidipine, efonidipine, felodipine, isradipine, lacidipine, lercanidipine, manidipine, nicardipine, nifedipine, nilvadipine, nimodipine, nisoldipine, nitrendipine, pranidipine, fendiline, gallopamil, verapamil, diltiazem, mibefradil, bepridil, flunarizine, fluspirilene, fendiline, gabapentin and pregabalin;
preferably wherein the plasma membrane calcium channel inhibitor is selected from one or more of arandipine, clevidipine, efonidipine, felodipine, isradipine, lercanidipine, manidipine, nifedipine, nimodipine, nitrendipine, gallopamil, verapamil, diltiazem, mibefradil, fluspirilene, gabapentin or pregabalin.

8. The combination of any one of claims 1-7, further comprising one or more calcium chelators.

9. The combination of claim 8, wherein the calcium chelator is selected from one or more of EDTA (Ethylenedioxy-diethylene-dinitrilo-tetraacetic acid); EGTA (Ethylene glycol-bis-(2-aminoethyl)-N,N,N',N'-tetraacetic acid); DTPA (diethylenetriaminepentaacetic acid); HEDTA (N-(2-Hydroxyethyl)ethylenediamine-N, N',N'-triacetic acid Trisodium salt); NTA (Nitrilotriacetic acid); BAPTA ((1,2-bis(o-aminophenoxy)ethane-N,N,N',N'-tetraacetic acid)); BAPTA AM (2-[N-[2-(acetyloxymethoxy)-2-oxoethyl]-2-[2-[2-[bis[2-(acetyloxymethoxy)-2-oxoethyl]amino]phenoxy]ethoxy]anilino]acetic acid acetyloxymethyl ester); citric acid; TPEN (N,N,N',N'-Tetrakis(2-pyridylmethyl)ethylenediamine); sodium citrate; and DMSA (dimercapto succinic acid);
preferably wherein the calcium chelator is one or more of DTPA, BAPTA AM, sodium citrate, and citric acid.

10. The combination of dantrolene, or a pharmaceutically acceptable salt or a solvate thereof, and quinine, or a pharmaceutically acceptable salt or a solvate thereof, for use in therapy.

11. Dantrolene, or a pharmaceutically acceptable salt or a solvate thereof, for use in therapy, wherein the dantrolene, or pharmaceutically acceptable salt or solvate thereof, is administered in a combination with quinine, or a pharmaceutically acceptable salt or a solvate thereof.

12. Quinine, or a pharmaceutically acceptable salt or a solvate thereof, for use in therapy, wherein the quinine, or pharmaceutically acceptable salt or solvate thereof, is administered in a combination with dantrolene, or a pharmaceutically acceptable salt or a solvate thereof.

13. The combination for use according to any one of claims 10-12, wherein the combination is as defined in any one of claims 2-9.

14. The dantrolene and/or the quinine, for use according to any one of claims 10-13, wherein the dantrolene, or pharmaceutically acceptable salt or solvate thereof, and quinine, or pharmaceutically acceptable salt or solvate thereof, are administered simultaneously, separately or sequentially.

15. The combination of any one of claims 1-9, or the dantrolene and/or the quinine for use according to any one of claims 10-14, for use in a method of treating or preventing (i) necrosis, (ii) tissue damage, (iii) organ damage, or (iv) cell death, in a subject.
